(19) Europäisches Patentamt
European Patent Office
Office européen des brevets

(11) EP 3 842 066 A1

(12) **EUROPEAN PATENT APPLICATION**

(43) Date of publication:
**30.06.2021 Bulletin 2021/26**

(51) Int Cl.:
***A61K 39/00*** *(2006.01)* ***A61K 39/395*** *(2006.01)*
***A61P 29/00*** *(2006.01)*

(21) Application number: **20199697.2**

(22) Date of filing: **18.02.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.02.2014 US 201461941352 P**

(62) Document number(s) of the earlier application(s) in accordance with Art. 76 EPC:
**15751704.6 / 3 107 564**

(71) Applicant: **CytoDyn Inc.**
**Vancouver, WA 98660 (US)**

(72) Inventor: **Montgomery, Alan B**
**Medina, WA 98039 (US)**

(74) Representative: **Potter Clarkson**
**The Belgrave Centre**
**Talbot Street**
**Nottingham NG1 5GG (GB)**

Remarks:
•This application was filed on 01-10-2020 as a divisional application to the application mentioned under INID code 62.
•Claims filed after the date of filing of the application / after the date of receipt of the divisional application (Rule 68(4) EPC).

(54) **USE OF ANTI-CCR5 ANTIBODIES IN GRAFT VERSUS HOST DISEASE**

(57) This composition and method provides for a method for reducing GVHD in a human subject which comprises administering to the subject at a predefined interval effective GVHD-reducing doses of (a) a humanized antibody designated PRO 140, or of (b) an anti-CCR5 receptor monoclonal antibody. This invention also provides a method for inhibiting in a human subject the onset or progression of GVHD. This invention also provides a method for treating a subject with GVHD comprising administering to the subject (a) a monoclonal antibody which (i) binds to a CCR5 receptor on the surface of the subject's CD4.sup.+ cells, and (b) a non-antibody CCR5 receptor antagonist, in amounts effective to treat the subject.

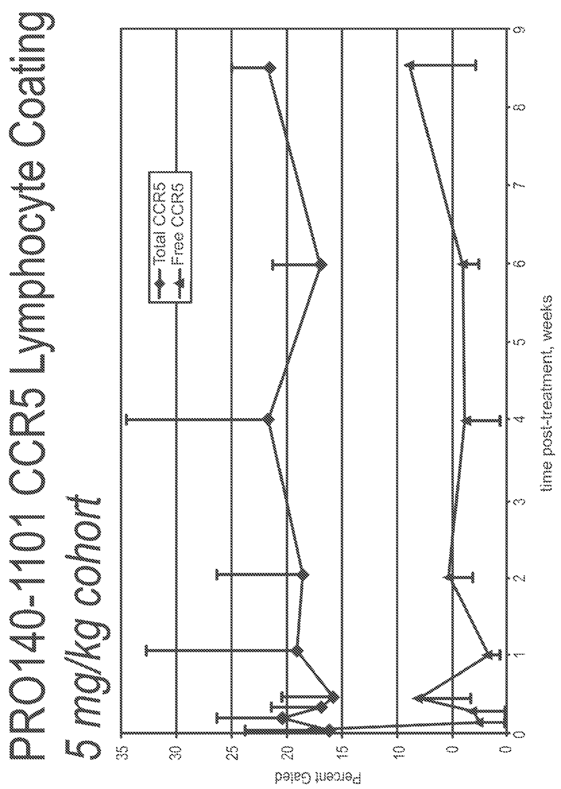


Figure 13

EP 3 842 066 A1

Printed by Jouve, 75001 PARIS (FR)

**Description**

RELATED APPLICATIONS

This application claims priority to U.S. provisional patent application serial number 61/941,352 filed on February 18, 2014, the contents of which are fully incorporated herein by reference.

INVENTION SUMMARY

This invention relates to the use of CCR5 receptor antagonist monoclonal antibodies and, possibly, other compounds to prevent, treat, or mitigate the effects of graft-versus-host disease (GVHD). In one embodiment, this invention includes the use of PRO 140, a known CCR5 receptor antagonist, either separately or together with other compounds to prevent, treat, or mitigate the effects of GVHD. In another embodiment, PRO 140 is used together with at least one other compound to achieve a synergistic effect to prevent, treat, or mitigate the effects of GVHD.

Because PRO 140 is a known CCR5 cell receptor antagonist, and has application, like maraviroc, to effectively block HIV-1 cell entry via the CCR5 cell receptor, PRO 140 may also have therapeutic applications for the prevention, treatment, or mitigation of GVHD, like maraviroc. PRO 140 and maraviroc are known to directly bind to the CCR5 cell receptor in different but necessarily neighboring locations, and ample evidence suggests that PRO 140's and maraviroc's mechanisms of action are complementary and, potentially, synergistic with respect to preventing certain CCR5 cell receptor activities. Further, because dual exposure of CCR5 receptors to both PRO 140 and maraviroc has been demonstrated to result in CCR5 cell receptor binding that results in synergistic HIV-1 cell entry blockade, it may be that such a combination of compounds may be similarly brought to bear to prevent, treat, or mitigate the effects of GVHD.

Indeed, it is contemplated that maraviroc-bound CCR5 receptors, upon exposure to PRO 140, may form a ternary complex that effectively prevents or reduces CCR5 activity as such relates to GVHD. While on the topic of imposing structural or chemical interference at the CCR5 cell receptor to prevent, treat, or mitigate the effects of GVHD, it is also noted that extended life maraviroc conjugates, including PEGylated compounds and chemically programmed antibodies, have been made without destroying maraviroc's binding capability. Accordingly, it is contemplated that maraviroc provides sufficient flexibility to exercise therapeutic effect despite, and perhaps further to, the immediate presence of neighboring CCR5 cell receptor binding entities. To this end, multiple CCR5 cell receptor binding agents, including any of antibodies or fragments thereof, proteins or fragments thereof, and small molecules, may be effectively, or most effectively used in concert.

CCR5 receptor antagonists generally include antibodies and fragments thereof, proteins and fragments thereof, and small molecule inhibitors or drugs. Antibodies and other protein therapeutics have some advantages and disadvantages when compared to small molecule inhibitors or drugs. Since antibodies and other protein therapeutics are often formulated for IV administration there may be an inconvenience caused by intravenous dose. However, because most transplant recipients have indwelling long-term Hickman catheters, any inconvenience due to IV administration is reduced. Because antibodies and other protein therapeutics typically have longer circulating half-lives in vivo, the frequency of dosing may be reduced. Accordingly, patient compliance with regard to maintaining their dosing regimes would likely be better than for orally dosed therapeutics which often require daily, or multiple daily, dosings. Further, because some antibiotics may be accompanied by few or no side effects, a patient's adherence to a regular dosing regimen may be further improved relative to other therapeutics that give rise to unpleasant side effects such as nausea. Small molecule therapies frequently have off target effects, which is less common in antibody therapies. For example, the adverse event profile of maraviroc is well described in HIV patients and includes diarrhea, nausea, fatigue, and headache. It is noted that, for some patients with GVHD, interpretation of adverse event causality could be problematic because these symptoms may associated with GVHD. Moreover, in the case of GVHD, the subject patient population may suffer from damaged bowels resulting in maladsorption and reduced effectiveness of orally ingested therapeutics, in which case an antibody, such as a monoclonal antibody, e.g., PRO 140, may provide superior pharmacodynamics coverage.

BACKGROUND

1. GRAFT VERSUS HOST DISEASE (GVHD)

GVHD is a major complication of allogeneic hematopoietic stem cell transplant (AHSCT) associated with significant morbidity and mortality. New discoveries in immunology have expanded our understanding of GVHD, in which tissue damage from chemotherapy or radiation results in cytokine release, which activates T cells, resulting in proliferation and differentiation, trafficking to target organs, and tissue destruction and inflammation. Insights into the mechanisms of this disease relate directly to the development of preventive strategies and therapies, such as immunosuppression, T-cell depletion, calcineurin inhibitors, CCR5 antagonists, gut decontamination, extracorporeal photopheresis, and more.

GVHD affects the gut, liver, and skin.

Chemokine receptor CCR5 plays a role in solid organ allograft rejection and mediates murine GVHD pathogenesis. It is reported that infiltrating lymphocytes in the skin of human acute GVHD samples are predominantly $CCR5^+$ T cells. Lisa Palmer, George Sale, John Balogun, Dan Li, Dan Jones, Jeffrey Molldrem, Rainer Storb, Qing Ma, Chemokine Receptor CCR5 Mediates Allo-Immune Responses in Graft-vs-Host Disease, Biol Blood Marrow Transplant 2010 March; 16(3): 311-319, doi: 10.1016/j.bbmt2009.12.002 ("Palmer 2010"). It has also been found that the $CCR5^+$ population exhibits the characteristics of the activated effector T cell phenotype. CCR5 expression is upregulated upon allogenic stimulation and $CCR5^+$ cells are proliferating with co-expression of T cell activation markers. Furthermore, the activated T cells producing inflammatory cytokine TNF$\alpha$, IL-2 or IFN-$\gamma$, are positive for CCR5. Thus, CCR5 is a marker for GVHD effector cells and that $CCR5^+$ T cells are active participants in the pathogenesis of human acute GVHD.

According to the Palmer 2010 article, GVHD classically develops over five steps. *See* Figure 19, Palmer 2010. First, tissue damage from the conditioning regimen (either radiation or chemotherapy) releases proinflammatory cytokines such as tumor necrosis factor-$\alpha$ (TNF-$\alpha$) and interleukin-1 (IL-1) and danger signals such as adenosine-5'-triphosphate (ATP) and nicotine adenine dinucleotide, as well as extracellular matrix proteins such as biglycan that promote activation and maturation of antigen-presenting cells (APCs). This is furthered by damage to the gastrointestinal epithelium, allowing translocation of lipopolysaccharide, which can activate innate immunity through Toll-like receptors, furthering the cytokine cascade. Polymorphisms in cytokine genes have been shown to affect the severity of GVHD.

Second, donor T-cell activation is triggered by recipient antigens presented by host APCs and sustained by donor APCs. This is mediated by human leukocyte antigen (HLA) proteins encoded by the major histocompatibility complex (MHC) found on chromosome 6. MHC compatibility is the most powerful determinant of GVHD, and there is a direct relationship between the frequency of GVHD and mismatch at HLA-A, -B, -C, and -DRB1 (mismatch at HLA-DQ and -DP appears less significant, although still important). However, despite full 8 of 8 or even 12 of 12 match, 40% of recipients still develop GVHD, thought secondary to minor histocompatibility antigens (MiHAs). MiHAs are also targets for GVT.

In addition to the interaction between the T-cell receptor and MHC, T-cell activation requires signaling between costimulatory molecules such as CD28 (present on the T cell) and B7.1 or B72 (CD80 or CD86, present on the APC); other T-cell:APC costimulatory signaling pairs include inducible costimulator (ICOS) (CD278):B7H (CD275), OX40 (CD134):OX40L (CD252), CD40L (CD154):CD40, and 4-1BB (CD137):glucocorticoid-induced tumor necrosis factor receptor (GITR). The absence of these costimulatory signals, particularly CD28:B7.1/B7.2, can lead to anergy; furthermore, this interaction can be blocked by coinhibitory molecules such as CTLA4 (CD152), which competes with CD28 for B7.1/B7.2. Programmed death-1 (PD-1) (CD279):programmed death ligand 1 (B7H1, CD274) are another pair of inhibitory molecules that can induce anergy or tolerance. Models that block these costimulatory or coinhibitory interactions have been shown to reduce or exacerbate GVHD, suggesting possible therapeutic targets.

Third, T cells proliferate and differentiate into naive, effector, memory, regulatory, Th1/Tc1, Th2/Tc2, Th17, and other subsets. Naive $CD44^{lo}CD62L^{hi}$ T cells appear to be essential to this response; interestingly, $CD44^{hi}CD62L^{lo}$ effector memory and $CD44^{hi}CD62L^{hi}$ central memory T cells may be able to promote GVT without GVHD. The balance between Th1/Tc1 and Th2/Tc2 subsets as well as other subsets such as Th17 and the productions of cytokines such as IL-4, IL-5, IL-6, IL-12, IL-13, IL-17, IL-21, IL-23, TNF-$\alpha$, transforming growth factor-$\beta$, and interferon-$\gamma$ (IFN-$\gamma$) have been shown to impact the manifestation of GVHD, although the various contributions of each of these elements are still under active investigation.

Fourth, activated T cells migrate from secondary lymphoid organs to target tissues (skin, liver, gut) through a combination of chemokine-receptor, selectin-ligand, and integrin-ligand interactions. Selectins and integrins mediate rolling and tethering of lymphocytes along high endothelial venules through interactions with their matching ligands. For example, interactions between L-selectin (CD62L) and $\alpha4\beta7$ integrin expressed on T cells and peripheral node addressin and mucosal addressin cell adhesion molecule expressed on secondary lymphoid tissue mediate homing to mesenteric lymph nodes and Peyer patches and induction of gut GVHD. Lymphocyte chemotaxis receptors such as chemokine receptor type 5 (CCR5), CCR6, and CCR7 are also essential to T-cell trafficking between secondary lymphoid tissues and target organs.

Fifth, once they reach target organs, T cells cause tissue destruction through direct cytotoxic activity as well as recruitment of other leukocytes. Cytotoxic activity is largely mediated by the Fas ligand:Fas and perforin-granzyme pathways. Interestingly, other cytolytic pathways, such as TNF-related apoptosis-inducing ligand, may preferentially mediate GVT but not GVHD. Cytokines such as TNF-$\alpha$, IFN-$\gamma$, IL-2, IL-7, IL-10, and others also appear to be essential to regulating leukocyte recruitment and tissue destruction; these effects are dependent on strength, timing, and other interactions, making the effects of individual cytokines difficult to predict.

Although the above discussion focuses on T cells, B cells also play a role in GVHD via antigen presentation, cytokine secretion, and antibody production.

In the United States, in 2011, over 7,500 unrelated donor bone marrow transplants were performed, with another 10,000 conducted outside of the United States. (*See* Transplants Data and Outcomes (accessed Oct 31, 2013) http://bloodcell.transplant.hrsa.gov/research/transplant_data/index.html; World Marrow Donor Association, Annual Report: Stem Cell Donor Registries. Leiden: World Marrow Donor Association and European Blood and Marrow Transplantation Group, 2010; and World Marrow Donor Association, Annual Report: Unrelated Cord Blood Banks/Registries. Leiden: World Marrow Donor Association, European Blood and Marrow Transplantation Group, and NetCord Foundation, 2010.) One-year survival is now approximately 60% with the two most common causes of death being disease relapse or GVHD. If one assumes that life long therapy is required, a new standard of care chronic treatment would over a five-year span be needed in over 20,000 patients in the US alone, with slightly larger numbers ex-US. If the therapy is priced at $20,000 a year, the total potential US market is about 400 million dollars. If the therapy decreases mortality or shows greater pharmacoeconomic benefit by preventing hospitalization for GVHD disease, higher pricing and higher value would be expected. In addition, control of GVHD would lead to greater use of BMT in non malignant disease such as sickle cell, and the total number of unrelated BMTs would likely increase. Ex US markets would be larger than the US, but with lower pricing, so total market sizes would be expected to be similar.

GVHD is a serious problem with over 500 clinical trials underway, indicative of the known and long-felt need for improved GVHD therapies. A summary of a sample of 75 of the 1131 GVHD trials currently available from a Clintrials.gov search on the term GVHD is provided below. Of these, 515 are currently open. Of the 1131 trials, 130 were in ocular GVHD, these were excluded from the sample.

| Phase | Number of Studies | Patients med (range) | Length of Study mo. med (range) | Completed[(1)] n (%) |
|---|---|---|---|---|
| **1** | 11 | 12 (3 - 40) | 6 (6 - 24) | 4 (36%) |
| **2** | 50 | 37 (5 - 180) | 12 (6 - 60) | 15 (31%) |
| **3** | 12 | 219 (50 - 312) | 12 (2 - 24) | 7 (58%) |
| **4** | 2 | 169 (37 - 300) | 17 (9 - 24) | 1 (50%) |

(1) Denominator includes only studies with known status

2. PREVENTION OF GVHD

According to the Sung 2013 article (see full reference cite below), immunosuppression has been the primary pharmacologic strategy to prevent GVHD. Methotrexate has been used since the 1950s as a way of shutting down T cells through inhibition of dihydrofolate reductase and production of thymidylate and purines. Anthony D. Sung and Nelson J. Chao, Concise Review: Acute Graft-Versus-Host Disease: Immunobiology, Prevention, and Treatment, Stem Cells Tranlations Medicine 2013; 2:25-32 ("Sung 2013"). Post-transplant cyclophosphamide is another method of eliminating rapidly dividing T cells that shows promise in recent clinical trials. The calcineurin inhibitors cyclosporine and tacrolimus inhibit T-cell proliferation; combinations with methotrexate have successfully been used since the 1970s and are the cornerstone of most prophylactic regimens. However, these agents have numerous side effects, including delayed cell count and immunological recovery, thrombotic microangiopathy, and posterior reversible encephalopathy syndrome. The inosine monophosphate dehydrogenase inhibitor mycophenolate mofetil and the mammalian target ofrapamycin (mTOR) inhibitor sirolimus have been proposed as alternate agents; however, there is no clear consensus on optimal drug combination, dosing, or timing.

Other drugs attempt to target cytokine/chemokine-receptor interactions that appear integral to development of GVHD. While some success has been reported with maraviroc, a CCR5 antagonist that blocks T-cell chemotaxis and dramatically decreased the incidence of gastrointestinal and liver GVHD as detailed below. At the same time, despite the central role of cytokines IL-1 and TNF-$\alpha$, drugs that block these pathways (etanercept, infliximab) failed to improve rates of acute GVHD.

Alternative methods of reducing GVHD involve dampening the cytokine storm that sets off the cascade. Reduced intensity conditioning causes less tissue damage and has been shown to reduce GVHD.

Additionally, gut decontamination with ciprofloxacin and metronidazole has been shown to decrease the risk of acute GVHD compared with ciprofloxacin alone. However, attempts to preserve epithelial integrity with keratinocyte growth factor (palifermin) did not decrease the risk of GVHD, although palifermin does reduce the risk of mucositis.

Given the central role of T cells in GVHD, T-cell depletion (TCD) has been studied since the 1980s as a preventative strategy. This can be done with physical techniques, such as ex vivo counterflow centrifugal elutriation or soybean lectin agglutination and E-rosetting, or by immunological methods, such as ex vivo or in vivo administration of anti-sera (anti-thymocyte globulin) or monoclonal antibodies; positive selection techniques can also isolate CD34+ cells ex vivo, allowing T cells to be discarded. Randomized trials have shown that although TCD successfully decreases the risk of GVHD, the risks of graft failure, disease relapse, and opportunistic infections are increased. However, some of these risks can be mitigated (e.g., higher CD34+ cell dose to promote engraftment, antibiotic prophylaxis to prevent opportunistic infections), and more recent single-arm trials have shown 3-year disease-free survival approaching 60%.

Furthermore, T-cell depletion strategies such as in vivo administration of the anti-CD52 antibody alemtuzumab can facilitate transplants from HLA-mismatched haploidentical donors without significant GVHD, opening up transplant options to patients without HLA-matched donors.

Alternative methods of suppressing T cells are promising. A single-arm trial of soluble CTLA4 targeting the CD28:B7 costimulatory pathway successfully induced anergy and reported a low rate of GVHD, although further studies are lacking. Another strategy is infusion of multipotent mesenchymal stromal cells (MSCs, also known as mesenchymal stem cells), which maintain the ability to differentiate into a variety of supportive cells. MSCs exert immunosuppressive effects on both lymphocytes and APCs. In a randomized phase II trial, prophylactic MSC administration resulted in only 5.3% grade II-IV acute GVHD compared with 38.9% in the control arm.

3. TREATMENT OF GVHD

Also, according to the Sung 2013 article, glucocorticoids are the gold standard for treatment of grade II-IV acute GVHD, even though the main mechanism of action still remains unclear (possibilities include suppression of proinflammatory cytokines, as well as direct lymphotoxic effects). A randomized clinical trial has suggested that methylprednisolone at 2 mg/kg per day is the ideal starting dose, with escalating doses or the addition of alternative agents if there is no response by 5 days. Acute adverse effects include hyperglycemia and psychosis; chronic changes include immunosuppression and infections, myopathy, osteoporosis and avascular necrosis of bone, cataracts, and fat distribution. Unfortunately, only about half of patients respond, and there is no clear second-line agent for steroid-refractory GVHD.

Many single-arm trials have shown benefit with other immunomodulators, such as anti-thymocyte globulin, tacrolimus, sirolimus, or mycophenolate mofetil, or antibody therapy against CD3, CD7, CD52, CD147, IL-2-R, IL-1, and TNF-$\alpha$; however, none of these agents have proven efficacy in randomized clinical trials. Intravenous immunoglobulin (IVIG) is the only drug that has been shown to reduce the rate of acute GVHD in a large randomized clinical trial (51% in controls vs. 34% in IVIG recipients), although its cost and concern for impaired humoral recovery limit its widespread use.

Many novel approaches are currently under investigation. Adoptive transfer of Tregs following in vitro enrichment and expansion has shown safety and decreased rates of GVHD compared with historical controls. Extracorporeal photopheresis involves ex vivo incubation of patient leukocytes with 8-methyoxypsoralen and ultraviolet A (UVA) irradiation, exposure to UVA, and reinfusion, resulting in immunomodulatory effects including lymphocyte apoptosis, increasing Tregs, and shifting from a Th1 to Th2 phenotype; studies are now looking at this strategy for prevention. Denileukin diftitox, a recombinant protein composed of IL-2 fused to diphtheria toxin, is another novel approach that showed promise in a phase II trial, as is pentostatin, a purine analog. Phototherapy using UVA irradiation with or without psoralen appears to help cutaneous lesions, and oral beclometasone may improve gastrointestinal GVHD. Finally, in addition to use as prophylaxis, mesenchymal stem cells have been used for treatment of GVHD, with promising response rates ranging from 71% to 94% and complete response rates of 55%-74%.

4. MORE ON THE ROLE CCR5 RECEPTOR IN GVHD

According to the Palmer 2010 article, chemokines and their receptors are important in many human diseases, including HIV, autoimmune disease, inflammatory disease and organ graft rejection. Currently small molecule antagonists and humanized monoclonal antibodies targeting chemokine receptors are developed by industry and tested in preclinical models as well as in phase I trials. Data demonstrates that $CCR5^+$ T cells are important in mediating GVHD in human.

CCR5 is upregulated upon allostimulation and expressed on activated T cells. In addition, the expression of CCR5 is restricted to the proliferating T lymphocytes. Furthermore, it has been found that activated T cells producing cytokine TNF$\alpha$, IL-2 or IFN-$\gamma$ are positive for CCR5. Data indicates that CCR5 expression on T cells mediates alloimmune responses in GVHD.

CCR5 is considered one of the "inflammatory" chemokine receptors regulated by proinflammatory stimuli to orchestrate immune responses, in comparison to the "homeostatic " group which are important in immune surveillance such as CCR7. Inflammatory chemokine receptors are upregulated during tissue damage or inflammation, and involved in T cell polarization. Gene expression profiles have shown that CCR5 was upregulated during aGVHD in human Data further demonstrated that CCR5 expression is on activated and proliferating T cells upon allogeneic stimulation. In contrast, CCR7 was found within both proliferating and non-proliferating T cells, and there was no significant increase in the expression of CCR7. Interestingly, CXCR3, another inflammatory chemokine receptor, does not show the same restriction to proliferating cells as CCR5. Although almost all proliferating T cells were positive for CXCR3, there were high percentage of non-proliferating T cells expressing this chemokine receptor. Thus, the data suggests a critical role of CCR5 expression in alloimmune responses and GVHD.

The protective effect of the CCR5 deletion mutation in GVHD has been demonstrated. A small cohort study found that in the absence of CCR5 on donor cells, there is a decreased incidence of GVHD and increased relapse rate in recipients received HLA-matched unrelated marrow donors. In particular, there is a significant reduction of skin GVHD.

We further examined the skin and lip biopsies from aGVHD patients for CCR5 expression in situ, and found the majority of infiltrating T cells were positive for CCR5. Altogether, these data confirm that CCR5 expression plays an important role in mediating human GVHD.

CCR5 is highly upregulated on both $CD4^+$(Th1) T cells and activated antigen specific $CD8^+$ T cells. Using intracellular cytokine assay, it has been found that activated $CD4^+$ T cells that produce inflammatory cytokine TNF$\alpha$, IL-2 or IFN-$\gamma$, were positive for CCR5. In addition, the TNF$\alpha$ and IL-2 producing $CD8^+$ T cells also expressed CCR5. This observation supported the notion that CCR5 is a marker for effector T cells that actively participate in the "cytokine storm" of GVHD.

Chemokines and their receptors play an important role in regulating leukocyte migration and activation. Recently, the functional state ofT cells has been characterized by the chemokine receptor expression pattern. In particular, chemokine receptor CCR5 is a marker for effector T cells. CCR5 is a co-receptor for HIV entry and has been studied extensively. The expression of CCR5 is very low on naïve T cells, but is highly upregulated on both $CD4^+$(Th1) T cells and activated antigen specific $CD8^+$ T cells.

The function of CCR5 and its ligands in GVHD has been primarily explored in murine models. It has been reported that $CCR5^+/CD8^+$ T cells mediate hepatic injury in mouse GVHD and blocking antibody to CCR5 reduces the damage. In addition, MIP-1$\alpha$, one of the ligands for CCR5 has also been shown to mediate mouse GVHD. While two groups demonstrated that genetic deletion of CCR5 in the donor can reduce acute GVHD in mice; others reported that $CCR5^{-/-}$ or MIP-1$\alpha^{-/-}$ donor T cells accelerate GVHD in liver and lung. These data suggested that the role of CCR5 in alloimmnnme responses is complicated, and probably regulated in strain-, target organ- or pretransplant conditioning-dependant fashion in murine GVHD models.

In addition to T cells, other cell types such as dendritic cells (DCs) and B cells are involved in the pathogenesis of GVHD. CCR5 has been shown to express on dendritic cells and specifically the dermal Langerhans cells. Langerhans cells represent the specialized DCs of the epidermis, and play an important role in skin GVHD. Clearly the role of CCR5 is intricate and complicated and the field is still unresolved regarding the role of CCR5 in human GVHD.

It has been postulated, however, that the CCR5 receptor is an important mediator of GVHD, especially in the organ damage that is the usual cause of death. Chemokines have been hypothesized to play a role in the pathogenesis of GVHD after allogeneic hematopoietic transplantation. Over a decade ago, reports had indicated that the absence of donor expression of CCR5 on T cells ameliorates GVHD in models using no conditioning of the recipient. (*See* M. Murai, H. Yoneyama, A. Harada, Z. Yi, C. Vestergaard, B. Guo, K. Suzuki, H. Asakura, K. Matsushima, Active participation of CCR5(+)CD8(+) T lymphocytes in the pathogenesis of liver injury in graft-versus-host disease, 104 J. CLIN. INVEST. 49-57 (July 1999).)

Subsequent work assessed the role of CCR5 on donor cells in models with intensive conditioning of the recipient, in an attempt to more accurately mirror the clinical experience. These studies led to the conclusion that the role of CCR5 in allogeneic bone marrow transplants and GVHD is more complex than initially thought. In a murine transplant model with intensive conditioning. the overall effect of absent CCR5 expression on donor cells results in greater GVHD and donor T-cell expansion. (*See* Lisbeth A. Welniak, Zhao Wang, Kai Sun, William Kuziel, Miriam R. Anver, Bruce R. Blazar, William J. Murphy, An absence of CCR5 on donor cells results in acceleration of acute graft-vs-host disease, 32 EXPERIMENTAL HEMATOLOGY 318-324, ISSUE 3 (March 2004).)

Further work on this approach was temporarily abandoned until, in part, a report of the "Berlin patient" cured of HIV with BM transplant was made. The outcome of allogeneic stem-cell transplantation in a patient with HIV infection and acute myeloid leukemia, using a transplant from an HLA-matched, unrelated donor who was screened for homozygosity for the CCR5 delta32 deletion indicated a potential cure for AIDS. This experiment also provided insights on GVHD. The patient did not experience severe GVHD. Except for the presence of grade I graft-versus-host disease of the skin, which was treated by adjusting the dosage of cyclosporine, there were no serious infections or toxic effects higher than grade I during the first year of follow-up. (*See* Gero Hütter, M.D., Daniel Nowak, M.D., Maximilian Mossner, B.S., Susanne Ganepola, M.D., Arne Müßig, M.D., Kristina Allers, Ph.D., Thomas Schneider, M.D., Ph.D., Jörg Hofmann, Ph.D., Claudia Kücherer, M.D., Olga Blau, M.D., Igor W. Blau, M.D., Wolf K. Hofmann, M.D., and Eckhard Thiel, M.D., Long-Term Control of HIV by CCR5 Delta32/Delta32 Stem-Cell Transplantation, 360 N. ENG. J. MED. 692-698 (2009).)

5. CCR5 ANTAGONISTS

Small molecule CCR5 receptor antagonist include, but are not limited to, (1-[(4,6-dimethyl-5-pyrimidinyl) carbonyl]-4-[4-[2-methoxy-1(R)-4-(trifluoromethyl)phenyl]ethyl-3(S)-methyl-1-piperazinyli-4-methylpiperidine) (UK-427,857) having the structure:

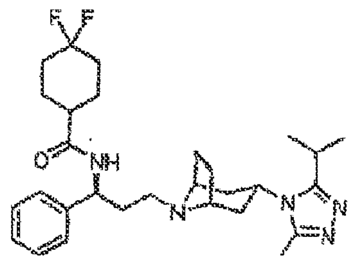

TAK-779 having the structure:

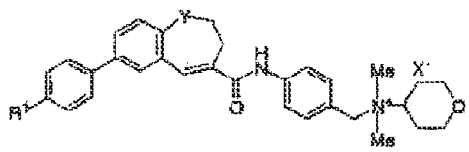

where Y = $-CH_2$, X = -C1, R1 = $-CH_3$
TAK-652 having the structure:

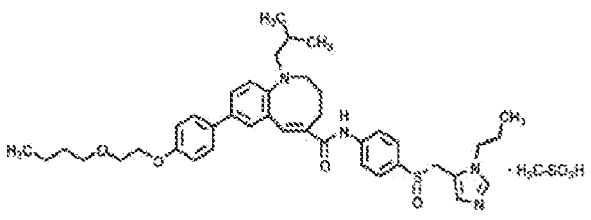

or GW873140.

A phase 1 study tested an approved CCR5 small molecule inhibitor, maraviroc (Selzentry®, Pfizer) for potential therapeutic use to prevent GVHD. Arguing that compelling data implicate the chemokine receptor CCR5 as pivotal in the pathogenesis of GVHD through control of lymphocyte trafficking, they first showed that maraviroc inhibits in vitro chemotaxis, and hypothesized that incorporating maraviroc into GVHD prophylaxis would be safe and efficacious. Their results have been reported in an abstract and are summarized below. (*See* R. Reshef, S.M. Luger, A.W. Loren, N.V. Frey, S.C. Goldstein, E.O. Hexner, E.A Stadtmauer, J. Smith, R. Mick, D.F. Heitjan, JA Hoxie, S.G. Emerson, R.H. Vonderheide, D.L. Porter, Feasibility, Safety and Efficacy of Maraviroc, a CCR5 Antagonist, in Graft-Versus-Host Disease Prevention After Reduced intensity Conditioned (RIC) Allogeneic Stem Cell Transplant (SCT) a Phase I/II Study, BIOLOGY OF BLOOD AND MARROW TRANSPLANTATION, Volume 17, Issue 2, Supplement, Page S331, February 2011.)

In a phase I trial to determine feasibility, safety and the appropriate dose of maraviroc, and to determine efficacy when combined with standard GVHD prophylaxis after RIC SCT, 21 pts received fludarabine and IV busulfan (6.4 mg/kg) followed by PBSCT from matched related (6), matched unrelated (12) and 1-antigen mismatched unrelated (3) donors. In addition to standard tacrolimus/methotrexate, maraviroc at 2 dose levels (150 and 300 mg bid) was given from day -2 to +30.

The median age was 61 (range 21-74). Indications for SCT were AML/MDS (10), NHL (4), MF (3), CLL, aplastic anemia, Hodgkin lymphoma and myeloma (1 each), maraviroc was well tolerated. 3/6 pts at the 150 mg bid dose level did not reach the targeted Cavg (100 ng/ml), while the 300 mg bid dose level resulted in adequate Cavg in 6/6 pts and was used as the phase II dose. The median time to ANC > 500/$\mu$L was 13d (range 10-21) and to plt > 20k/$\mu$L was 13d (range 11-24) with no primary graft failures. The median whole blood donor chimerism at day 100 was 97% (range 83-100).

In this high-risk population, the cumulative incidence of acute GVHD grade II-IV and III-IV at day 100 is 18% (+/-9.8%) and 6% (+/-5.9%) in the 100 mg and 300 mg doses, respectively. Importantly, by day 100 all cases of GVHD involved only the skin. At a median follow up of245 days, 4/21 pts relapsed (2 AML, 1 NHL, 1 MF) and 7/21 pts died (3 disease-related, 1 GVHD, 1 sepsis, 1 SOS, 1 unrelated).

Biological activity of maraviroc was assessed in pharmacodynamic assays, which test the capacity of patient serum to inhibit CCR5 internalization and chemotaxis. Patient serum from multiple time points prevented internalization of CCR5 by RANTES. In vitro chemotaxis of normal donor T-cells in response to RANTES was significantly impaired following incubation in patient serum from day 0 of transplant (on maraviroc) as compared to day 60 (off maraviroc).

These results led them to conclude that maraviroc 300mg bid was well tolerated and biologically active. Patients

exhibited limited GVHD without excessive relapses or engraftment failures. Inhibition of lymphocyte trafficking represents a promising strategy to reduce acute GVHD. They are currently conducting a phase II trial with an estimated completion date of February 2016

A human proof of concept Phase 2 study is underway with maraviroc. However, these data are not scheduled to be available until at least 2016. Nonetheless, investigator interest in potential applications for use of CCR5 antagonists including, but by no means limited to maraviroc, to help prevent, treat, and mitigate the effects ofGVHD.

6. CCR5 BLOCKADE AND DEVELOPMENT ANTI-CCR5 ANTAGONISTS FOR HIV-1 THERAPY

Infection of cells by human immunodeficiency virus type 1 (HIV-1) is mediated by the viral envelope (Env) glycoproteins gp120 and gp41, which are expressed as a noncovalent, oligomeric complex on the surface of virus and vitally infected cells. Entry of the virus into target cells proceeds through a cascade of events at the cell surface that include (1) binding of the viral surface glycoprotein gp120 to a cell surface receptor, (2) Env binding to fusion coreceptors, and (3) multiple conformational changes in gp.sup.41.

The first high-affinity interaction between the virion and the cell surface is the binding of gp120 to cell surface CD4, which is the primary receptor for HIV-1 (Dalgleish et al.; 1984; Klatzmarm et al., 1984; Maddon et al., 1986; McDougal et al., 1986). This binding induces conformational changes in gp120, which enable it to interact with one of several chemokine receptors (Berger, 1997; Bieniasz et al., 1998; Dragic et al., 1997; Littman, 1998). The CC-chemokine receptor 5 (CCR5) is the major co-receptor for macrophage-tropic (R5) strains, and plays a crucial role in the transmission of HIV-1 (Berger. 1997; Bieniasz et al., 1998; Dragic et al., 1997; Littman, 1998). T cell line-tropic (X4) viruses use CXCR4 to enter target cells, and usually, but not always, emerge late in disease progression or as a consequence of virus propagation in tissue culture. Some primary HIV-1 isolates are dual-tropic (R5X4) since they can use both co-receptors, though not always with the same efficiency (Connor et al., 1997; Simmons et al., 1996). Binding of gp120 to a chemokine receptor in turn triggers conformational changes in the viral transmembrane glycoprotein gp41, which mediates fusion of the viral and cellular membranes.

Each stage of this multi-step process can be blocked with inhibitors of the appropriate viral or cellular protein, and the inhibitors of gp120, gp41, CD4 and coreceptor are collectively known as entry inhibitors. Entry inhibitors represent at least 4 distinct classes of agents based on their molecular targets and determinants of viral resistance (Olson and Maddon, 2003). Table 1 lists HIV-1 entry inhibitors known to be in clinical development or approved for clinical use.

PRO 542 is a tetravalent, third-generation CD4-IgG2 fusion protein comprising the D1D2 domains of CD4 genetically fused to the heavy and light chain constant regions of human IgG2 (Allaway et al., 1995; Zhu et al., 2001). This agent binds the HIV-1 envelope glycoprotein gp120 with nanomolar affinity and may inhibit virus attachment both by receptor blockade and by detaching gp120 from the virion surface, thereby irreversibly inactivating the virus.

Table 1. HIV-1 entry inhibitors

| Compound | Molecular Class | Target | Stage of Entry | Developer |
|---|---|---|---|---|
| PRO542 | CD4-Ig Fusion Protein | ppl20 | Attachment | Progenies |
| BMS-488043 | Small Molecule | gp120 | Attachment | Bristol-Myers Squibb |
| TNX-355 | Humanized antibody | CD4 | Post-Attachment | Tanox |
| PRO 140 | Humanized antibody | CCR5 | Coreceptor | Progenies |
| CCR5mAb004 | Human antibody | CCR5 | Coreceptor | Human Genome |
| Sciences | | | | |
| SCH-D (vicriviroc) | Small Molecule | CCR5 | Coreceptor | Schering-Plough |
| | | | | |
| UK-427,857 (maraviroc) | Small Molecule | CCR5 | Coreceptor | Pfizer |
| GW873140 | Small Molecule | CCR5 | Coreceptor | GlaxoSmithKline |
| TAK-652 | Small Molecule | CCR5 | Coreceptor | Takeda |
| AMD070 | Small Molecule | CXCR4 | Coreceptor | AnorMed |
| T-20 (enfuvirtide) | Peptide | gp41 | gp41 Fusion | Trimeris/Roche |

BMS-488043 is an optimized analog of BMS-378806 (see PCT International Publication Nos. WO 01/62255

A1 and WO 03/082289 A1), which has been variously reported to block gp120 attachment to CD4 (Lin et al., 2002; 2003) and post-attachment events (Si et al., 2004).

TNX-355 is a humanized IgG4 version of the anti-CD4 monoclonal antibody (mAb) 5A8, which blocks fusion events that occur post-attachment of gp120 to CD4 (Burkly et al., 1992; Moore et al., 1992).

PRO 140, a humanized anti-CCR5 mAb, and the small-molecule CCR5 antagonists, SCH-D (also now designated SCH 417670 or vicriviroc), UK-427,857 (also designated maraviroc) and GW873140, are discussed below.

CCR5mAb004 is a fully human mAb, generated using the Abgenix XenoMouse .RTM. technology, that specifically recognizes and binds to CCR5 (Roschke et al., 2004). CCR5mAb004 has been reported to inhibit CCRS-dependent entry of HIV-1 viruses into human cells, and recently entered Phase 1 clinical trials (HGS Press Release, 2005).

The first small-molecule anti-CCRS antagonist identified as capable of inhibiting HIV-I infection was TAK-779 (Baba et aL, 1999). However, TAK-779 exhibited poor oral bioavailability (Baba et al., 2005) and local injection site irritation (Iizawa et al., 2003), and has been replaced in clinical development by a TAK-779 derivative, TAK-652 (Baba et al., 2005). TAK-652 is an orally bioavailable CCR5 antagonist with potent anti-HIV-1 activity in the nanomolar range *in vitro* and promising pharmacological profiles *in vivo* (Baba et al., 2005).

AMD070 is a second-generation CXCR4 inhibitor; the first-generation CXCR4 inhibitor AMD3100 did not demonstrate a favorable safety window for HIV-1 therapy (Schols et al., 2002).

Finally, T-20 was approved for salvage therapy of HIV-1 infection following favorable antiviral and safety profiles in each of two pivotal Phase 3 studies (Lalezari et al., 2003; Lazzarin et al., 2003).

CCR5 as a Target for Anti-HIV-1 Therapy

As first demonstrated in 1986, HIV-1 binds to target cells via the CD4 receptor but requires additional host cell factors to mediate entry (Maddon et aL, 1986). Over the next decade, a number of candidate coreceptors were proposed, but none reproducibly mediated viral entry when coexpressed with CD4 in otherwise nonpermissive cells. However, in 1996, certain chemokine receptors, mainly CCR5 and CXCR4, were shown to serve as requisite fusion coreceptors for HIV-1.

Cocchi et al. (1995) provided the first link between HIV-1 and chemokines, which are small (.about.8 kDa) homologous soluble proteins. Chemokines mediate the recruitment and activation of immune cells. They are classified as CC-, CXC-, CX.sub.3C- and XC-chemokines based on the number and sequential relationship of the first two of four conserved cysteine residues; most are either CC- or CXC-chemokines. The CC-chemokines RANTES, MIP-1.alpha. and MIP-1.beta., were shown to block replication of primary macrophage-tropic strains of HIV-1 (Cocchi et al., 1995). Using expression cloning techniques, Feng et al. (1996) discovered that the chemokine receptor fusin (later renamed CXCR4) was a fusion coreceptor for strains of HIV-1 adapted to growth on T cell lines. Shortly thereafter, several groups reported the cloning of CCR5, a CC chemokine receptor with specificity for RANTES, MIP-1.alpha. and MIP-1.beta. (Combadiere et al., 1996; Raport et al., 1996; Samson et al., 1997), and others then demonstrated that CCR5 was the main entry cofactor used by primary macrophage-tropic HIV-1 isolates (Alkhatib et al., 1996; Choe et al., 1996; Deng et al., 1996; Doranz et al., 1996; Dragic et al., 1996). The patterns of CCR5 and CXCR4 expression helped solve longstanding riddles concerning the tropism of different strains of HIV-1. Macrophage-tropic, T-cell-line-tropic and dual-tropic viruses could be more descriptively classified as being R5, X4 and R5X4 viruses based on their abilities to utilize CCR5, CXCR4 or both receptors, respectively, for entry.

A variety of other chemokine receptors can function as HIV-1 coreceptors when over-expressed *in vitro*. The list includes CCR8, Apj, V28, US28, CCR2b, CCR3, gprl, Bonzo (STRL33, TYMSTR), and BOB (gpr15). Clearly, proteins belonging to the chemokine receptor family have biochemical properties that promote HIV-1 membrane fusion. However, most of the above-mentioned coreceptors are not very efficient, are not normally coexpressed with CD4, and function only with certain strains of HIV-1, HIV-2 or SIV. The *in vivo* relevance of these alternative coreceptors has not been established.

Several factors make CCR5 an attractive target for new antiretroviral therapies. CCR5 plays a central role in HIV-1 transmission and pathogenesis, and naturally-occurring mutations in CCR5 confer protection from HIV-1 infection and disease progression. The most notable CCR5 polymorphism involves a 32 bp deletion in the coding region of CCR5 (A32) (Liu et aL, 1996). The A32 allele encodes a nonfunctional receptor that fails to reach the cell surface. Individuals who possess one normal and one mutant CCR5 gene express lower levels of CCR5, and their T cells are less susceptible to R5 virus infection *in vitro* (Liu et aL, 1996; Wu et al, 1997). A32 heterozygotes experience a milder course of disease characterized by reduced viral burdens and delayed progression to AIDS (Huang et aL, 1996; Michael et aL, 1997). These results support the concept that reducing CCR5 availability can lower viral replication and slow disease progression.

Individuals with two mutant CCR5 genes comprise a significant fraction of people of northern European descent; the demography is suggestive of a prior pandemic of a CCR5-using pathogen. Such individuals represent human CCR5 "knockouts" in that they do not express a functional CCR5 protein. Except in rare instances (Balotta et aL, 1997; Biti et al., 1997; O'Brien et aL, 1997), these individuals are resistant to HIV-1 infection (Huang et al., 1996; Liu et al., 1996; Michael et al., 1997; Samson et al., 1997), and their T cells cannot be infected with R5 viruses *in vitro* (Liu et al., 1996). These findings underscore the central role of CCR5 in HIV-1 transmission. In fact, it is now known that R5 viruses

mediate transmission in nearly all cases and mediate progression to AIDS in most cases.

Importantly, individuals who lack CCR5 enjoy normal health and display no obvious immunologic or other defects. This may reflect the redundancy of chemokine signaling pathways and the rather limited pattern of expression of CCR5. CCR5 expression is largely confined to activated T cells and macrophages, which represent the primary targets for HIV-1 infection *in vivo*, although low-level CCR5 expression has been reported on other tissues, such as smooth muscle (Schecter et al., 2000).

CCR5 knockout mice have been generated and provide further insight into the effects of abrogating CCR5 function. CCR5 knockout mice develop normally and are ostensibly healthy, although minor alterations in immune responses can be observed upon challenge with particular pathogens (Huffnagle et aL, 1999; Schuh et al., 2002; Tran et al., 2000; Zhou et aL, 1998). In contrast, the CXCR4 knockout is a lethal phenotype in mice (Lapidot et al., 2001), and has not been observed in humans.

Taken together, these genetic analyses strongly support a new therapeutic approach based on CCR5 as a drug target The error-prone nature of reverse transcriptase generates immense genetic diversity that fosters the development of drug-resistant isolates, and HIV-1's ability to utilize multiple fusion coreceptors provides one path to resistance. Drug-resistant viruses have been isolated for all marketed antiretrovirals, which nevertheless provide important therapeutic benefit when used in appropriate combinations. Thus, despite the potential emergence of drug-resistant viruses, CCRS-targeting agents may serve as a new treatment paradigm for HIV-1 infection.

Although the apparent non-essential nature of CCR5 suggests that CCR5 antagonists may be well tolerated *in vivo,* further studies are required to determine that long-term effects of abrogating CCR5 function in individuals whose immune systems developed in its presence. Such potentially deleterious effects may be mitigated by use of agents that bind to CCR5 and inhibit binding of HIV-1 thereto, but do not impair normal CCR5 function. One agent demonstrated to have such properties is the humanized anti-CCR5 mAb, PRO 140, which effectively blocks HIV-1 replication at concentrations that do not inhibit the physiologic activity of CCR5 (Olson et al., 1999). PRO 140 was identified using a fluorescence resonance energy transfer (RET) assay screen for anti-HIV activity. It is potently antiviral, having an IC.sub.90 of about 4 .mu.g/ml (Olson et al., 1999; Trkola et al., 2001) and protects diverse primary target cell types (Ketas et al., 2003; Olson and Maddon, 2003). Repeated administration of PRO 140 led to prolonged control of HIV-1 replication without viral escape in the hu-PBL SCID mouse model, and PRO 140 is currently in Phase 1 human clinical trials.

Subsequent to the identification of the small-molecule CCR5 antagonist, TAK-779 (Baba et al., 1999), several other small-molecule CCR5 antagonists have been identified. Four of these (SCH-C, SCH-D, UK-427,857, GW873140) have completed similarly designed Phase 1 studies in HIV-infected individuals (Reynes et al., 2002; Schurmarm et al., 2004; Dorr et al., 2003; Lalezari et al., 2004). Each of these agents mediated dose-dependent .about.1 log.sub.10 mean reductions in HIV-1 RNA levels during the treatment period of 10-14 days. As expected, viral loads rebounded to baseline levels following cessation of therapy. The most common drug-related side-effects were neurologic (headache, dizziness) and gastrointestinal (nausea, diarrhea, flatulence), and these were not dose limiting. With the exception of SCH-C (Reyes et al., 2001), none of the above-identified agents induced clinically significant changes in QTc intervals.

A double-blind, placebo-controlled, single oral dose study has also been conducted to evaluate the safety, tolerability, and pharmacokinetics ofTAK-652, the successor compound to TAK-779, in healthy male volunteers (Baba et al., 2005). The single administration ofTAK-652 solution was reportedly safe and well tolerated (Baba et al., 2005).

Overall, these studies provide preliminary validation of CCR5 as a target for HIV-1 therapy. While the small-molecule CCR5 antagonists represent patentably distinct chemical series with differing pharmacokinetic and metabolic properties, the compounds share many properties in their inhibition of CCR5 function, binding site on CCR5, resistance profiles, and dosing regimen. These similarities may conceivably limit the number of genuine treatment options afforded by small-molecule CCR5 antagonists. Moreover, it remains to be determined whether there are untoward consequences of chronic blockade of CCR5 function, and the utility of small-molecule CCR5 antagonists for HIV-1 therapy remains to be established by demonstration of appropriate safety and efficacy in Phase 3 clinical studies.

Monoclonal Antibody Therapeutics

In recent years, mAb products have provided new standards of care in diverse disease settings. Currently, 18 mAbs are approved by the U.S. Food and Drug Administration (FDA) for indications including cancer, autoimmune disease, transplant rejection and viral infection. Notably, 14 mAbs have been approved since 2000. In many instances, mAbs provide safety, efficacy and ease-of-use profiles that are unrivalled by small-molecule compounds. Examples include Synagis (MedImmune, Inc., Gaithersburg, Md.), a humanized mAb to respiratory syncytial virus (RSV), and Rituxan (Genentech, San Francisco, Calif.), an anti-CD20 mAb that provides the standard of care for non-Hodgkin's lymphoma.

The humanized anti-CCR5 mAb, PRO 140, is structurally, functionally and mechanistically distinct from the small-molecule CCR5 antagonists and therefore represents a unique CCR5 inhibitor class. PRO 140 is a humanized version of the murine mAb, PA14, which was generated against CD4.sup.+CCR5.sup.+ cells (Olson et al., 1999). PRO

140 binds to CCR5 expressed on the surface of a cell, and potently inhibits HIV-1 entry and replication at concentrations that do not affect CCR5 chemokine receptor activity *in vitro* and in the hu-PBL-SCID mouse model of HIV-1 infection (Olson et al., 1999; Trkola et al., 2001). The latter finding provides *in* vivo proof-of-concept for PRO 140 anti-HIV-1 therapy, and PRO 140 is currently undergoing Phase 1a clinical studies.

Important differences between PRO 140 and small-molecule CCR5 antagonists are summarized in Table 2. It is evident from Table 2 that, whereas small-molecule CCR5 antagonists in development share many properties, PRO 140 is clearly distinct from these small-molecule inhibitors. The differences between the two CCR5 inhibitor classes reveal that PRO 140 may offer a fundamentally distinct, and in many ways complementary, product profile from that of small-molecule CCR5 antagonists. Indeed, PRO 140 represents a novel therapeutic approach to treating HIV-1 infection and could play an important role in HIV-1 therapy irrespective of whether small-molecule CCR5 antagonists are ultimately clinically approved.

Synergistic Inhibition of HIV-1 Infection by Different Classes of Inhibitors

Synergistic inhibition of HIV-1 entry has previously been demonstrated using certain anti-Env antibodies in combination with other anti-Env antibodies (Thali et al., 1992; Tilley et al., 1992; Laal et al., 1994; Vijh-Warrier et al., 1996; Li et al., 1997; Li et al., 1998), anti-CD4 antibodies (Buddy et al., 1995), or CD4-based proteins (Allaway et al., 1993). Similarly, synergies have been observed using anti-CCR5 antibodies in combination with other anti-CCRS antibodies, CC-chemokines, or CD4-based proteins (Olson et al., 1999). Prior studies described in PCT International Publication No. WO 00/35409, published Jun. 22,2000, examined combinations of HIV-1 attachment inhibitors and CCR5 coreceptor inhibitors. Prior studies described in PCT International Publication No. WO 01/55439, published Aug. 2, 2001, examined combinations of inhibitors of gp41 fusion intermediates and HIV-1 attachment. Prior studies described in PCT International Publication No. WO 02/22077, published Mar. 21, 2002, examined combinations of fusion inhibitors and CCR5 coreceptor inhibitors, as well as the triple combination of fusion inhibitors, CCR5 coreceptor inhibitors and HIV-1 attachment inhibitors. However, no prior study has examined the combination of different classes of CCR5 coreceptor inhibitors, such as anti-CCR5 mAbs and non-antibody CCR5 antagonists.

Table 2. Comparison of PRO 140 and small-molecule CCR5 antagonists under development

| | Small Molecules | PRO 140 |
|---|---|---|
| Identification Screen | Chemokine Binding | HIV-1 Entry |
| Block Natural Activity of CCR5 | Yes | No |
| Potential for Immune Suppression/ Dysregulation | Yes | No |
| Tolerability | Cardiac, Neurological Toxicities for some | No Toxicity |
| Binding site on CCR5 | Common Hydrophobic Pocket defined by Transmembrane Regions of CCR5 | Extracellular Epitope that spans Multiple Hydrophilic Domains |
| Viral Cross-Resistance | Significant | Limited |
| Development of Resistance *In Vitro* | 6 to 19 weeks | None at 40 weeks |
| Drug-Drug Interactions | Significant | Unlikely |
| Food Interactions | Significant | Unlikely |
| Dosing | Once or Twice Daily | Biweekly to Monthly |

SUMMARY OF THE INVENTION

This invention relates to the use of CCR5 receptor antagonist monoclonal antibodies and, possibly, other compounds to prevent, treat, or mitigate the effects of graft-versus-host disease (GVHD). In one embodiment, this invention includes the use of PRO 140, a known CCR5 receptor antagonist, either separately or together with other compounds to prevent, treat, or mitigate the effects of GVHD. In another embodiment, PRO 140 is used together with at least one other compound to achieve a synergistic effect to prevent, treat, or mitigate the effects of GVHD.

Because PRO 140 is a known CCR5 cell receptor antagonist, and has application, like maraviroc, to effectively block HIV-1 cell entry via the CCR5 cell receptor, PRO 140 may also have therapeutic applications for the prevention,

treatment, or mitigation of GVHD, like maraviroc. PRO 140 and maraviroc are known to directly bind to the CCR5 cell receptor in different but necessarily neighboring locations, and ample evidence suggests that PRO 140's and maraviroc's mechanisms of action are complementary and, potentially, synergistic with respect to preventing certain CCR5 cell receptor activities. Further, because dual exposure of CCR5 receptors to both PRO 140 and maraviroc has been demonstrated to result in CCR5 cell receptor binding that results in synergistic HIV-1 cell entry blockade, it may be that such a combination of compounds may be similarly brought to bear to prevent, treat, or mitigate the effects of GVHD.

Indeed, it is contemplated that maraviroc-bound CCR5 receptors, upon exposure to PRO 140, may form a ternary complex that effectively prevents or reduces CCR5 activity as such relates to GVHD. While on the topic of imposing structural or chemical interference at the CCR5 cell receptor to prevent, treat, or mitigate the effects of GVHD, it is also noted that extended life maraviroc conjugates, including PEGylated compounds and chemically programmed antibodies, have been made without destroying maraviroc's binding capability. Accordingly, it is contemplated that maraviroc provides sufficient flexibility to exercise therapeutic effect despite, and perhaps further to, the immediate presence of neighboring CCR5 cell receptor binding entities. To this end, multiple CCR5 cell receptor binding agents, including any of antibodies or fragments thereof, proteins or fragments thereof, and small molecules, may be effectively, or most effectively used in concert.

CCR5 receptor antagonists generally include antibodies and fragments thereof proteins and fragments thereof, and small molecule inhibitors or drugs. Antibodies and other protein therapeutics have some advantages and disadvantages when compared to small molecule inhibitors or drugs. Since antibodies and other protein therapeutics are often formulated for IV administration there may be an inconvenience caused by intravenous dose. However, because most transplant recipients have indwelling long-term Hickman catheters, any inconvenience due to IV administration is reduced. Because antibodies and other protein therapeutics typically have longer circulating half-lives in vivo, the frequency of dosing may be reduced. Accordingly, patient compliance with regard to maintaining their dosing regimes would likely be better than for orally dosed therapeutics which often require daily, or multiple daily, dosings. Further, because some antibiotics may be accompanied by few or no side effects, a patients adherence to a regular dosing regimen may be further improved relative to other therapeutics that give rise to unpleasant side effects such as nausea. Small molecule therapies frequently have off target effects, which is less common in antibody therapies. For example, the adverse event profile of maraviroc is well described in HIV patients and includes diarrhea, nausea, fatigue, and headache. It is noted that, for some patients with GVHD, interpretation of adverse event causality could be problematic because these symptoms may associated with GVHD. Moreover, in the case of GVHD, the subject patient population may suffer from damaged bowels resulting in maladsorption and reduced effectiveness of orally ingested therapeutics, in which case an antibody, such as a monoclonal antibody, e.g., PRO 140, may provide superior pharmacodynamics coverage.

This method provides a method for reducing GVHD in a human subject which comprises administering to the subject at a predefined interval effective GVHD-reducing doses of (a) a humanized antibody designated PRO 140, or of (b) an and-CCR5 receptor monoclonal antibody which (i) binds to CD4+CCR5+ cells in the subject and inhibits fusion with such cells, (ii) inhibits fusion with CD4+CCR5+ cells with a potency equal or greater than that of PRO 140, (iii) coats CD4+CCR5+ cells in the subject without reducing the number of such cells in the subject, and/or (iv) binds to the subject's CD4+CCR5+ cells without inducing an increase in the subject's plasma concentration of circulating.beta.-chemokines, wherein PRO 140 comprises (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:HuPRO140 HG2-VH (ATCC Deposit Designation PTA-4098) or the plasmid designated pVg4HuPR0140 (mut B+D+I)-VH (ATCC Deposit Designation PTA-4099), wherein the effective GVHD reducing dose comprises from 0.1 mg per kg to 10 mg per kg of the subject's body weight, so as to thereby reduce the subject's GVHD.

This invention also provides a method for inhibiting GVHD in a human subject or the onset or progression of an GVHD associated disorder, the inhibition of which is effected by inhibiting fusion to CCR5.sup.+CD4.sup.+ target cells in the subject, comprising administering to the subject at a predefined interval effective fusion-inhibitory doses of a humanized antibody designated PRO 140, or of an anti-CCR5 receptor antibody which (i) binds to CD4+CCR5+ cells in the subject and inhibits fusion with such cells, (ii) inhibits fusion with the subject's CD4+CCR5+ cells with a potency characterized by an IC90 of 10 .mu.g/ml or less, (iii) coats the subject's CD4+CCR5+ cells without reducing the of such cells in the subject, and/or (iv) binds to the subject's CD4+CCR5+ cells without inducing an increase in the subject's plasma concentration of circulating .beta.-chemokines, wherein PRO 140 comprises (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:HuPRO140 HG2-VH (ATCC Deposit Designation PTA-4098) or the plasmid designated pVg4:HuPRO140 (mut B+D+I)-VH (ATCC Deposit Designation PTA-4099), wherein each administration of the antibody delivers to the subject from 0.1 mg per kg to 10 mg per kg of the subject's body weight, so as to thereby inhibit the onset or progression of the GVHD-associated disorder in the subject

This invention further provides a method for reducing the likelihood of a human subject's developing GVHD

which comprises administering to the subject at a predefined interval effective fusion-inhibitory doses of a humanized antibody designated PRO 140, or of an anti-CCR5 receptor antibody which (i) binds to CD4+CCR5+ cells in the subject and inhibits fusion with such cells, (ii) inhibits fusion with the subject's CD4+CCR5+ cells with a potency characterized by an IC90 of 10.mu.g/ml or less, (iii) coats the subject's CD4+CCR5+ cells without reducing the number of such cells in the subject, and/or (iv) binds to the subject's CD4+CCR5+ cells without inducing an increase in the subject's plasma concentration of circulating .beta.-chemokines, wherein PRO 140 comprises (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:HuPRO140 HG2-VH (ATCC Deposit Designation PTA-4098) or the plasmid designated pVg4:HuPRO140 (mut B+D+I)-VH (ATCC Deposit Designation PTA-4099), wherein each administration of the antibody delivers to the subject from 0.1 mg per kg to 10 mg per kg of the subject's body weight, so as to thereby reduce the likelihood of the subject's developing GVHD.

The present invention provides a method for treating a subject with GVHD comprising administering to the subject (a) an antibody which (i) binds to a CCR5 receptor on the surface of a CD4.sup.+ cell and (ii) inhibits fusion to a CCR5.sup.+CD4+ cell, and (b) a non-antibody antagonist of a CCR5 receptor, in amounts effective to treat the subject

This invention also provides a method for inhibiting in a subject the onset or progression of a GVHD-associated disorder, the inhibition of which is effected by inhibiting fusion to CCR5.sup.+CD4.sup.30 target cells in the subject, comprising administering to the subject (a) an antibody which (i) binds to a CCR5 receptor on the surface of a CD4.sup.+ cell and (ii) inhibits fusion to a CCR5.sup.+CD4+ cell, and (b) a non-antibody antagonist of a CCR5 receptor, in amounts effective to inhibit fusion to the CCR5.sup.+CD4+ target cells, so as to thereby inhibit the onset or progression of GVHD in the subject.

The invention further provides a method for reducing the likelihood of a subject's developing GVHD comprising administering to the subject (a) an antibody which (i) binds to a CCR5 receptor on the surface of a CD4.sup.+ cell and (ii) inhibits fusion to a CCR5.sup.+CD4+ cell, and (b) a non-antibody antagonist of a CCR5 receptor, in amounts effective to reduce the likelihood of the subject's developing GVHD.

This invention also provides a method of potentiating GVHD inhibitory activity of (i) an anti-CCR5 receptor monoclonal antibody or (ii) a non-antibody CCR5 receptor antagonist in the treatment of GVHD in a subject, comprising: administering to the subject an inhibitory activity potentiating amount of the anti-CCR5 receptor monoclonal antibody in combination with a GVHD inhibitory activity potentiating amount of a non-antibody CCR5 receptor antagonist, wherein the combination produces a synergistic effect on inhibiting GVHD, thereby potentiating the inhibitory activity of (i) the anti-CCR5 receptor monoclonal antibody or (ii) the non-antibody CCR5 receptor antagonist. In one embodiment, due to the synergistic effect the non-antibody CCR5 receptor antagonist causes an approximately 4- to 10-fold dose reduction of the anti-CCR5 receptor monoclonal antibody and the anti-CCR5 receptor monoclonal antibody causes an approximately 3- to 16-fold dose reduction of the non-antibody CCR5 receptor antagonist.

## BRIEF DESCRIPTION OF THE SEVERAL VIEWS OF THE DRAWINGS

The accompanying Figures, which are incorporated in and constitute a part of this specification, illustrate various exemplary embodiments.

Throughout this application, various publications are referenced in parentheses by author name and date, or by a patent or patent publication number. Full citations for these publications may be found at the end of the specification immediately preceding the claims. The disclosures of each of these publications in its entirety are hereby incorporated by reference into this application in order to more fully describe the state of the art as known to those skilled therein as of the date of this application.

FIG. 1: Humanized PRO 140 is patently antiviral The *in vitro* neutralization activity of murine and humanized PRO 140 was tested against four primary R5 HIV-1 isolates using a whole virus replication assay. The data reflect the median values from 8 or more independent assays. The genetic subtypes of the viruses are indicated in parentheses.

FIG. 2: Antiviral activity is independent of target cell Inhibition of infection of four different target cells by three primary R5 HIV-1 isolates with was tested.

FIG. 3: *In vitro* HIV-1 susceptibility to PRO 140 quantified using the PhenoSense.TM. entry assay. PRO 140 was tested for activity against 20 primary HIV-1 isolates in the PhenoSense HIV Entry.TM. assay at ViroLogic, Inc. Drug susceptibility is reported as IC.sub.50 values, which represent the concentration required for 50% inhibition of viral infectivity.

FIG. 4: PRO 140 blocks HIV-1 but not chemokine signaling. The effects of PRO 140 on the inhibition of RANTES-induced calcium mobilization in L1.2-CCR5 cells and on inhibition of HIV-1.sub.JR-FL replication in PBMC cultures were determined. Similar results were obtained for MIP-1.alpha. and MIP-1.beta..

FIG. 5: PRO 140 provides prolonged control of viral replication in HIV-1-infected mice. SCID mice were reconstituted

with normal human peripheral blood mononuclear cells and infected 2 weeks later with HIV-1.sub.JR-CSF. Multiple doses of PRO 140 were administered following attainment of steady state viral levels. Plasma viral loads pre- and post-injection are indicated.

FIG. 6: PRO 140 coats but does not deplete CCR5 lymphocytes. Healthy male volunteers (n=4) were treated with a single intravenous infusion of PRO 140 at a dose level of 2 mg/kg. At the indicated times post-treatment, blood was collected and analyzed for CCR5 lymphocyte levels. The group mean values and standard deviations are indicated.

FIG. 7: Serum concentrations of PRO 140. Healthy male volunteers were treated with a single intravenous infusion of PRO 140 at dose levels of 0.1, 0.5 and 2.0 mg/kg, as indicated. At the indicated times post-treatment, serum was collected, cryopreserved, and analyzed for PRO 140 levels. Data for individual patients are indicated.

FIG. 8: PRO 140 does not affect plasma chemokine levels. Healthy male volunteers were treated with a single intravenous infusion of 0.1 mg/kg PRO 140 (Cohort 1), 0.5 mg/kg PRO 140 (Cohort 2) or matched placebo. At the indicated times post-treatment, plasma was collected, cryopreserved and analyzed for levels of RANTES. The Lower Limit of Quantification of the assay was 415 pg RANTES/mL plasma. Data represent the group mean values.

FIG. 9: Scheme for chemical synthesis of SCH-D.

FIG. 10: Scheme for chemical synthesis of TAK-779. The method is as described in Shiraishi et al., 2000.

FIG. 11: Scheme for chemical synthesis of UK-427,857. The method is as described in PCT International Publication No. WO 01/90106 A2, published Nov. 29, 2001.

FIG. 12: Synergistic inhibition of HTV-1 fusion exhibited by PRO 140 with different compounds. Interactions between PRO 140 and small-molecule, peptide, mAb, and chimeric CD4-immunoglobulin inhibitors of CCR5, CD4, gp120 and gp41 targets for inhibiting HIV-1 fusion were assessed using the RET assay. Mean combination index (CI) values with 95% confidence intervals are plotted for data obtained using the compounds combined in a 1:1 molar ratio. A CI value of <1 indicates synergistic interactions; a CI value of 1 indicates additive interactions; and a CI value of >1 indicates antagonistic interactions.

FIG. 13: PRO 140 coats but does not deplete lymphocytes. Healthy male volunteers (n=4) were treated with a single intravenous infusion of PRO 140 at a dose level of 5 mg/kg. At the indicated times post-treatment, blood was collected and analyzed for CCR5 lymphocyte levels. The group mean values and standard deviations are indicated.

FIG. 14: PRO 140 is active against HIV-1 strains that are resistant to small-molecule CCR5 antagonists. Variants of HIV-1 resistant to AD101 (a small-molecule CCR5 inhibitor structurally related to SCH-C) and SCH-D (Kuhmann et al., 2004; Maroznan et al. 2005) were tested for sensitivity to the anti-CCR5 mAb, PA14. The extent of viral replication in primary CD4+ T-cells is represented relative to p24 antigen production in the absence of any inhibitor, which is defined as 100%. Individual data points were the average of values derived from 4 separate experiments, each performed using duplicate wells. The data show that whereas the AD101- and SCH-D-resistant HIV-1 variants were resistant to SCH-C and SCH-D, respectively, replication of these variants was potently inhibited by PA14 (Maroznan et al. 2005).

FIG. 15: Dose-response curves for inhibition of HIV-1.sub.JR-FL envelope-mediated membrane fusion by combinations of CCR5 inhibitors. Dilutions were analyzed in triplicate wells, and the data points depict the mean and standard deviations of replicates. (A) PRO 140 and UK-427,857 were tested individually and in a 1:1 fixed molar ratio over the indicated range of concentrations. In the experiment depicted, IC50 and IC90 values were 2.9 nM and 11 nM for PRO140, 5.0 nM and 21 nM for UK-427,857, and 2.1 nM and 4.6 nM for the combination. CI50 and CI90 values were 0.58 and 0.32, respectively. (B) SCH-D and UK-427,857 were tested individually and in a 1:1 fixed molar ratio over the indicated range of concentrations. In the experiment depicted, IC50 and IC90 values were 5.5 nM and 34 nM for SCH-D, 9.7 nM and 59 nM for UK-427,857, and 6.1 nM and 31 nM for the combination. CI50 and CI90 values were 0.87 and 0.73, respectively.

FIG. 16: Inhibition of PRO 140-PE binding to CEM.NKR-CCR5 cells by unlabeled PRO 140, UK-427,857 and SCH-D. CEM.NKR-CCR5 cells were incubated with varying concentrations of unlabeled PRO 140, UK-427,857 or SCH-D for 30 min at room temperature in PBSA buffer prior to addition of 5 nM PRO 140-PE for an additional 30 min. Cells were washed and then analyzed by flow cytometry for both the mean fluorescence intensity (MH) of binding and the percent of cells gated for positive binding of PRO 140-PE. Inhibition was assessed on the basis of both MFI (A) and percent cells gated (B).

FIG. 17: Inhibition of .sup.3H-UK-427,857 binding by unlabeled UK-427,857, SCH-D and PRO 140. (A) CEM.NKR-CCR5 cells were pre-incubated with varying concentrations of unlabeled UK-427,857, SCH-D or PRO 140 for 30 min in PBSA buffer at ambient temperature prior to the addition of at 2 nM .sup.3H-UK-427,857 for an additional 30 min. Cells were washed and then analyzed for radioactivity by scintillation counting. (B) The stability of UK-427,857 binding under the assay conditions was examined by pre-incubating CEM.NKR-CCR5 cells with 2 nM .sup.3H-UK-427,857 prior to washing, addition of unlabeled compounds for 30 min, and processing as described above.

FIG. 18: Prior art graphic depiction of CCR5 illustrating the binding sites for mAbs and small molecules. Amino acids are indicated in the single-letter code. Residues implicated in mAb binding are coded by filled symbols in the

extracellular domain or sub-domain. Short double dash = Nt; arrow = ECL1; long single dash = amino-terminal portion of ECL2; small dots = carboxy-terminal portion of ECL2. A putative binding site for maraviroc and vicriviroc in the transmembrane helices (residues W86, Y108, I198, Y251 and E283) is illustrated with dark open circles. The assignment of the seven transmembrane helices is indicated with cylinders. *See* William C. Olson and Jeffrey M. Jacobson, CCR5 Monoclonal Antibodies for HIV-1 Therapy, Curr Opin HIV AIDS. 2009 March; 4(2): 104-111.
FIG. 19: Prior art illustration of chain of events leading to GVHD. *See* Sung 2013 (Figure 1).

DETAILED DESCRIPTION OF THE INVENTION

Disclosure in this specification relating to treating an HIV-1 infection in a subject using CCR5 antagonists is provided herein as such may similarly relate to treating GVHD in a subject.

Terms

As used in this application, except as otherwise expressly provided herein, each of the following terms shall have the meaning set forth below.

"Administering" refers to delivering in a manner which is effected or performed using any of the various methods and delivery systems known to those skilled in the art Administering can be performed, for example, topically, intravenously, pericardially, orally, parenterally, via implant, transmucosally, transdermally, intradermally, intramuscularly, subcutaneously, intraperitoneal, intrathecally, intralymphatically, intralesionally, epidurally, or by *in vivo* electroporation An agent or composition may also be administered in an aerosol, such as for pulmonary and/or intranasal delivery. Administering can also be performed, for example, once, a plurality of times, and/or over one or more extended periods.

An "antibody" shall include, without limitation, an immunoglobulin molecule comprising two heavy chains and two light chains and which recognizes an antigen. The immunoglobulin molecule may derive from any of the commonly known classes, including but not limited to IgA, secretory IgA, IgG and IgM.IgG subclasses are also well known to those in the art and include but are not limited to human IgG1, IgG2, IgG3 and IgG4. "Antibody" includes, by way of example, both naturally occurring and non-naturally occurring antibodies; monoclonal and polyclonal antibodies; chimeric and humanized antibodies; human or nonhnman antibodies; wholly synthetic antibodies; and single chain antibodies A nonhuman antibody may be humanized by recombinant methods to reduce its immunogenicity in man. Methods for humanizing antibodies are well known to those skilled in the art. "Antibody" also includes, without limitation, a fragment or portion of any of the afore-mentioned immunoglobulin molecules and includes a monovalent and a divalent fragment or portion. Antibody fragments include, for example, Fc fragments and antigenbinding fragments (Fab).

An "anti-chemokine receptor antibody" refers to an antibody which recognizes and binds to an epitope on a chemokine receptor. As used herein, "anti-CCR5 antibody" refers to an antibody which recognizes and binds to an epitope on the CCR5 chemokine receptor.

"Attachment" means the process that is mediated by the binding of the HIV-1 envelope glycoprotein to the human CD4 receptor, which is not a fusion coreceptor.

As used herein, "CCR5" is a chemokine receptor which binds members of the C-C group of chemokines and whose amino acid sequence comprises that provided in Genbank Accession Number 1705896 and related polymorphic variants. As used herein, CCR5 includes, without limitation, extracellular portions of CCR5 capable of binding the HIV-1 envelope protein. "CCR5" and "CCR5 receptor" are used synonymously.

"CD4" means the mature, native, membrane-bound CD4 protein comprising a cytoplasmic domain, a hydrophobic transmembrane domain, and an extracellular domain which binds to the HIV-1 gp120 envelope glycoprotein.

"CDR" , or complementarity determining region, means a highly variable sequence of amino acids in the variable domain of an antibody.

A "cell" includes a biological cell, e.g., a HeLa cell, and a non-biological cell, e.g., a phospholipid vesicle or virion. A "cell susceptible to HIV infection" may also be referred to as a "target cell" and includes a cell capable of being infected by or fusing with HIV or an H1V-infected cell

"CXCR4" is a chemokine receptor which binds members of the C-X-C group of chemokines and whose amino acid sequence comprises that provided in Genbank Accession No 400654 and related polymorphic variants. As used herein, CXCR4 includes extracellular portions of CXCR4 capable of binding the HIV-1 envelope protein.

"Exposed" to HIV-1 refers to contact with HIV-1 such that infection could result

A "fully human" antibody refers to an antibody wherein all of the amino acids correspond to amino acids in human immunoglobulin molecules. "Fully human" and "human" are used synonymously.

"HIV" refers to the human immunodeficiency virus. HIV shall include, without limitation, HIV-1. HIV-1 includes but is not limited to extracellular virus particles and the forms of HIV-1 associated with HIV-1 infected cells. The human immunodeficiency virus (HIV) may be either of the two known types of HIV (HIV-1 or HIV-2). The HIV-1 virus may represent any of the known major subtypes (classes A, B, C, D, E, F, G and H) or outlying subtype (Group O). HIV-1.sub.JR-FL is a strain that was originally isolated at autopsy from the brain tissue of an AIDS patient The virus has been cloned and the DNA sequences of its envelope glycoproteins are known (GenBank Accession No. U63632). In terms of sensitivity to inhibitors of viral entry, HIV-1.sub.JR-FL is known to be highly representative of primary HIV-1 isolates.

A "humanized" antibody refers to an antibody wherein some, most or all of the amino acids outside the CDR regions are replaced with corresponding amino acids derived from human immunoglobulin molecules. In one embodiment of the humanized forms of the antibodies, some, most or all of the amino acids outside the CDR regions have been replaced with amino acids from human immunoglobulin molecules, whereas some, most or all amino acids within one or more CDR regions are unchanged. Small additions, deletions, insertions, substitutions or modifications of amino adds are permissible as long as they do not abrogate the ability of the antibody to bind a given antigen. Suitable human immunoglobulin molecules include IgG1, IgG2, IgG3, IgG4, IgA, IgE and IgM molecules. A "humanized" antibody retains an antigenic specificity similar to that of the original antibody.

"Monoclonal antibodies," also designated a mAbs, are antibody molecules whose primary sequences are essentially identical and which exhibit the same antigenic specificity. Monoclonal antibodies may be produced by hybridoma, recombinant, transgenic or other techniques known to those skilled in the art.

A "non-antibody antagonist of a CCR5 receptor" refers to an agent that does not comprise an antibody, and which binds to a CCR5 receptor and inhibits the activity of this receptor. Such inhibition can include inhibiting the binding of HIV-1 to the CCR5 receptor. By way of example, non-antibody antagonists include nucleic acids, carbohydrates, lipids, oligopeptides, and small organic molecules.

"Reducing the likelihood of a subject's contracting a viral infection" means reducing the likelihood of the subject's becoming infected with the virus by at least two-fold. For example, if a subject has a 1% chance of becoming infected with the virus, a two-fold reduction in the likelihood of the subject contacting a viral infection would result in the subject having a 0.5% chance of becoming infected with the virus. In the preferred embodiment of this invention, reducing the likelihood of the subject's contracting a viral infection means reducing the likelihood of the subject's becoming infected with the virus by at least tenfold.

A "small-molecule" CCR5 receptor antagonist includes, for example, a small organic molecule which binds to a CCR5 receptor and inhibits the activity of the receptor. Such inhibition includes, e.g., inhibiting the binding of HIV-1 to the receptor. In one embodiment, the small organic molecule has a molecular weight less than 1,500 daltons. In another embodiment, the molecule has a molecular weight less than 600 daltons.

"Subject" includes any animal or artificially modified animal capable of becoming infected with HIV. Animals include, but are not limited to, humans, non-human primates, dogs, cats, rabbits, ferrets, and rodents such as mice, rats and guinea pigs. Artificially modified animals include, but are not limited to, SCID mice with human immune systems. In the preferred embodiment, the subject is a human.

"Synergy" between two or more agents refers to the combined effect of the agents which is greater than their additive effects. Synergistic, additive or antagonistic effects between agents may be quantified by analysis of the dose-response curves using the Combination Index (CI) method. A CI value greater than 1 indicates antagonism; a CI value equal to 1 indicates an additive effect; and a CI value less than 1 indicates a synergistic effect. In one embodiment, the CI value of a synergistic interaction is less than 0.9. In another embodiment, the CI value is less than 0.8. In a preferred embodiment, the CI value is less than 0.7.

"Treating an HIV-1 infection in a subject" refers to slowing, stopping or reversing the progression of an HIV-1 disorder in the subject In the preferred embodiment, "treating" refers to reversing the progression to the point of eliminating the disorder. As used herein, "treating" also means reducing the number of viral infections, reducing the number of infectious viral particles, reducing the number of vitally infected cells, or ameliorating symptoms associated with HIV-1. Reducing viral load in a subject is one embodiment of treating the subject.

## Embodiments of the Invention

Various embodiments of this invention described herein relate to treating an HIV-1 infection in a subject and may be similarly applicable to treating GVHD in a subject because both HIV-1 and GVHD disease states are mediated, in part, via CCR5 cell receptors. Accordingly, throughout this application, all disclosure and references to treating an HIV-1 infection in a subject, and methods for such treatment, are intended to be understood and to similarly address and be readily relatable and applicable to treating GVHD in a subject That is, for the embodiments described herein, references to HIV-1 may be used interchangeably with references to GVHD.

In one embodiment the anti-CCRS receptor monoclonal antibody binds to the same CCR5 epitope as that to which PRO 140 binds. The anti-CCR5 receptor monoclonal antibody can be, for example, a humanized, human, or chimeric antibody. In the preferred embodiment, the antibody administered to the subject is the antibody designated PRO 140.

In one embodiment of the invention, the predefined interval is at least once weekly. In another embodiment,

the predefined interval is every two to four weeks. In a further embodiment, the predefined interval is every two weeks, or every four weeks. In a further embodiment, the predefined interval is at least once monthly, every six weeks or every eight weeks. In one embodiment, the antibody is administered via intravenous infusion. In another embodiment, the antibody is administered via subcutaneous injection. In one embodiment, the subject is treatment-naive. In the preferred embodiment, the subject is treatment-experienced.

In the methods of this invention, the antibody may be administered at the same time, concurrently, prior to the administration of the small-molecule CCR5 antagonist or subsequent to the administration of the small-molecule CCR5 antagonist. With respect to the administration of two or more agents to a subject in order to treat the subject, each agent may be administered to the subject within the same treatment time period as is each other agent The agents can be administered together, at the same time and in the same or different compositions or via the same or different routes of administration Alternatively, each agent is administered via a dosing regimen (e.g., frequency, route and amount) different from that by which each other agent is administered. For example, the first of two administered agents (e.g., an antibody) may be administered via subcutaneous injection at two-week intervals for a one-year treatment time; period, whereas during that same one-year period, the second administered agent (e.g., a small molecule) is orally administered twice per day. Accordingly, "concurrent administration" refers to the administration of at least two agents within one treatment period.

In one embodiment, the monoclonal antibody is PA14 produced by the hybridoma cell line designated PA14 (ATCC Accession No. HB-12610), or an antibody that competes with monoclonal antibody PA-14 in binding to the CCR5 receptor. In another embodiment, the monoclonal antibody is the humanized antibody designated PRO 140, or an antibody that competes with PRO 140 in binding to the CCR5 receptor, wherein PRO 140 comprises (i) two light chains, each light chain comprising the expression product of the plasmid designated pVK-HuPRO140-VK (ATCC Deposit Designation PTA 4097), and (ii) two heavy chains, each heavy chain comprising the expression product of either the plasmid designated pVg4:HuPRO140 HG2-VH (ATCC Deposit Designation PTA-4098) or the plasmid designated pVg4:HuPRO140 (mut B+D+I) (ATCC Deposit Designation PTA-4099). In another embodiment, the monoclonal antibody is the humanized antibody designated PRO140. In yet another embodiment, the monoclonal antibody is CCR5mAb004 or 2D7.

In one embodiment, the non-antibody CCR5 receptor antagonist is SCH-D, TAK-779, TAK-652, UK-427,857, RANTES, GW873140, or a combination thereof. In another embodiment, the non-antibody CCR5 receptor antagonist is a small organic molecule that competes with SCH-D in binding to the CCR5 receptor. In another embodiment, the non-antibody CCR5 receptor antagonist is a small organic molecule that competes with UK-427,857 in binding to the CCR5 receptor. In yet another embodiment, the non-antibody CCR5 receptor antagonist is a small organic molecule that competes with TAK-779 in binding to the CCR5 receptor. In one embodiment, the non-antibody CCR5 receptor antagonist is a small organic molecule that competes with TAK-652 in binding to the CCR5 receptor. In another embodiment, the non-antibody CCR5 receptor antagonist is a small organic molecule that competes with GW873140 in binding to the CCR5 receptor.

In one embodiment of any of the methods described herein, the anti-CCR5 antibody is a monoclonal antibody. In another embodiment, the antibody is a polyclonal antibody. In a further embodiment, the antibody is a humanized antibody. In a still further embodiment, the antibody is a human antibody. In an additional embodiment, the antibody is a chimeric antibody. In one embodiment, the antibody is the anti-CCR5 human antibody designated CCR5mAb004, produced by Human Genome Sciences.

Murine hybridomas secreting monoclonal antibodies PA8. PA9, PA10, PA11, PA12 and PA14 were deposited pursuant to, and in satisfaction of, the requirements of the Budapest Treaty on the International Recognition of the Deposit of Microorganisms for the Purposes of Patent Procedure (the "Budapest treaty") with the American Type Culture Collection (ATCC), 10801 University Boulevard, Manassas, Va. 20110-2209 on Dec. 2, 1998 under the following Accession Nos.: ATCC Accession No. HB-12605 (PA8), ATCC Accession No. HB-12606 (PA9), ATCC Accession No. 12607 (PA10), ATCC Accession No. HB-12608 (P11), ATCC Accession No. HB-12609 (PA12), and ATCC Accession No. HB-12610 (PA14).

In a further embodiment of the present invention, the monoclonal antibody is PA14 produced by the hybridoma cell line designated PA14 (ATCC Accession No. HB-12610), or an antibody that competes with monoclonal antibody PA14's binding to the CCR5 receptor. In a still further embodiment, the monoclonal antibody is an antibody that binds to the same epitope as that to which monoclonal antibody PA14 binds. When binding to the same epitope occurs, competitive inhibition results.

In another embodiment, the monoclonal antibody is selected from the group consisting ofPA14 produced by the hybridoma designated PA14 (ATCC Accession No. HB-12610), PA8 produced by the hybridoma designated PA8 (ATCC Accession No. HB-12605), PA9 produced by the hybridoma designated PA9 (ATCC Accession No. HB-12606), PA10 produced by the hybridoma designated PA10 (ATCC Accession No. HB-12607), PA11 produced by the hybridoma designated PA11 (ATCC Accession No. HB-12608), PA12 produced by the hybridoma designated PA12 (ATCC Accession No. HB-12609), and 2D7 (Wu et al., 1997). In a further embodiment, the monoclonal antibody is PA14.

One skilled in the art would know how to make the humanized antibodies of the subject invention. Methods for making fully human antibodies are also well known to one skilled in the art. Nucleic acids encoding heavy and light chains of the humanized PRO 140 antibody have been deposited with the ATCC. Specifically, the plasmids designated pVK-HuPRO140, pVg4-HuPRO40 (mut B+D+I) and pVg4-HuPRO140 HG2, respectively, were deposited pursuant to, and in satisfaction of, the requirements of the Budapest Treaty with the ATCC, Manassas, Va., U.S.A. 20108, on Feb. 22, 2002, under ATCC Accession Nos. PTA 4097, PTA 4099 and PTA 4098, respectively.

In a preferred embodiment of the instant methods, the monoclonal antibody is the humanized antibody designated PRO 140 or an antibody that competes with PRO 140's binding to the CCR5 receptor, wherein PRO 140 comprises (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:140 HG2-VH (ATCC Deposit Designation PTA-4098) or the plasmid designated pVg4:HuPRO140 (mut B+D+I)-VH (ATCC Deposit Designation PTA-4099). In a further embodiment, the monoclonal antibody is a humanised or human antibody that binds to the same epitope as that to which antibody PRO 140 binds. In another embodiment, the monoclonal antibody is the humanized antibody designated PRO 140. In a further embodiment, the monoclonal antibody is the human antibody designated CCR5mAb004 (Roschke et al., 2004; HGS Press Release, 2004; 2005).

In one embodiment of the methods described herein, the portion of the antibody comprises a light chain of the antibody. In another embodiment, the portion of the antibody comprises a heavy chain of the antibody. In a further embodiment, the portion of the antibody comprises an Fab portion of the antibody. In a still further embodiment, the portion of the antibody comprises an F(ab').sub.2 portion of the antibody. In an additional embodiment, the portion of the antibody comprises an Fd portion of the antibody. In another embodiment, the portion of the antibody comprises an Fv portion of the antibody. In a further embodiment, the portion of the antibody comprises a variable domain of the antibody. In a still further embodiment, the portion of the antibody comprises one or more CDR domains of the antibody. In yet another embodiment, the portion of the antibody comprises six CDR domains of the antibody.

In one embodiment of the instant methods, the antibody is administered to the subject a plurality of times and each administration of the antibody delivers from 0.01 mg per kg body weight to 50 mg per kg body weight of the antibody to the subject. In another embodiment, each administration of the antibody delivers from 0.05 mg per kg body weight to 25 mg per kg body weight of the antibody to the subject In a further embodiment, each administration of the antibody delivers from 0.1 mg per kg body weight to 10 mg per kg body weight of the antibody to the subject In a still further embodiment, each administration of the antibody delivers from 0.5 mg per kg body weight to 5 mg per kg body weight of the antibody to the subject In another embodiment, each administration of the antibody delivers from 1 mg per kg body weight to 3 mg per kg body weight of the antibody to the subject In a preferred embodiment, each administration of the antibody delivers about 2 mg per kg body weight of the antibody to the subject

In one embodiment, the antibody is administered a plurality of times, and a first administration of the antibody is separated from the subsequent administration of the antibody by an interval of less than one week. In another embodiment, the first administration of the antibody is separated from the subsequent administration of the antibody by an interval of at least one week. In a further embodiment, the first administration of the antibody is separated from the subsequent administration of the antibody by an interval of one week. In another embodiment, the first administration of the antibody is separated from the subsequent administration of the antibody by an interval of two to four weeks. In a preferred embodiment, the first administration of the antibody is separated from the subsequent administration of the antibody by an interval of two weeks. In a further embodiment, the first administration of the antibody is separated from the subsequent administration of the antibody by an interval of four weeks. In yet another embodiment, the antibody is administered a plurality of times, and a first administration of the antibody is separated from the subsequent administration of the antibody by an interval of at least one month.

In a further embodiment, the antibody is administered to the subject via intravenous infusion. In a preferred embodiment, the antibody is administered to the subject via subcutaneous injection. In another embodiment, the antibody is administered to the subject via intramuscular injection.

In one embodiment of the instant methods, the non-antibody CCR5 receptor antagonist is a small organic molecule. In another embodiment, the CCR5 receptor antagonist is selected from the group consisting of SCH-D, UK-427,857, TAK-779, TAK-652, GW873140 and RANTES. In a further embodiment, the CCR5 receptor antagonist is an agent that competes with SCH-D's binding to the CCR5 receptor. In a still further embodiment, the CCR5 receptor antagonist is an agent that competes with UK-427,857's binding to the CCR5 receptor. In another embodiment, the CCR5 receptor antagonist is an agent that competes with TAK-779's binding to the CCR5 receptor. In yet another embodiment, the CCR5 receptor antagonist is an agent that competes with TAK-652's binding to the CCR5 receptor. In a further embodiment, the CCR5 receptor antagonist is an agent that competes with GW873140's binding to the CCR5 receptor.

In an additional embodiment of the methods described herein, the CCR5 receptor antagonist is administered a plurality of times and each administration of the CCR5 receptor antagonist delivers from 0.5 mg to 2,500 mg of the

antagonist to the subject In another embodiment, each administration of the CCR5 receptor antagonist delivers from 5 mg to 1,250 mg of the antagonist to the subject In yet another embodiment, each administration of the CCR5 receptor antagonist delivers from 5 mg to 15 mg of the antagonist to the subject In a further embodiment, each administration of the CCR5 receptor antagonist delivers from 50 mg to 1,250 mg of the antagonist to the subject. In a still further embodiment, each administration of the CCR5 receptor antagonist delivers from 200 mg to 800 mg of the antagonist to the subject In another embodiment, each administration of the CCR5 receptor antagonist delivers from 300 mg to 600 mg of the antagonist

Because of their rapid clearance, small-molecule CCR5 receptor antagonists require at least daily or twice-daily dosing in order to maintain selective pressure on the virus. Table 3 summarizes the dosing regimens employed with various small-molecule CCR5 antagonists currently undergoing clinical trials. In one embodiment of the present methods, the CCR5 receptor antagonist is administered orally to the subject at least once per day. In another embodiment, the CCR5 receptor antagonist is administered orally to the subject once or twice per day. In a further embodiment, the CCR5 receptor antagonist is administered orally three or fewer times per day.

Table 3. Dosing regimens of small-molecule CCR5 receptor antagonists undergoing clinical trials

| Compound | Dosage[a] | Clinical Trial |
|---|---|---|
| SCH-D | 5-15 mg daily | Phase II |
| UK-427,857 | 300 mg daily or twice daily | Phase II and III |
| GW873140 | 50-1200 mg once daily, or 200-800 mg daily or twice daily | Phase II |

Dosages are indicated for the CCR5 antagonists at www.clinicaltrials.gov web site sponsored by the National Institute of Allergy and Infectious Diseases (NIAID). Dosage information for GW873140 was obtained from Demarest et al. (2004).

This invention further provides a composition of matter comprising (a) a monoclonal antibody (e.g., PRO 140) which (i) binds to a CCR5 receptor and (ii) inhibits GVHD, and (b) a non-antibody CCR5 receptor antagonist (e.g., any of SCH-D, UK-427,857, TAK-779, TAK-652, GW873140 and RANTES). The composition can further comprise a pharmaceutically acceptable carrier. This invention also provides a method for determining whether a monoclonal antibody (e.g., PRO 140) which (i) binds to a CCR5 receptor and (ii) inhibits GVHD, behaves synergistically with a non-antibody CCR5 receptor antagonist with respect to inhibiting GVHD, comprising determining the presence or absenoe of such synergy according to the experimental methods detailed below. Finally, this invention provides a kit for performing the instant methods comprising, in separate compartments and preferably in readily administrable forms, (a) a monoclonal antibody (e.g., PRO 140) which (i) binds to a CCR5 receptor and (ii) inhibits GVHD, and (b) a non-antibody CCR5 receptor antagonist (e.g., any of SCH-D, UK-427,857, TAK-779, TAK-652, GW873140 and RANTES). The antibody and antagonist are each preferably admixed with a pharmaceutically acceptable carrier.

The following Experimental Details are set forth to aid in an understanding of the subject matter of this disclosure as such may be extrapolated to apply to GVHD therapies, but are not intended to, and should not be construed to, limit in any way the claims which follow thereafter.

**Part I**

Materials and Methods

Compounds and mAbs

PRO 140 was prepared by expression in Sp2/0 cells using Hybridoma serum-free medium supplemented with 2 mM L-glutamine (Invitrogen, Carlsbad, Calif). Bulk mAb was clarified using a 5.0 .mu.m Depth filter (Sartorius, Goettingen, Germany) followed by passage over a 0.2 .mu.m sterilizing grade filter (Sartorius). The mAb was purified by passage first over an affinity column (MabSelect Protein A column, Amersham, Piscataway, N.J.) and then by ion exchange chromatography (SP Sepharose Cation Exchange resin, Amersham). PRO 140 was nanofiltered using a Viresolve.TM. 10 Opticap NFP capsule (Millipore, Billerica, Mass.) followed by a 0.2 .mu.m filter and concentrated/diafiltered over disposable TFF cartridges (Millipore). The mAb was then polished over a hydroxyapatite column (Bio-Rad, Hercules, Calif.), concentrated to 10 mg/ml in phosphate-buffered saline and stored at -70.degree. C. or colder prior to use.

RANTES was purchased from R&D Systems (Minneapolis, Minn.). The anti-CCRS mAb 2D7 was purchased from BD Biosciences (Cat #555993), and the anti-CCRS mAb CTC5 was purchased from R&D Systems (Cat

#FAB1802P).

RET Assay

The HIV-1 RET assay has been described in detail previously (Litwin et al., 1996). Briefly, fluorescein octadecyl ester (F18; Molecular Probes, Eugene, Oreg.; 5 mg,/ml in ethanol), was diluted 1:800 in DMEM labeling medium (DMEM; Invitrogen, Carlsbad, Calif.) with 10% fetal bovine serum (FBS; HyClone, Logan, Utah) and adjusted to an A.sub.506 of 0.34.+-. 10%. Octadecyl rhodamine B chloride (R18; Molecular Probes; 10 mg/ml in ethanol) was diluted 1:2050 in labeling medium and adjusted to an A.sub.565 of 0.52 10%. Both dyes were further diluted 2-fold by addition to cells in T75-cm.sup.2 flasks. HeLa-Env.sub.JRFL and CEM NKR-CCR5 cells were incubated overnight in F18- and R18-containing culture medium, respectively. The following day, medium from HeLa-Env.subJRFL cells was removed and 10 ml of 0.5 mM EDTA was added and incubated at 37.degree. C. for 5 min. EDTA was removed and the flask was returned to the incubator for another 5 min followed by striking of the flask to dislodge cells. Ten ml of PBS- with 15% FBS were added to the flask and the contents were transferred to a 50-ml conical centrifuge tube. Suspension CEM NKR-CCR5 cells were added directly to a separate 50-ml conical centrifuge tube. Both cell lines were centrifuged at 300.times.g for 5 min. The supernatant was discarded and cells were resuspended in 10 ml of PBS-/15% FBS. The centrifugation/wash step was repeated twice, after which the cells were counted and concentrations adjusted to 1.5.times.10.sup.6 cells/mL Ten μl of each cell type (15,000 cells) were seeded into wells of a 384-well plate. Inhibitor compounds were added immediately thereafter to bring the final well volume to 40 μl, and the plates were incubated for 4 h at 37.degree. C. Compounds were tested individually and in combination at a fixed molar ratio or mass ratio over a range of serial dilutions. The plates were then read on a fluorescence plate reader (Victor.sup.2, Perkin Elmer, Boston, Mass.) using the excitation/emission filter combinations shown in Table 4.

Table 4. Excitation/emission filter combinations for RET assay

| Scan No. | Excitation wavelength | Emission wavelength |
|---|---|---|
| 1 | 450 nm/50 nm | 530 nm/25 mn |
| 2 | 530 mn/25 nm | 590 mn/35 nm |
| 3 | 450 nm/50 mn | 590 nm/35 nm |

The "% RET" was calculated according to the following formula after subtraction of background (blank) readings:

$$\% \text{ RET}=100.\text{times}.[(A_3-(A_1 \text{ x } F_{spill})-(A_2 \text{ x } R_{spill}))/A_2]$$

Where: $F_{spill}$= HeLa cells alone, Scan 3/Scan 1;
$R_{spill}$ = CEM cells alone, Scan 3/Scan 2;
$A_1$ = Scan 1 value for HeLa and CEM cells in combination;
$A_2$= Scan 2 value for HeLa and CEM cells in combination; and
$A_3$ = Scan 3 value for HeLa and CEM cells in combination.

The "% Inhibition" was calculated according to the following formula:

$$\% \text{ Inhibition} = 100.\text{times}.[(\text{Max } \% \text{ RET-}\% \text{ RET for sample well})/(\text{Max } \% \text{ RET-Min } \% \text{ RET})]$$

Where: Max % RET = average of % RET values for HeLa and CEM cell combination without added inhibitor; and Min % RET = average of % RET values for HeLa and CEM cell combination in presence of 500 ng/ml of Leu-3a mAb (an antibody that targets CD4 and fully blocks fusion in the RET assay at this concentration).

Fifty percent inhibition ($IC_{50}$) values were determined by fitting the inhibition data with a nonlinear, four-parameter, variable slope equation (GraphPad Prism, 4.02; GraphPad Software, San Diego, Calif.). Upper and lower inhibition values were constrained to 100% and 0%, respectively for curve fitting.

**Preparation of PBMCs**

Replication of authentic HIV-1 is measured in activated peripheral blood mononuclear cells (PBMCs) using the

monocyte/macrophage-tropic HIV-1 clone, JRFL (HIV-$1_{JRFL}$), for these studies.

PBMCs are isolated from 4 separate donors (Leukopacks) by centrifugation on a Ficoll gradient. CD8 cells are depleted using RosetteSep CD8 Depletion Cocktail (#15663, StemCell Research, Vancouver, BC). Cells are diluted to 4.times.$10^6$/ml and added in equal parts to three T175-$cm^2$ flasks and then stimulated by addition of one of the following media: IL-2 Medium [RPMI 1640 (#10-040-CV, Cellgro, Herndon, Va.), 10% FBS (#35-010-CV), 2 mM L-Glutamine (#25-005-CI), 100 U/ml IL-2 (Sigma, St Louis, Mo.)]; PHA 5 Medium: [IL-2 Medium with 5 ug/ml Phytohemaggtutinin PHA-P (PHA) (#L8754, Sigma, St Louis, Mo.), filtered]; or PHA 0.5 Medium: [IL-2 Medium with 0.5 ug/ml PHA, filtered]. Each flask receives a total of 50-150 ml of medium. Flasks are incubated for 3 days at 37.degree. C. followed by pooling of the contents prior to use in the infection assay.

### Virus Titration

Serial dilutions of virus are tested in quadruplicate on activated PBMCs (1.4.times.$10^5$ PBMC/well). Titration Medium [IL-2 Medium with 100 IU/ml penicillin/streptomycin (#30-002-CI, Cellgro)] is utilized for virus titrations. Fifty μl of diluted virus is added to 100 μl of PBMCs in flat bottom, tissue-culture treated 96-well plates (VWR#29442-054, Corning, Corning, N.Y.) and the plates are incubated at 37.degree. C. in a humidified, 5% $CO_2$ incubator. After 7 days, 50 μl are removed from each well an for virus levels by p24 antigen ELISA (Perkin Elmer, Boston, Mass.). Virus titer is determined by the method of Reed and Muench (Table 11, see below).

### Neutralization Assay

Stimulated PBMCs are seeded into wells of 96-well flat bottom plates at a density of 1.4.times.$10^5$ cells/well. Virus is diluted to 2,000 $TCID_{50}$/ml and mixed with serial 0.5 log.sub.10 dilutions of compound for 1 h at 37.degree. C. prior to addition to the cell plates. The final amount of virus added per well is 100 $TCID_{50}$. The final DMSO concentration in the assay is always 0.5% whenever small molecule inhibitors are being tested. Plates are incubated at 37.degree. C. for 5 days, at which time an aliquot of supernatant is removed for p24 antigen ELISA. If control wells (virus without inhibitor) exhibit low p24 antigen levels then the plates are brought back to full volume with Titration medium and incubated for an additional 24 h.

### Data Analysis

Neutralization activity is displayed by plotting the percent inhibition of p24 antigen production (after background values are subtracted from all datapoints) versus log.sub.10 drug concentration. The percent inhibition is derived as follows [1-(p24 levels in the presence of drug/p24 levels in the absence of drug)].times. 100. IC.sub.50 values are determined by fitting the inhibition data with a non-linear, four-parameter, variable slope equation (GraphPad Prism, ver. 4.02; GraphPad Software, San Diego, Calif). Upper and lower inhibition values are constrained to 100% and 0%, respectively for curve fitting,

### Phase 1a Clinical Study

Individuals were treated in sequential, dose-rising cohorts of 5 subjects (4 active and 1 placebo) each and evaluated for up to 120 days post-treatment. A population of healthy, ie., HN-1 uninfected, male volunteers with no abnormal findings on physical exam, medical history and ECG, aged 19-50, was administered a single intravenous infusion of PRO 140 (0.1, 0.5, 2.0 and 5.0 mg per kg body weight). Safety assessments consisted of monitoring the following: vital signs (blood pressure, pulse, temperature, etc; hematology (hemoglobin, hematocrit, leukocytes, platelets, etc.); serum chemistries (AST/ALT, alkaline phosphatase, BUN, creatinine, etc.); urinalysis (pH, specific gravity, protein, glucose, leukocytes, etc.); and ECGs (12-lead).

### Measurement of Coating of CCR5 Cells by PRO 140

Whole blood specimens were combined separately with the indicated phycoerythrin-labeled anti-CCR5 antibodies or with appropriate isotype-control antibodies. Erythocytes were lysed and leukocytes were stabilized using the ImmunoPrep Reagent System (Beckman Coulter), and the cells were analyzed on a TQ Prep.TM. flow cytometry workstation (Beckman Coulter). Data were expressed as the percent of CCR5 cells relative to all cells gated in the analysis. CTC5 is an anti-CCR5 antibody that does not compete with PRO 140. 2D7 is an anti-CCR5 antibody that does compete with PRO 140.

**Measurement of Serum Concentrations of PRO 140**

Sera were diluted as appropriate and combined with L1.2-CCR5 cells, which are mouse pre-B lymphoma cells engineered to stably express human CCR5. In order to generate a standard curve, PRO 140 standard was tested in parallel at concentrations ranging from 0.062 to 4.0 mg/ml in 10% normal human serum (NHS). 10% NHS containing no PRO 140 was analyzed as a negative control Following incubation with test samples, cells were washed and combined with a FITC-labeled sheep antibody against human IgG4 (The Binding Site Limited, Cat. #AF009). Cells were washed again and analyzed by flow cytometry. The concentration of PRO 140 was determined by comparing the median fluorescence intensity (MFI) of the test sample with MFI values of the standard curve.

**Determination of Plasma RANTES Concentration**

The assay employed the Quantikine.TM. Human RANTES Immunoassay Kit (R&D Systems, Minneapolis, Minn.). Briefly, platelet-poor plasma was collected in CTAD/EDTA tubes and stored at - 20.degree. C. Test samples and RANTES standard were added to microtiter plates that were pre-coated with a mouse monoclonal antibody to RANTES. Following incubation, plates were washed and contacted with an anti-RANTES polyclonal antibody conjugated to horseradish peroxidase (HRP). Plates were washed again prior to addition of tetramethlybenzidine substrate for colorimetric detection. The Lower Limit of Quantification of the assay was 415 pg RANTES/ml plasma.

Results and Discussion

PRO 140 is a humanized IgG4,.kappa. anti-CCR5 mAb being developed for HIV-1 therapy. This antibody has been shown to broadly and potently inhibit CCR5-mediated fusion of HIV-1 to target cells *in vitro*. PRO 140 is also highly active in a therapeutic hu-PBL-SCID mouse model, and preliminary data are now available from a Phase la clinical study in healthy human subjects.

**In Vitro Antiviral Activity of PRO 140**

Murine and humanized PRO 140 were tested against four primary R5 HIV-1 isolates as described in the Methods. FIG. 1 shows that PRO 140 has potent antiviral activity *in vitro*, neutralizing a variety of primary R5 strains with an IC90 of 3-4 .mu.g/ml. PRO 140 exhibited similar antiviral activity to the murine mAb, PA14, from which PRO 140 is derived.

**Preliminary Data from Phase 1a Clinical Study**

The primary objective of the Phase 1a study was to evaluate the safety and tolerability of PRO 140 given as a single dose in a rising dose cohort regimen in healthy male subjects. The secondary objectives were (1) to gain information about the pharmacokinetics of intravenously administered PRO 140, and (2) to gain information on the effects of PRO 140 on blood levels of CCR5+cells and chemokines.

**Pharmacokinetics of PRO 140**

Healthy male volunteers were treated with a single intravenous infusion of PRO 140 at dose levels of 0.1, 0.5, 2.0 and 5.0 mg/kg. PRO 140 and placebo were generally well tolerated with no significant changes in ECGs and no dose-limiting toxicity.

Serum was collected post-treatment, cryopreserved, and analyzed for PRO 140 levels. Peak serum concentrations ranged to 3 mg/ml at 0.1 mg/kg and 12 mg/ml at 0.5 mg/kg. Serum concentrations remained detectable (>400 ng/ml for up to 5 days at 0.1 mg/kg, 21 days at 0.5 mg/kg, and for over 60 days following a single 2 mg/kg injection (FIG. 7). Serum concentrations of PRO 140 increased proportionally with dose level, and the clearance rate was similar to that of other humanized mAbs. Pharmacokinetic (PK) metrics were determined using WinNonLin (PharSight Corporation, Mountain View, Calif.) using a noncompartmental model, and the terminal serum half-life of PRO 140 was determined to be 10-12 days. As expected, no subject developed antibodies to the humanized PRO 140.

**Coating and Non-Depletion of CCR5 Lymphocytes by PRO 140**

Healthy male volunteers (n=4) were treated with a single intravenous infusion of PRO 140 at a dose level of 2 mg/kg. For up to 60 days post-treatment, at the times indicated in FIG. 6, blood was collected and analyzed for CCR5 lymphocyte levels.

Following treatment with PRO 140, there was no decrease in the overall number of CCR5 lymphocytes at measured by CTC5 binding; however, the binding of antibody 2D7 was significantly decreased (FIG. 6). Background binding of isotype control antibodies was unchanged. Since the binding of CTC5 is not decreased by the presence of PRO 140, the CTC5-PE values are a measure of the total number of circulating CCR5 lymphocytes. Since 2D7 competes with PRO 140, the 2D7-PE values reflect the number of CCR5 lymphocytes that are not coated with PRO 140.

The data indicate that a single 2 mg/kg dose of PRO 140 effectively coats CCR5 lymphocytes without cellular depletion for two weeks, and cells remain partially coated for >4 weeks. In addition, CCR5 coating was more prolonged in patients treated with 5 mg/kg PRO 140. The data indicate that a single 5 mg/kg dose of PRO 140 effectively coats CCR5 lymphocytes without cellular depletion and the cells remain partially coated for >60 days (FIG. 13). Since CCR5 coating is the mechanism whereby PRO 140 inhibits HIV, viral loads in HIV-infected individuals could be expected to decrease in a similar temporal manner.

**Effect of PRO 140 on Plasma Chemokine Levels**

Healthy male volunteers were treated with a single intravenous infusion 010.1 mg/kg PRO 140 (Cohort 1), 0.5 mg/kg PRO 140 (Cohort 2) or matched placebo. Plasma was collected post-treatment at the indicated times, cryopreserved and analyzed for levels of RANTES, a CC-chemokine that serves as a natural ligand for CCR5. RANTES levels were measured by ELISA in platelet-depleted plasma pre-dose and up to 28 days post-dose. As shown in FIG. 8, there was no significant change in RANTES levels following PRO 140 treatment (P >0.14 all times). These data are consistent with *in vitro* findings that PRO 140 does not antagonize CCR5 function. The findings suggest that PRO 140 does not have untoward effects on CCR5-mediated immune function in treated patients.

The results described herein indicate that in addition to PRO 140 broadly and potently inhibiting CCR5-mediated HIV-1 entry without CCR5 antagonism or other immunologic side effects in preclinical testing, this has demonstrated favorable tolerability, PK and immunologic profiles in preliminary results from an ongoing Phase 1a study in healthy volunteers. Thus, in many respects, PRO 140 offers a novel and attractive product profile for anti-HIV-1 therapy.

Moreover, the activities of anti-CCR5 mAbs are fundamentally distinct from, but complementary to, those of small-molecule CCR5 antagonists (see Table 2) which are also currently undergoing human clinical trials. PRO 140 has recently been shown to work synergistically with non-antibody CCR5 antagonists in inhibiting CCR5-mahated HIV-1 fusion to target cells. Accordingly, combination therapy comprising administration of anti-CCR5 mAbs and non-antibody CCR5 antagonists may offer powerfully effective, new approaches to preventing and treating HIV-1 infection.

**Part II**

<u>RET EXAMPLE 1: Combination Testing of Pro 140 and HIV-1 Entry Inhibitors in the Fluorescence</u>

<u>Materials and Methods</u>

**Compounds and mAbs**

PRO 140 was prepared by expression in Sp2/0 cells using Hybridoma serum-free medium supplemented with 2 mM L-glutamine (Invitrogen, Carlsbad, Calif.). Bulk mAb was clarified using a 5.0 .mu.m Depth filter (Sartorius, Goettingen, Germany) followed by passage over a 02 .mu.m sterilizing grade filter (Sartorius). The mAb was purified by passage first over an affinity column (MabSelect Protein A column, Amersham, Piscataway, N.J.) and then by ion exchange chromatography (SP Sepharose Cation Exchange resin, Amersham). PRO 140 was nanofiltered using a Viresolve.TM. 10 Opticap NFP capsule (Millipore, Billerica, Mass.) followed by a 0.2 .mu.m filter and concentrated/diafiltered over disposable TFF cartridges (Millipore). The mAb was then polished over a hydroxyapatite column (Bio-Rad, Hercules, Calif.), concentrated to 10 mg/ml in phosphate-buffered saline and stored at -70.degree. C. or colder prior to use.

SCH-D (Schering Plough; Tagat et al., 2004), TAK-779 (Takeda Pharmaceuticals; Shiraishi et al., 2000), UK-427,857 (Pfizer, Wood and Armour, 2005), and BMS378806 (Bristol-Myers Squibb; Lin et al., 2003) were prepared by commercial sources.

SCH-D has the following structure:

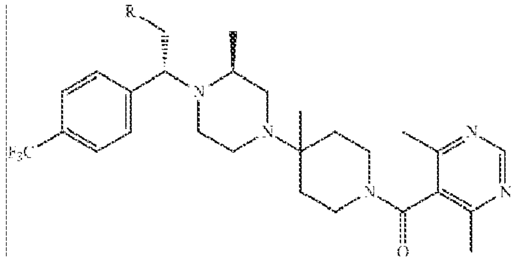

R -OCH3 (R,S)

SCH-D (also designated SCH-417690): 1-[(4(4,6-dimethyl-S-pyrimidinyl)carlxmyl]-4-[4-[2-methoxy-1(R)-4-(trifluoromethyl)phenyl]ethyl-3(S)-methyl-1-piperazmyl]-4-methylpiperidine (Schering-Plough)

SCH-D was synthesized according to the procedure described in Tagat et al. (2004) and set forth in FIG. 1.

TAK-779 has the following structure:

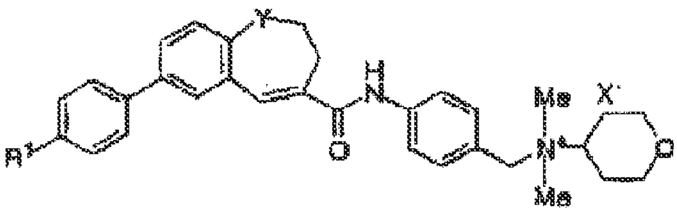

where Y = $-CH_2$, X =-C1, R1 = $-CH_3$

TAK-779 was synthesized according to the procedure described in Shiraishi et al. (2000) and set forth in FIG. 2.

TAK-652 has the following structure:

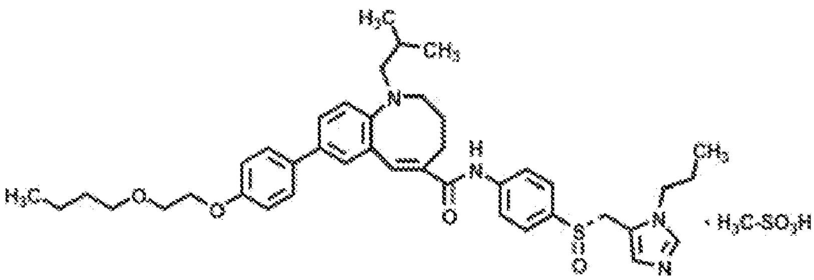

UK-427,857 has the following structure:

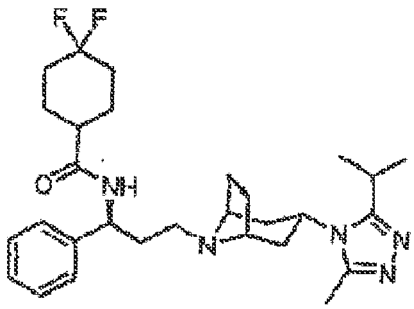

UK-427,857 was synthesized according to the procedure described in PCT International Publication No. WO 01/90106 and set forth in FIG. 3.

BMS378806 has the following structure:

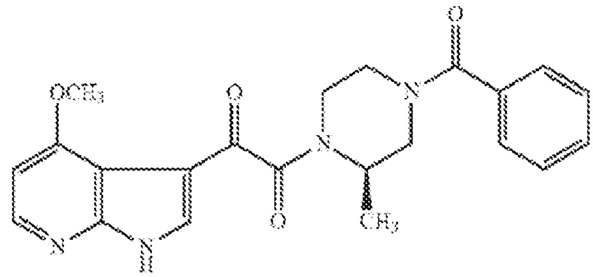

BMS378806: (R)-N-(benzoyl)-3-methyl-N'-[(4-methoxy-7-azaindol-3-yl)-oxoacetyl]-piperazine (Bristol-Myers Squibb). It was synthesized according to the procedure described in U.S. Pat. No. 6,476,034 (compound 17a).

Nevirapine (Boehringer Ingelheim; Merluzzi et al., 1990) and atazanavir (Bristol-Myers Squibb; Robinson et al., 2000) were purchased from commercial sources. PRO 542 was expressed in Chinese hamster ovary cells and purified as described previously (Allaway et al., 1995). T-20 (Fuzeon.RTM.) was synthesized by solid-phase fluroenylmethoxycarbonyl chemistry, was purified by reverse-phase chromatography and was analyzed for purity and size by HPLC and mass spectroscopy as described previously (Nagpshima et al., 2001). AZT was purchased from Sigma Chemicals (St Louis, Mo.). RANTES was purchased from R&D Systems (Minneapolis, Minn.). The anti-CCR5 mAb 2D7 was purchased from Pharmingen (San Diego, Calif.), and the anti-CD4 mAb Leu-3A was purchased from Becton Dickinson (Franklin Lakes, NJ.).

For testing, small molecule compounds were solubilized in dimethylsulfoxide (DMSO) to 10 mM and then diluted in DMSO to 200.times. the final concentration to be utilized in the antiviral assay. Serial dilutions of small molecules were conducted in DMSO. Subsequent dilutions were conducted in medium to achieve a final DMSO concentration in the assay of 0.5%. Peptides and mAbs were diluted in PBS in the absence of DMSO. Typically, inhibitor concentrations in the RET assay included eleven 3-fold dilutions ranging from 200 nM to 3.0 pM.

**Cell Preparation**

HeLa cells were engineered to express HIV -1 gp120/gp41 from the macrophage-tropic primary isolate JRFL as described (HeLa-Env.sub.JRFL: Litwin et al., 1996). Briefly, the HIV-1.sub.LA1 Env gene was excised from the plasmid pMA243 (Dragic et al., 1992) and the HIV-1.subJRFL Env gene was inserted. The HTV-1.subJRFL Env gene was amplified from the plasmid pUCFL112-1 (Koyanagi et al., 1987). The resulting plasmid, designated JR-FL-pMA243, was sequenced by standard methods and transfected into HeLa cells using lipofectin (Gibco BRL/Invitrogen, Carlsbad, Calif.). HeLa-Env.sub.JRFL transfectants were selected in methotrexate (Sigma, St Louis, Mo.) and cloned twice by limiting dilution. The transduced human T cell leukemia line CEMNKR-CCR5 cells were obtained from the NIH AIDS Research and Reference Program (Cat. No. 458).

**RET Assay**

The HIV-1 RET assay has been described in detail previously (Litwin et al., 1996). Briefly, fluorescein octadecyl ester (F18; Molecular Probes, Eugene, Oreg.; 5 mg/ml in ethanol), was diluted 1:800 in DMEM labeling medium (DMEM; Invitrogen, Carlsbad, Calif.) with 10% fetal bovine serum (FBS; HyClone, Logan, Utah) and adjusted to an A.sub.506 of 0.34.+-.10%. Octadecyl rhodamine B chloride (R18; Molecular Probes; 10 mg/ml in ethanol) was diluted 1:2050 in labeling medium and adjusted to an A.sub.565 of 0.52.+-. 10%. Both dyes were further diluted 2-fold by addition to cells in T75-cmsup.2 flasks. HeLa-Env.sub.JRFL and CEM NKR-CCR5 cells were incubated overnight in F18- and R18-containing culture medium, respectively. The following day, medium from HeLa-Env.sub.JRFL, cells was removed and 10 ml of 0.5 mM EDTA was added and incubated at 37.degree. C. for 5 min. EDTA was removed and the flask was returned to the incubator for another 5 min followed by striking of the flask to dislodge cells. Ten ml of PBS- with 15% FBS were added to the flask and the contents were transferred to a 50-ml conical centrifuge tube. Suspension CEM NKR-CCR5 cells were added directly to a separate 50-ml conical centrifuge tube. Both cell lines were centrifuged at 300.times.g for 5 min. The supernatant was discarded and cells were resuspended in 10 ml ofPBS-/15% FBS. The centrifugation/wash step was repeated twice, after which the cells were counted and concentrations adjusted to 1.5.times.10.sup.6 cells/ml. Ten μl of each cell type (15,000 cells) were seeded into wells of a 384-well plate. Inhibitor compounds were added immediately thereafter to bring the final well volume to 40 p1, and the plates were incubated for 4 h at 37.degree. C. Compounds were tested individually and in combination at a fixed molar ratio or mass ratio over a range of serial dilutions. The plates were then read on a fluorescence plate reader (Victor.sup.2, Perkin Elmer, Boston, Mass.) using the excitation/emission filter combinations shown in Table 5.

Table 5. Excitation/emission filter combinations for RET assay

| Scan No. | Excitation wavelength | Emission wavelength |
|---|---|---|
| 1 | 450 nm/50 nm | 530 nm/25 nm |
| 2 | 530 nm/25 nm | 590 nm/35 nm |
| 3 | 450 nm/50 nm | 590 nm/35 nm |

The "% RET" was calculated according to the following formula after subtraction of background (blank) readings:

$$\% RET=100.times.[(A_3-(A_1 x F_{spill})-(A_2 x R_{spill}))/A_2]$$

Where: $F_{spill}$= HeLa cells alone, Scan 3/Scan 1;
$R_{spill}$ = CEM cells alone, Scan 3/Scan 2;
$A_1$= Scan 1 value for HeLa and CEM cells in combination;
$A_2$= Scan 2 value for HeLa and CEM cells in combination; and
$A_3$= Scan 3 value for HeLa and CEM cells in combination.

The "% Inhibition" was calculated according to the following formula:

$$\% Inhibition = 100 x [(Max \% RET-\% RET for sample well)/(Max \% RET-Min \% RET)]$$

Where: Max % RET = average of% RET values for HeLa and CEM cell combination without added inhibitor; and Min % RET = average of % RET values for HeLa and CEM cell combination in presence of 500 ng/ml of Leu-3a mAb (an antibody that targets CD4 and fully blocks fusion in the RET assay at this concentration).

Fifty percent inhibition ($IC_{50}$) values were determined by fitting the inhibition data with a non-linear, four-parameter, variable slope equation (GraphPad Prism, 4.02; GraphPad Software, San Diego, Calif.). Upper and lower inhibition values were constrained to 100% and 0%, respectively for curve fitting.

**Synergy Determinations**

Cooperative inhibition effects of drug combinations were determined by the method of Chou and Talalay (1984). IC.sub.50 values were generated for all combinations as described above. Combination Index (CI) and Dose Reduction (DR) values were calculated according to the following formulas:

$$C I = ( IC 50 Dcomb 1 IC 50 Dsolo 1 ) + ( IC 50 Dcomb 2 IC 50 Dsolo 2 ) + .alpha. ( ( IC 50 Dcomb 1 ) ( IC 50 Dcomb 2 ) ( IC 50 Dsolo 1 ) ( IC 50 Dsolo 2 ) ) \#\#EQU00001\#\#$$

$$DR (for compound 1)=(IC.sub.50Dsolo1/IC.sub.50Dcomb1)$$

$$DR (for compound 2)=(IC.sub.50Dsolo2/IC.sub.50Dcomb2)$$

Where: "IC.sub.50Dcomb1"=IC.sub.50 of drug 1 in combination with drug 2; [0220] "IC.sub.50Dsolol"=IC.sub.50 of drug 1 when tested alone; [0221] "IC.sub.50Dcomb2"=IC.sub.50 of drug 2 in combination with drug 1; [0222] "IC.sub.50Dsolo2"=IC.sub.50 of drug 2 when tested alone; [0223] .alpha.=0 if the effects of the two drugs are mutually exclusive; and [0224] .alpha.=1 if the effects of the two drugs are mutually nonexclusive
Combinations with CI<1 are determined to be synergistic, whereas combinations with CI>1 are determined to be antagonistic. Additivity is reflected in combinations for which CI=1.

Ninety five percent Confidence Intervals were calculated in Microsoft Excel using the formula:

=Confidence(alpha,stdev,n)

Where: alpha=0.05 (95% confidence); [0228] stdev=standard deviation of dataset mean; and [0229] n=number of replicates.

Results

**Preparation of Small-Molecule Fusion Inhibitors**

SCH-D, TAK-779, UK-427,857, and BMS378806 were prepared by commercial sources. The desired quantities and HPLC purity of the compounds were realized. Purity of the compounds was supported by results obtained from elemental analysis, and the identities of the products were confirmed by proton NMR (proton and carbon- 13) and/or mass spectrum data.

Synergistic Interactions Revealed by RET Assay

Synergy experiments were conducted using the cell-cell RET fusion assay to assess initially the potential for cooperative interactions between PRO 140 and small-molecule and peptide-based inhibitors of CCR5, CD4, HIV-1 gp 120 and HIV-1 gp41. The experiments were then extended to the CCR5-specific murine monoclonal antibody, 2D7 (Wu et al., 1997).

Experiments measuring inhibition of HIV-1 Env-mediated fusion were first conducted using combinations of PRO 140 with, respectively, PRO 140 itself 3 small-molecule CCR5 antagonists (SCH-D, TAK-779, UK427857), the natural peptide ligand of CCR5 (RANTES), and an anti-CCR5 mAb (2D7), a peptide-based inhibitor of gp41 (T-20), a protein-based inhibitor of gp120 (PRO 542), a small-molecule inhibitor of gp120 (BMS378806), and an anti-CD4 mAb (Leu3A). Mass ratios of PRO 140 to other entry inhibitors ranged from 0.75 to 364. The results are shown in Table 6.

Table 6. Combination Index and Dose Reduction Values for inhibition of HIV-1 Env-mediated fusion with combinations of PRO 140 and entry inhibitors

| PRO 140 in combination with:[a] | No. of tests | Cpd mass ratios[b] | Inhibitor target | Mean CF[c] | Mean Dose Reduction (PRO 140) | Mean Dose Reduction (Cpd in combination) |
|---|---|---|---|---|---|---|
| | | | | Cell-cell fusion assay | | |
| PRO 140 | 9 | 1 | CCR5 | 0.97 ±0.08 | 2.07±0.18 | 2.07 ± 0.18 |
| TAK-779 | 8 | 282 | CCR5 | 0.36 ± 0.10 | 4.10 ±2.03 | 15.86 ±7.10 |
| SCH-D | 9 | 279 | CCR5 | 0.51 ±0.05 | 421 ±0.96 | 3.90 ± 0.71 |
| UK-427,857 | 3 | 292 | CCR5 | 0.59 ± 0.04 | 4.16-±0.41 | 2.98 ± 0.65 |
| RANTES | 4 | 19 | CCR5 | 0.59 ± 0.08 | 4.13 ± 0.99 | 3.24±1.06 |
| 2D7 | 2 | 1 | CCR5 | 0.93 ± 0.04 | 1.87 ± 0.07 | 2.54 ± 0.13 |
| T-20 | 7 | 33 | gp41 | 0.84 ±0.16 | 1.77 ±0.40 | 7.47±3.34 |
| PRO 542 | 6 | 0.75 | gp120 | 0.96 ± 0.17 | 1.59 ± 021 | 5.54 ± 1.49 |

(continued)

| PRO 140 in combination with:[a] | No. of tests | Cpd mass ratios[b] | Inhibitor target | Mean CF[c] | Mean Dose Reduction (PRO 140) | Mean Dose Reduction (Cpd in combination) |
|---|---|---|---|---|---|---|
| BMS-378806 | 7 | 364 | gp120 | 121± 0.21 | 1.64 ±0.30 | 2.85 ± 0.76 |

[a] Compounds were tested at a 1:1 molar ratio.
[b] Mass of PRO 140/mass of other HIV-1 entry inhibitor tested in combination. Molecular weights of inhibitors are: PRO 140 .apprxeq. 150,000 g/mole; SCH-D = 538 g/mole; TAK-779 = 531 g/mole (hydrochloride salt); UK-427,857 = 514 g/mole; RANTES .apprxeq. 7,800 g/mole; 2D7 .apprxeq. 150,000 g/mole; T-20 = 4,492 g/mole; PRO 542 .apprxeq. 200,000 g/mole; BMS-378806 = 412 g/mole.
[c] Combination Index at $IC_{50}$ value. The mutually exclusive CI formula (.alpha. = 0) was utilized for PRO 140 in combination with molecules that bind CCR5, and the mutually non-exclusive formula (.alpha. = 1) was utilized for PRO 140 in combination with molecules that bind other targets (Chou and Rideout, 1991).

Two small-molecule CCR5 antagonists, SCH-D and TAK-779, were assayed in combination. PRO 542, a recombinant antibody-like fusion protein in which the heavy- and light-chain variable domains of human IgG2 have been replaced with the D1D2 domains of human CD4, was also tested in combination with the anti-CD4 mAb, Leu-3A. The results of these assays are shown in Table 7.

Table 7. Other drug combinations tested in the RET assay for cooperativity

| Drug1 | Drug2 | Molar rations (Drug 1 to 2) | N | Mean CI± stdev[a] | Mean DR (Drug 1) | MeanDR (Drug 2) |
|---|---|---|---|---|---|---|
| SCH-D | TAK-779 | 1:1 | 4[b] | 1.12 ±0.32 | 1.48 ± 0.96 | 4.31± 1.82 |
| PRO 542 | Leu-3A | 22.9:1 | 2 | 169± 0.3 | 0.7±0 | 0.16±0 |

[a] CI values were calculated using the mutually exclusive formula for SCH-D vs. TAK-779 (i.e., where $\alpha$ = 0) and the mutually non-exclusive formula for PRO 542 vs. Leu-3A (i.e., where $\alpha$ = 1; see methods).
[b] One aberrant datapoint was culled from the calculation of Mean CI and Mean DRs.

The effect of varying the relative amounts of compounds in the combinations on the level of cooperativity was also measured. Molar ratios of 5:1 and 1:5 PRO 140 were used. The results are tabulated in Table 9, and the mean CI values with 95% confidence intervals are plotted in FIG. 4 for the 1:1 molar ratio data. In addition to PRO 140, the inhibitory activity of mAb 2D7, a CCR5-specific murine antibody (Wu et al., 1997) was also tested in combination with the small-molecule CCR5 antagonists and with RANTES using the fluorescent RET assay. The results are shown in Table 8.

Table 8. Combination Index and Dose Reduction Values for inhibition of HIV-1 Env-mediated fusion with combinations of PRO 140 and entry inhibitors

| PRO 140 in combination with:[a] | Ratio[a] | Cpd mass ratios[b] | Mean Combination Index[c] | Mean Dose Reduction (PRO 140) | Mean Dose Reduction (Cpd in combination) |
|---|---|---|---|---|---|
| | | | Cell-cell fusion assay | | |
| PRO 140 | 5:1 | 5 | 1.15 ±0.09 | 1.05±0.08 | 526 ± 0.41 |
| PRO 140 | 1:5 | 0.2 | 1.09-±0.08 | 5.54±0.38 | 1.10-±0.08 |
| TAK-779 | 5:1 | 1410 | 0.57-±0.07 | 1.89±0.14 | 33.59±18.85 |
| TAK-779 | 1:5 | 56.4 | 052 ±0.20 | 5.58±0.52 | 3.78 ±1.95 |
| SCH-D | 5:1 | 1395 | 0.66±0.10 | 1.92 ± 0.40 | 8.44± 127 |
| SCH-D | 1:5 | 55.8 | 0.69-±0.05 | 9.95±2.03 | 1.73±0.19 |
| UK-427,857 | 5:1 | 1460 | 0.66± 0.11 | 2.00±0.35 | 7.25± 2.19 |

(continued)

| PRO 140 in combination with:[a] | Ratio[a] | Cpd mass ratios[b] | Mean Combination Index[c] | Mean Dose Reduction (PRO 140) | Mean Dose Reduction (Cpd in combination) |
|---|---|---|---|---|---|
| | | | Cell-cell fusion assay | | |
| UK-427,857 | 1:5 | 58.4 | 0.73 ± 0.05 | 1131 ±2.14 | 1.58-±0.17 |
| RANTES | 5:1 | 95 | 0.84±0.14 | 1.63 ± 0.43 | 5.39±1.13 |
| RANTES | 1:5 | 3.8 | 0.66-±0.06 | 13.64-±4.75 | 1.75 ±0.28 |
| T-20 | 5:1 | 165 | 1.10±0.12 | 0.98±0.11 | 31.85±10.19 |
| T-20 | 1:5 | 6.6 | 0.76 -± 027 | 2.93 ±0.68 | 3.85±1.50 |
| PRO 542 | 5:1 | 3.75 | 1.13±0.10 | 1.01 ±0.07 | 15.73±4.15 |
| PRO 542 | 1:5 | 0.15 | 1.18±0.17 | 2.83±0.50 | 1.71±029 |
| BMS-378806 | 5:1 | 1820 | 1.12 -±0.10 | 1.14-±0.06 | 8.88 ±4.16 |
| BMS-378806 | 1:5 | 72.8 | 1.55±024 | 3.64±0.73 | 1 |

[a] Molar ratio of PRO 140 to other entry inhibitor tested in combination (n=3 for all experimental results)
[b] Mass of PRO 140/mass of other HIV-1 entry inhibitor tested in combination. Molecular weights of inhibitors are: PRO 140 .apprxeq. 150,000 g/mole; SCH-D = 538 g/mole; TAK-779 = 531 g/mole (hydrochloride salt); UK-427,857 = 514 g/mole; RANTES .apprxeq. 7,800 g/mole; T-20 = 4,492 g/mole; PRO 542 .apprxeq. 200,000 g/mole; BMS-378806 = 412 g/mole.
[c] Combination Index at $IC_{50}$ value. The mutually exclusive CI fomula ($\alpha$ = 0) was utilized for PRO 140 in combination with molecules that bind CCR5, and the mutually non-exclusive formula ($\alpha$ = 1) was utilized for PRO 140 in combination with molecules that bind other targets (Chou and Rideout, 1991).

Table 9. Combination Index and Dose Reduction Values for inhibition of HIV-1 Env-mediated fusion with combinations of 2D7 and entry inhibitors

| 2D7 in combination with:[a] | Cpd Mass Rotios[c] | Inhibitor target | Mean Combination Index[b] | Mean Dose Reduction (2D7) | Mean Dose Reduction (Cpd in combination) |
|---|---|---|---|---|---|
| | | | Cell-cell fusion assay | | |
| TAK-779 | 282 | CCR5 | 0.15 ± 0.03 | 17.20 ± 3.23 | 11.95 ± 4.94 |
| SCH-D | 279 | CCR5 | 0.57 ± 0.10 | 3.25 ± 0.56 | 4.04 ± 0.78 |
| UK427857 | 292 | CCR5 | 0.58 ± 0.03 | 2.45 ± 0.12 | 5.73 ± 0.54 |
| RANTES | 19 | CCR5 | 0.62 ± 0.04 | 1.94 ± 0.08 | 10.18 ± 1.86 |
| PRO 140 | 1 | CCR5 | 0.93 ± 0.04 | 2.54 ± 0.13 | 1.87 ± 0.07 |

[a] Compounds were tested at a 1:1 molar ratio (all data are n = 3 except for 2D7 and PRO 140, where n=2)
[b] Combination Index at $IC_{50}$ value. The mutually exclusive CI fomula ($\alpha$ = 0) was utilized for 2D7 in combination with molecules that bind CCR5 (Chou and Rideout, 1991).
[c] Mass of 2D7/mass of other HIV-1 entry inhibitor tested in combination. Molecular weights of inhibitors are: 2D7 .apprxeq. 150,000 g/mole; SCH-D = 538 g/mole; TAK-779 = 531 g/mole (hydrochloride salt); UK-427,857 = 514 g/mole; RANTES .apprxeq. 7,800 g/mole.

EXAMPLE 2: Combination Testing of Pro 140 with Small Molecule, Peptide and Protein Inhibitors, and HIV-1 in the HIV-1 Pseudovirus Particle (HIV-1PP) Assay

Materials and Methods

**Preparation of HIV-1 Pseudopartides**

HIV-1 pseudopartides (HIV-1pp) are generated in 293T cells by transient coexpression of an HIV-1-based NL4/3luc+env-plasmid and a construct encoding HIV-1.sub.JRFL Env. The NLA/3luc+env-plasmid was obtained from the NIH AIDS Research and Reference Reagent Program (Cat No. 3418), and the HIV-1.sub.JRFL Env was inserted into the pcDNA3.1 vector (Invitrogen). Briefly, 293T cells are calcium phosphate transfected with a 1:1 ratio of NL4/3luc+env-reporter vector and Env expression vector in Hepes buffer (Protection Mammalian Transfection Kit, Promega). After 16 h the transfection medium is aspirated and fresh cell culture medium (DMEM with 10% FBS, glutamine and antibiotics) is added and the incubation is continued at 37.degree. C. for an additional 24-32 h. Cell culture supernatants are collected 48 h post-transfection and centrifuged at 1,400 rpm for 10 min to pellet cell debris. The viral supernatant is brought to a final concentration of 5% sucrose and stored aliquoted at -80.degree. C.

**Cells**

U87-CD4-CCR5 cells were obtained from the NIH AIDS Research and Reference Program (Cat. No. 4035). These cells are maintained in culture medium (DMEM with 10% FBS, antibiotics and glutamine) containing 0.3 mg/ml G418 and 0.5 mg/ml puromycin. Cells are grown in T175-cm.sup.2 flasks at 37.degree. C. and diluted 1:5 every 3-4 days. For assay plate preparation, cells are typsinized and seeded into wells of 96-well tissue-culture treated flat bottom opaque polystyrene plates (Perkin Elmer, Boston, Mass.) at a density of 3.times.10.sup.3 cells/well. Plates are incubated for no more than 4 h at 37.degree. C. in a humidified 5% CO.sub.2 incubator prior to their use in the HIV-1 pp susceptibility assay.

**Compound Preparation**

Fifty μl of diluted compound at 4.times. the desired final concentration are added per well For compounds solubilized in DMSO, the 4.times. stock will contain 2% DMSO (such that the final DMSO concentration in the assay is always 0.5% for small molecules). Control wells receiving no compound are included on each plate. In addition, an AZT inhibition control is included in each assay. Compounds are tested individually and at a fixed mass or molar ratio over a broad range of concentrations.

**Virus Addition**

A vial of frozen, aliquoted HIV-1pp is thawed in a 37.degree. C. waterbath and then placed on wet ice. Virus is diluted in cold cell culture medium as necessary to achieve the desired final virus concentration in the HIV-1pp assay (about 10,000 relative light units (rlu) per well), 50 μl of diluted virus are added per well, bringing the final well volume to 200 μL A no-virus control (minimum or background luminescence) and a no-compound control (maximum luminescence) are included on each plate. The plates are incubated for 72 h at 37.degree. C. in a humidified 5% CO.sub.2 incubator followed by processing for luciferase signal (see below).

**Plate Processing for Luciferase Assay**

Assay medium is aspirated and 200 μl of PBS are added to each well. The PBS is aspirated and 50 μl of 1.times. Cell Lysis Reagent (Promega-Cat. No. E1531) are added to each well Assay plates are then frozen for at least 2 h at -80.degree. C. followed by thawing at room temperature and vigorous mixing with an electronic pipettor. 25 μl from each well are transferred to an opaque 96-well plate (Costar #3922). Four replicates are pooled into the same well on the opaque plate. 100 μl of freshly thawed and reconstituted luciferase substrate (Luciferase Assay System, Promega-Cat. No. E1501) are added to each well of the plate with the electronic pipettor, and luminescence is detected immediately on a Dynex MLX plate reader set to medium gain.

**Data Analysis**

Neutralization activity is displayed by plotting the percent inhibition of luciferase activity (after background rlu values are subtracted from all datapoints) versus log.sub.10 drug concentration. The percent inhibition is derived as

follows: [1-(luciferase activity in the presence of drug/luciferase activity in the absence of drug)].times.100. IC.sub.50 values are determined by fitting the inhibition data with a non-linear, four-parameter, variable slope equation (GraphPad Prism, ver. 4.02; GraphPad Software, San Diego, Calif.). Upper and lower inhibition values are constrained to 100% and 0%, respectively for curve fitting.

**Synergy Determination**

Cooperative interactions between PRO 140 and small-molecule and peptide-based inhibitors of CCR5, CD4, HIV-1 gp120, HIV-1 gp41 and HIV-1 reverse transcriptase (see Tables 4 and for listing of HIV-1 inhibitors approved for clinical use) are determined as described in Example 1. Cooperative inhibition effects of drug combinations are determined by the method of Chou and Talalay (1984). IC.sub.50 values are generated for all combinations as described above. Combination Index (CI) and Dose Reduction (DR) values are calculated according to the following formulas:

C I = ( IC 50 Dcomb 1 IC 50 Dsolo 1 ) + ( IC 50 Dcomb 2 IC 50 Dsolo 2 ) + .alpha. ( ( IC 50 Dcomb 1 ) ( IC 50 Dcomb 2 ) ( IC 50 Dsolo 1 ) ( IC 50 Dsolo 2 ) ) ##EQU00002##

DR (for compound 1)=(IC.sub.50Dsolo1/IC.sub.50Dcomb1)

DR (for compound 2)=(IC.sub.50Dsolo2/IC.sub.50Dcomb2)

Where: "IC.sub.50Dcomb1 "=IC.sub.50 of drug 1 in combination with drug 2;
"IC.sub.50Dsolo1"=IC.sub.50 of drug 1 when tested alone;
"IC.sub.50Dcomb2"=IC.sub.50 of drug 2 in combination with drug 1;
"IC.sub.50Dsolo2"=IC.sub.50 of drug 2 when tested alone;
.alpha.=0 if the effects of the two drugs are mutually exclusive; and
.alpha.=1 if the effects of the two drugs are mutually nonexclusive.

Combinations with CI<1 are determined to be synergistic, whereas combinations with CI>1 are determined to be antagonistic. Additivity is reflected in combinations for which CI=1.

EXAMPLE 3: Combination Testing of Pro 140 with Small Molecule, Peptide and Protein Inhibitors in the HIV-1 Authentic Virus Replication Assay

Materials and Methods

**Preparation of PBMCs**

Replication of authentic HIV-1 is measured in activated peripheral blood mononuclear cells (PBMCs) using the monocyte/macrophage-tropic HIV-1 clone, JRFL (HIV-1.sub.JRFL), for these studies.

PBMCs are isolated from 4 separate donors (Leukopacks) by centrifugation on a Ficoll gradient. CD8 cells are depleted using RosetteSep CD8 Depletion Cocktail (#15663, StemCell Research, Vancouver, BC). Cells are diluted to 4.times.10.sup.6/ml and added in equal parts to three T175-cm.sup.2 flasks and then stimulated by addition of one of the following media: IL-2 Medium [RPMI 1640 (#10-040-CV, Cellgro, Herndon, Va.), 10% FBS (#35-010-CV), 2 mM L-Glutamine (#25-005-CI), 100 U/ml IL-2 (Sigma, St Louis, Mo.)]; PHA 5 Medium: [IL-2 Medium with 5 ug/ml phytohemagglutinin PHA-P (PHA) (#L8754, Sigma, St Louis, Mo.), filtered]; or PHA 0.5 Medium: [IL-2 Medium with 0.5 ug/ml PHA, filtered]. Each flask receives a total of 50-150 ml of medium. Flasks are incubated for 3 days at 37.degree. C. followed by pooling of the contents prior to use in the infection assay.

**Virus Titration**

Serial dilutions of virus are tested in quadruplicate on activated PBMCs (1.4.times.10.sup.5 PBMC/well). Titration Medium [IL-2 Medium with 100 IU/ml penicillin/streptomycin (#30-002-CI, Cellgro)] is utilized for virus titrations. Fifty μl of diluted virus is added to 100 μl of PBMCs in flat bottom, tissue-culture treated 96-well plates (VWR#29442-054, Coming, Coming, NY) and the plates are incubated at 37.degree. C. in a humidified, 5% CO.sub.2 incubator. After 7

days, 50 μl are removed from each well and tested for virus levels by p24 antigen ELISA (Perkin Elmer, Boston, Mass.). Virus titer is determined by the method of Reed and Muench (Table 10).

**Neutralization Assay**

Stimulated PBMCs are seeded into wells of 96-well flat bottom plates at a density of 1.4.times.10.sup.5 cells/well. Virus is diluted to 2,000 TCID.sub.50/ml and mixed with serial 0.5 log.sub.10 dilutions of compound for 1 h at 37.degree. C. prior to addition to the cell plates. The final amount of virus added per well is 100 TCID.sub.50. The final DMSO concentration in the assay is always 0.5% whenever small molecule inhibitors are being tested. Plates are incubated at 37.degree. C. for 5 days, at which time an aliquot of supernatant is removed for p24 antigen ELISA. If control wells (virus without inhibitor) exhibit low p24 antigen levels then the plates are brought back to full volume with Titration medium and incubated for an additional 24 h.

Table 10. Reed and Muench formula for calculating virus titer[a]

| No. of pos. wells | $TCID_{50}$/ml ($10^x$) | No. of pos. wells | $TCID_{50}$/ml ($10^x$) | No. of pos. wells | $TCID_{50}$/ml ($10^x$) | No. of pos. wells | $TCID_{50}$/ml ($10^x$) |
|---|---|---|---|---|---|---|---|
| 1 | 0.74 | 21 | 2.49 | 41 | 4.23 | 61 | 5.98 |
| 2 | 0.83 | 22 | 2.57 | 42 | 4.32 | 62 | 6.07 |
| 3 | 0.92 | 23 | 2.66 | 43 | 4.41 | 63 | 6.15 |
| 4 | 1.00 | 24 | 2.75 | 44 | 4.49 | 64 | 6.24 |
| 5 | 1.09 | 25 | 2.83 | 45 | 4.58 | 65 | 6.33 |
| 6 | 1.17 | 26 | 2.92 | 46 | 4.67 | 66 | 6.42 |
| 7 | 1.26 | 27 | 3.01 | 47 | 4.76 | 67 | 6.50 |
| 8 | 135 | 28 | 3.10 | 48 | 4.84 | 68 | 6.59 |
| 9 | 1.44 | 29 | 3.18 | 49 | 4.93 | 69 | 6.68 |
| 10 | 1.52 | 30 | 3.27 | 50 | 5.02 | 70 | 6.77 |
| 11 | 1.61 | 31 | 336 | 51 | 5.11 | 71 | 6.85 |
| 12 | 1.70 | 32 | 3.45 | 52 | 5.19 | 72 | 6.94 |
| 13 | 1.79 | 33 | 3.53 | 53 | 5.28 | 73 | 7.03 |
| 14 | 1.87 | 34 | 3.62 | 54 | 5.37 | 74 | 7.12 |
| 15 | 1.96 | 35 | 3.71 | 55 | 5.46 | 75 | 7.20 |
| 16 | 2.05 | 36 | 3.80 | 56 | 5.54 | 76 | 729 |
| 17 | 2.14 | 37 | 3.88 | 57 | 5.63 | 77 | 7.38 |
| 18 | 222 | 38 | 3.97 | 58 | 5.72 | 78 | 7.47 |
| 19 | 231 | 39 | 4.06 | 59 | 5.81 | 79 | 7.55 |
| 20 | 2.40 | 40 | 4.15 | 60 | 5.89 | 80 | 7.64 |

[a] To calculate virus titer, first multiply the total number of positive wells by 2 (the chart was designed to be used with replicates of 8), then look up the corresponding TCID.sub.50/mL titer and add 0.7 (the formula requires the addition of a log dilution factor).

**Data Analysis**

Neutralization activity is displayed by plotting the percent inhibition of p24 antigen production (after background values are subtracted from all datapoints) versus log.sub. 10 drug concentration. The percent inhibition is derived as follows [1-(p24 levels in the presence of drug/p24 levels in the absence of drug)].times.100. IC.sub.50 values are determined by fitting the inhibition data with a non-linear, four-parameter, variable slope equation (GraphPad Prism, ver. 4.02; GraphPad Software, San Diego, Calif). Upper and lower inhibition values are constrained to 100% and 0%, respectively for curve fitting.

**Synergy Determinations**

Cooperative interactions between PRO 140 and small-molecule and peptide-based inhibitors of CCR.5, CD4, HIV-1 gp120, HIV-1 gp41, HIV-1 reverse transcriptase and HIV-1 protease (Table 8) are determined as described for Example 1. Cooperative inhibition effects of drug combinations are determined by the method of Chou and Talalay

(1984). IC.sub.50 values are generated for all combinations as described above. Combination Index (CI) and Dose Reduction (DR) values are calculated according to the following formulas:

C I = ( IC 50 Dcomb 1 IC 50 Dsolo 1 ) + ( IC 50 Dcomb 2 IC 50 Dsolo 2 ) + .alpha. ( ( IC 50 Dcomb 1 ) ( IC 50 Dcomb 2 ) ( IC 50 Dsolo 1 ) ( IC 50 Dsolo 2 ) ) ##EQU00003##

DR (for compound 1)=(IC.sub.50Dsolo1/IC.sub.50Dcomb1)

DR (for compound 2)=(IC.sub.50Dsolo2/IC.sub.50Dcomb2)

Where: "IC.sub.50Dcomb1"=IC.sub.50 of drug 1 in combination with drug 2; [0273] "IC.sub.50Dsolo1"=.ICsub.50 of drug 1 when tested alone; [0274] "IC.sub.50Dcomb2"=IC.sub.50 of drug 2 in combination with drug 1; [0275] "IC.sub.50Dsolo2"=ICsub.50 of drug 2 when tested alone; [0276] .alpha.=0 if the effects of the two drugs are mutually exclusive; and [0277] .alpha.=1 if the effects of the two drugs are mutually nonexclusive.

Combinations with CI<1 are determined to be synergistic, whereas combinations with CI>1 are determined to be antagonistic. Additivity is reflected in combinations for which CI=1.

Discussion

PRO 140 is a CCR5-specific mAb being developed for HIV-1 therapy. It is a humanized IgG4,.kappa. version (see PCT International Publication No. WO 03/072766, published Sep. 4, 2003) of the murine antibody, PA14 (Olson et al., 1999; PCT International Publication No. WO 00/35409, published Jun. 20, 2000), which binds to the CCR5 receptor on the surface of a cell and inhibits CCR5-mediated fusion of HIV-1 to the cell. The studies described herein concern the testing of the antiviral activity of PRO 140 in combination with small-molecule and peptide inhibitors of HIV-1 infection. Data generated from this testing were analyzed for potential cooperative effects on inhibition of HIV-1 infection.

In one series of experiments, inhibition of HIV-1 infection was assayed using a fluorescence resonance energy transfer (RET) assay, which measures the fusion of effector cells (HeLa-Env.subJRFL) expressing recombinant HIV-1 strain JRFL envelope glycoproteins (Env) to target cells (CEM NKR-CCR5) expressing CD4 and CCR5 (Litwin et al., 1996). In this assay, effector cells are labeled with the F18 dye and target cells with the R18 dye. HIV-1 Env-mediated fusion of effector and target cells results in the placement of these two dyes within close proximity in the cell membrane. When F18 is excited at its optimum wavelength (450 nm), it emits light at a wavelength (530 nm) that will excite R18 when the two dyes are co-localized in the same membrane, resulting in R18-specific emission at 590 nm. Drug susceptibility is measured by adding serial concentrations of drugs to target cells prior to addition of effector cells. Inhibition of HIV-1 Env-mediated fusion is reflected in a reduction in fluorescence emission due to R18 in a dose-dependent manner, providing a quantitative measure of drug activity.

Initial experiments measuring inhibition of HIV-1 Env-mediated fusion were conducted in order to demonstrate the robustness of the assay system for quantifying cooperative interactions. In these experiments, PRO 140 was run in combination with itself, a combination that should result in combination index (CI) values indicative of additive interactions. Using the methodology of Chou and Talalay (1984), CI values of <1.0, =1.0 and >1.0 are taken to indicate synergistic, additive and antagonistic interactions, respectively. Indeed, PRO 140 run in combination with itself returned a CI value of 0.97.+-.0.08 (n=9; Table 7), indicating that the assay system accurately represented this interaction.

Synergy experiments were then conducted between PRO 140 and 3 small-molecule (SCH-D, TAK-779, UK427857), one peptide (RANTES) and one mAb (2D7) antagonist of CCR5. In addition, cooperative interactions were measured between PRO 140 and T-20 (peptide-based inhibitor of gp41), PRO 542 (protein-based inhibitor of gp120), BMS378806 (small molecule inhibitor of gp120) and Leu-3A (anti-CD4 mAb).

The results (see Table 7) revealed potent synergy between PRO 140 and all 3 small-molecule CCR5 antagonists as well as RANTES. CI values between PRO 140 and these CCR5 antagonists ranged from 0.36.+-.0.10 to 0.59.+-.0.08. Dose reduction values indicated that the compound in combination exerted about a 4-fold effect on PRO 140 activity, whereas the effect of PRO 140 on the compound in combination ranged from about 3- to about 16-fold (Table 7). Modest synergy to additivity was observed between PRO 140 and T-20, PRO 542, BMS-378806 and 2D7 (CI=0.84.+-.0.16, 0.96.+-.0.17, 121.+-.0.21, and 0.93.+-.0.04, respectively).

Small molecule antagonists of CCR5 run in combination (SCH-D and TAK-779) returned a mean CI value of 1.12.+-.0.32, indicating a slightly additive interaction (Table 8). Conversely, the combination of the recombinant antibody-like fusion protein PRO 542 with the anti-CD4 mAb, Leu-3A, resulted in a mean CI value of 16.9.+-.0.3, indicating potent

antagonism between these two HIV-1 inhibitors (Table 8).

Varying the molar ratios of compounds demonstrated similar patterns of cooperativity. At both 5:1 and 1:5 molar ratios of PRO 140 to SCH-D, TAK-779, UK-427,857 and RANTES, potent synergistic inhibition of HIV-1-Env-mediated entry was observed (Table 9). This represents a broad range of inhibitor mass ratios, from a low of 0.15 to a high of 1,820. CI values between PRO 140 and CCR5 antagonists ranged from 0.52.+-.0.20 to 0.84.+-.0.14. More modest synergy to additivity was observed for combinations of PRO 140 with T-20, PRO 542 or BMS-378806. The results of these investigations identify clearly the potent synergistic activities of PRO 140 with CCR5 antagonists, as well as more modest synergy between PRO 140 and T-20 (see FIG. 4).

The HIV-1 inhibitory activity of the CCR5-specific murine mAb, 2D7, in combination with the small-molecule CCR5 antagonists and with RANTES, was also tested using the fluorescent RET assay. 2D7 was found to act synergistically with these CCR5 antagonists and with RANTES (Table 10). CI values between 2D7 and these CCR5 antagonists ranged from 0.15.+-.0.03 to 0.62.+-.0.04. Dose reduction values indicated that the compound in combination exerted about a 2- to 3-fold effect on 2D7 activity, except for TAK-779 which had an approximately 17-fold effect on 2D7 activity. The effect of 2D7 on the compound in combination ranged from about 2- to about 12-fold (Table 10). As observed previously, PRO 140 and 2D7 in combination were essentially additive or modestly synergistic (CI=0.93.+-.0.04).

These results indicate that synergistic inhibition of HIV-1 Env-mediated cell-cell fusion is observed between multiple mAbs and small molecules that bind to CCR5. This property may be broadly applicable to mAbs that target CCR5, including, for example, the mAb CCR5mAb004 that has been shown to bind to and antagonize CCR5 and block HIV-1 entry in a cell-cell fusion assay (Roschke et aL, 2004). A large and growing number of small molecules have been identified as CCR5 antagonists (see Table 11). Certain of these small molecule CCR5 antagonists may also produce synergistic inhibition of HIV-1 Env-mediated fusion in combination with PRO140 and other anti-CCR5 mAbs.

An alternative approach for examining synergistic interactions utilizes a virus-cell fusion assay as described previously (Nagashima et al., 2001; Trkola et al., 1998). In this assay an HIV genomic vector (pNLluc.sup.+Env.sup.-) containing a luciferase reporter gene is pseudotyped with Env from HIV.sup.-1.sub.JRFL. Recombinant pseudotyped virus particles are used to infect U87 cells expressing CD4 and CCR5 (U87-CD4-CCR5). Production of luciferase in target cells is dependent on virus entry and the completion of one round of virus replication. Drug susceptibility is measured by adding serial concentrations of drugs to target cells prior to addition of pseudotyped virus particles. Inhibition of virus entry is reflected in a reduction in luciferase activity in a dose-dependent manner, providing a quantitative measure of drug susceptibility. Since the HIV genomic vector requires expression of functional HIV-1 reverse transcriptase (RT) to drive luciferase expression, this pseudovirus assay is also sensitive to inhibition by nucleotide/nucleoside reverse transcriptase inhibitors (NRTIs) and non-nudeoside reverse transcriptase inhibitors (NNRTIs). As such, the HIV-1pp assay is suitable for examining cooperative interactions between PRO 140 and small-molecule, peptide and protein inhibitors of CCR5, CD4, HIV-1 gp120, HIV-1 gp41 and HIV-1 reverse transcriptase.

Table 11. Small-Molecule CCR5 antagonists

| Small Molecule CCR5 antagonist | Reference |
|---|---|
| 1,3,4-trisubstituted pyrrolidines | Kim et al., 2005 |
| Modified 4-piperidinyl -2-phenyl-1-(phenylsulfonylamino)-butanes | Shah et al., 2005 |
| Anibamine.TFA, Ophiobolin C, and 19,20-epoxycytochalasin Q | Jayasuriya et al., 2004 |
| 5-(piperidin-1-yl)-3-phenyl-pentylsulfones | Shankaran et al., 2004a |
| 4-(heteroarylpiperdin-1-yl-methyl)-pyrrolidin-1-yl-acetic acid antagonists | Shankaran et al., 2004b |
| Agents coining 4-(pyrazolyppiperidine side chains | Shu et al., 2004 |
| Agents containing 4-(pyrazolyl)piperidine side chains | Shen et al., 2004a; 2004b |
| 3-(pyrrolidin-1-yl)propionic acid analogues | Lynch et al., 2003c |
| [2-(R)-N-methyl-N-(1-(R)-3-(S)-((4-(3-benzyl-1-ethyl-(1H)-pyrazol-5-yl)piperidin-1-yl)methyl)-4-(S)-(3-fluorophenyl)cyclopent-1-yl)amino1-3-methylbutanoic acid (MRK-1) | Kumar et al., 2003 |
| 1,3,4 trisubstituted pyrrolidines bearing 4-aminoheterocycle substituted piperidine side chains | Willoughby et al., 2003; Lynch et al., 2003a; Lynch et al. 2003b; Hale et al., 2002 |
| Bicyclic isoxazolidines | Lunch et al., 2002 |
| Combinatorial synthesis of CCR5 antagonists | Willoughby et al., 2001 |

(continued)

| Small Molecule CCR5 antagonist | Reference |
|---|---|
| Heterocycle-containing compounds | Kim et al., 2001b |
| Antagonists containing hydantoins | Kim et al., 2001a |
| 1,3,4 trisubstituted pyrrolidines | Hale et al., 2001 |
| 1[N-(xnethyl)-N-(phenylsulfonypaminol-2-(phenyl)-4-(4-(N-(alkyl)-N-(benzytoxycarbonyl)amino)piperidin-1-yl)butanes | Finke et al., 2001 |
| Compounds from the plant Lippia alya | Hedge et al., 2004 |
| Piperazine-based CCR5 antagonists | Tagat et al., 2004 |
| Oximino-piperidino-piperidine-based CCR5 antagonists | Palani et al., 2003b |
| Rotamers of SCH 351125 | Palani et al., 2003a |
| Small-Molecule CCR5 antagonist | Reference |
| Piperazine-based symmetrical heteroaryl carboxamides | McCombie et al., 2003 |
| Oximino-pperidino-piperidine amides | Palani et al., 2002 |
| Sch-351125 and Sch-350634 | Este, 2002 |
| SCH-C | Strizki et al., 2001 |
| 1-[(2,4-dimethyl-3-pyridinyl)carbonyl]-4-methyl-4-[3(S)-methyl-4-[1(S) 44-(trifluoromethyl)phenyl]ethyl]-1-piperazinyl]-piperidine N1-oxide (Sch-350634) | Tagat et al., 2001a |
| 4-[(Z)-(4-bromophenyl)-(ethoxyimino)methyl 1-1'-[(2,4-dimethyl-3-pyridinyl) carbonyl]-4'-methyl-1,4'-bipiperidine N-oxide (SCH 351125) | Palani et al., 2001 |
| 2(S)-methyl piperazines | Tagat et al., 2001b |
| Piperidine-4-carboxamide derivatives | Imamura et al., 2005 |
| 1-benzazepine derivatives containing a sulfoxide moiety | Seto et al., 2005 |

A third approach for examining antiviral synergy utilizes a whole virus assay. Cooperativity between all classes of inhibitor molecules can be examined in this assay format.

In both the virus-cell fusion luciferase assay and the whole virus assay, IC.sub.50 values are generated for all combinations as described herein for the RET assay. Cooperative inhibition effects of drug combinations are determined by the method of Chou and Talalay (1984).

PRO 140 broadly and potently inhibited CCR5-mediated HIV-1 entry without CCR5 antagonism or other immunologic side effects in preclinical testing. More recently, PRO 140 has demonstrated favorable tolerability, PK and immunologic profiles in preliminary results from an ongoing Phase 1a study in healthy volunteers. Thus, in many respects, PRO 140 offers a novel and attractive product profile for anti-HIV-1 therapy. Moreover, the activities of and-CCR5 mAbs are fundamentally distinct from, but complementary to, those of small-molecule CCR5 antagonists (see Table 2).

It might have been expected that combinations of anti-CCR5 mAbs and non-antibody CCR5 antagonists would produce additive effects in inhibiting fusion of HIV-1 to CD4.sup.+CCR5.sup.+ target cells since both classes of agents bind to the same target molecule. Surprisingly, however, the data presented herein reveal that anti-CCR5 mAbs, exemplified by PRO 140 and 2D7, exhibited potent and reproducible synergy with non-antibody CCR5 antagonists, exemplified by SCH-D, TAK-779, UK-427,857 and RANTES, in inhibiting HIV-1 Env-mediated cell-cell fusion. Synergies routinely translated into 4- to 10-fold dose reductions, suggesting significant improvement in inhibitory potency for the drug combinations. In contrast, purely additive effects were observed for combinations of non-antibody CCR5 antagonists. These findings likely reflect the different patterns of CCR5 recognition of these molecules: whereas small-molecule CCR5 antagonists bind a common hydrophobic pocket within the transmembrane domains of CCR5, PRO 140 recognizes a hydrophilic, extracellular epitope of CCR5. Overall, the data support the use of PRO 140 in combination with non-antibody HIV-1 entry inhibitors and suggest that PRO 140 represents a distinct subclass of CCR5 inhibitor.

Moreover, the available data suggest that the observed synergy may also be exhibited by combinations involving anti-CCRS mAbs other than PRO 140, including, but not limited to, mAb CCR5mAb004 (Roschke et al., 2004), as well

as non-antibody CCR5 antagonists other than SCH-D, TAK-779, UK-427,857 and RANTES. Thus, these antibodies likely produce synergistic effects in combination with GW873140 (Lalezari et al., 2004), TAK-652 (Baba et al., 2005), and at least certain of the small-molecule CCR5 antagonists listed in Table 12. Accordingly, combination therapy comprising administration of anti-CCRS mAbs and non-antibody CCR5 antagonists may offer powerfully effective, new approaches to preventing and treating HIV-1 infection. It is expected that such therapy will result in more potent and more durable ant-HIV-1 treatments. Additionally, the synergistic effects described herein may enable a reduction in dosages of drugs administered to a subject as well as a reduction in dosing frequency.

EXAMPLE 4: Loading and Maintenance Dose Regimens

The loading regimen, which can, for example, be more dose-intensive than the maintenance regimen, can, for example, have the following characteristics:

Number of doses: 1 or more (up to about 5 doses).
Dose level: About 25%, 50%, 75%, 100%, 150% or 200% greater than the maintenance dose regimen.
Dose frequency: About 1.5.times., 2.times., 3.times. or 4.times. more frequently than the maintenance dose regimen.

As an example, if the maintenance dose regimen is 2 mg/kg every two weeks, the loading dose regimen could comprise weekly 2 mg/kg doses. Alternatively, the loading dose regimen could comprise a single 4 mg/kg dose or multiple 4 mg/kg doses at weekly or biweekly intervals.
The loading dose regimen can be designed, for example, so as to accelerate the achievement of a pharmacokinetic steady state in the subject, as defined by uniform peak and trough blood concentrations of drug between doses. A preferred loading dose regimen can be determined by routine experimentation wherein the drug is administered to the subject by differing loading and maintenance regimens, and blood levels of drug are measured.
Also, in another embodiment, PRO 140 is administered according to a fixed-dose regimen such as, for example, 75 mg, 150 mg, 300 mg and 600 mg per administration.

**Part III**

Materials And Methods

**Inhibitors**

PRO 140 was expressed in mammalian cells and purified by protein A, ion exchange and hydroxyapatite chromatographies. UK-427,857 (Dorr et al. 2005), SCH-D (Tagat et al. 2004), TAK-779 (Baba et al. 1999), enfuvirtide (T-20 (Wild et al. 1992); BMS-378806 (Lin et al. 2003)) and PRO 542 (CD4-IgG2, (Allaway et al. 1995)) were prepared according to published methods. Zidovudine (azidothymidine, AZT), RANTES, the CCR5 mAb 2D7 and the CD4 mAb Leu-3A were purchased from Sigma Chemicals (St Louis, Mo.), R&D Systems (Minneapolis, Minn.), Pharmingen (San Diego, Calif.), and Becton Dickinson (Franklin Lakes, NJ.), respectively. UK-427,857 and SCH-D were radiolabeled with tritium by GE Healthcare (Piscataway, N.J.), and PRO 140 was conjugated to phycoerythrin (PE) by Southern Biotech, Inc. (Birmingham, Ala.).

**HIV-1 Membrane Fusion Assay**

HIV-1 envelope-mediated membrane fusion was examined using a fluorescence resonance energy transfer (RET) assay (Litwin et al. 1996) with modifications. Briefly, HeLa cells that stably express HIV-1.subJR-FL gp120/gp41 (Litwin et al. 1996) and CEM.NKR-CCRS cells (NIH AIDS Research and Reference Reagent Program, (Spenlehauer et al. 2001; Trkola et al. 1999)) were labeled separately overnight with fluorescein octadecyl ester (F18; Molecular Probes, Eugene, Oreg.) and rhodamine octadecyl ester (R18; Molecular Probes), respectively. Cells were washed in phosphate-buffered saline containing 15% fetal bovine serum (PBSF) and co-seeded at 15,000 cells/well into a 384-well plate. Inhibitors were added, and the plates were incubated in PBSF plus 0.5% dimethlysulfoxide (DMSO) for 4 h at 37.degree. C. prior to measurement of RET using a Victor.sup.2 plate reader (Perkin-Elmer, Boston, Mass.) as previously described (Litwin et al. 1996). The CD4 mAb Leu3a was used as a control inhibitor, and percent inhibition was calculated as: (RET in the absence of inhibitor-RET in the presence of inhibitor)/(RET in the absence of inhibitor-RET in the presence of Leu3a).times.100.

## HIV-1 Pseudovirus Assay

A self-inactivating (SIN) vector was derived from the pNL4-3DELTAEnv-luciferase vector (Dragic et al. 1996) by deleting 507 basepairs in the U3 region of the 3' long terminal repeat (LTR) so as to remove the TATA box and transcription factor binding sites. The human cytomegalovirus promoter was inserted upstream of the luciferase (luc) gene to enable expression of luciferase following integration.

Reporter viruses pseudotyped with HTV-1.sub.JR-FL or HIV-1.sub.SF162 envelopes were generated by cotransfection of 293T cells with the SIN vector and the appropriate pcDNA env-expressing vector as previously described (Dragic et al, 1996). U87-CD4-CCR5 cells (8,000/well; NIH AIDS Research and Reference Reagent Program) were infected with 125-375 pg of HIV-1 pseudoviruses in 384-well plates in the presence or absence of inhibitors). Cultures were incubated for 72 h at 37.degree. C. in DMEM containing 10010 fetal bovine serum, 1 mg/mL puromycin, 0.3 mg/mL geneticin, antibiotics, and 0.5% DMSO. Luciferase activity (relative light units or RLU) was measured using BrightGlo reagent (Promega, Madison, Wis.) according to the manufacturer's instructions. Percent inhibition was calculated as: (1-RLU in the presence of inhibitor/RLU in the absence of inhibitor).times.100. IC50 and IC90 were used to denote the respective concentrations required for 50% and 90% inhibition of HIV-1.

## Synergy Determinations

Experimental design and data analysis were based on the combination index (CI) method (Chou et al. 1991; Chou et al. 1984). Compounds were tested individually and in combination at a fixed molar ratio over a range of serial dilutions. Entry inhibitors were combined in equimolar amounts, whereas a 1:10 molar ratio was used for PRO 140 In combination with azidothymidine and nevirapine. Dose-response curves were tit using a four-parameter sigmoidal equation with upper and lower inhibition values constrained to 100% and 0%, respectively, in order to calculate concentrations required for 50% (IC50) and 90% (IC90) inhibition (GraphPad Prism, GraphPad Software, San Diego, Calif). CI values for 50% (CI50) and 90% (CI90) inhibition were calculated as previously described (Chou et al. 1991; Chou et al. 1984). The mutually exclusive CI formula was used for combinations of CCR5 inhibitors, while the mutually non-exclusive formula was utilized for combinations of inhibitors to distinct targets (Chou et al. 1991). Each test was conducted 4-12 times. Synergy, additivity and antagonism are indicated by CI<1, CI=1 and CI>1, respectively.

## Competition Binding Assays

To examine inhibition of PRO 140 binding, CEM.NKR-CCR5 cells were suspended in phosphate-buffeted saline with 0.1% sodium azide (PBSA) and incubated with varying concentrations of unlabeled CCR5 antagonists at ambient temperature for 30 minutes. Azide was added to block CCR5 internalization during the assay. Cells were washed in PBSA and incubated with 5 nM PRO 140-PE for an additional 30 minutes prior to washing and analysis by flow cytometry using a FACSCalibur instrument (Becton Dickinson). The extent of PRO 140-PE binding was measured in terms of both the mean fluorescence intensity (MFI) and the percent of cells gated for positive staining.

To examine inhibition of UK-427,857 binding, CR5 cells were pre-incubated with unlabeled CCR5 inhibitors as described above prior to addition of 2 nM .sup.3H-UK-427,857 for an additional 30 minutes. The cells were washed in PBSA and lysed with 0.5N HCl prior to scintillation counting using a Wallac1410 instrument An additional study reversed the order of addition in order to examine the stability of UK-427,857 binding over the course of the assay. Cells were pre-incubated with 2 nM.sup.3H-UK-427,857 for 30 min prior to washing, addition of unlabeled inhibitors, and processing as described above. EC50 and EC90 were used to denote the concentrations of unlabeled compound required to inhibit binding of labeled compound by 50% and 90%, respectively.

## Statistical Analyses

Two-tailed t-tests were used to test mean CI50 and CI90 values for the null hypothesis H.sub.0: CI=1 (additivity) using GraphPad Prism software. P values were corrected for multiple comparisons from .alpha.=0.05 according to the Bonferroni method (Cudeck and O'Dell 1994), excluding the PRO 140/PRO .left brkt-bot.40 mock combination that was included as an assay control In the Bonferroni correction, P=.alpha/n, where n is the number of comparisons. Twenty-two synergy comparisons (11 compounds.times.2 CI values) were made based on data generated in the membrane fusion assay, resulting in a corrected P value of 0.0023. In the pseudovirus assay, 32 synergy comparisons (8 compounds.times.2 viruses.times.2 CI values) resulted in a corrected P value of 0.0016,

Results

**Inhibition of HIV-1 Membrane Fusion**

PRO 140 and UK-427,857 were used individually and together to inhibit HIV-1.sub.JR-FL envelope-mediated membrane fusion in the RET cell-cell fusion assay, and representative dose-response curves for the individual agents and combination are illustrated in FIG. 15A. Although both PRO 140 and UK-427,857 individually blocked HIV-1 fusion at low nanomolar potency, the combination was markedly more potent. In this assay, 50% inhibition was obtained using 2.9 nM PRO 140 alone, 5.0 nM UK-427,857 used alone, or 2.1 nM of the combination (1.05 nM PRO 140 plus 1.05 nM UK-427,857). This supra-additive effect is indicative of antiviral synergy between the two agents. In contrast, the combination of SCH-D and UK-427,857 was no more potent than individual agents (FIG. 15B). In this example, the dose-response curves for the individual inhibitors and the combination were overlapping, with 50% inhibition requiring 9.7 nM UK-427,857, 5.5 nM SCH-D and 6.1 nM of the combination. The data suggest purely additive effects for these inhibitors.

These studies were extended to additional CCR5 (TAK-779, RANTES and 2D7), gp120 (BMS-378806 and PRO 542) and gp41 (enfuvirtide) inhibitors, and were repeated four or more times for each condition. CI50 and CI90 values were calculated for each condition and averaged across the independent assays. Cooperativity was assessed using t-tests to determine if the CI50 and CI90 values were significantly different from one. As a test of these methods, a PRO 140/PRO 140 mock combination was examined by adding PRO 140 to the assay wells in two separate additions. CI50 and CI90 values for the PRO 140/PRO 140 combination were 0.96 and 0.97, respectively (Table 12); therefore, purely additive effects were observed for this mock combination, as expected.

Table 12. CI values for inhibition of HIV-1.sub.JR-FL envelope-mediated membrane fusion[a]

| 1st Inhibitor | Target | IC50, nM | IC90, nM | 2nd Inhibitor | CI50 | P value | C190 | P value |
|---|---|---|---|---|---|---|---|---|
| PRO 140 | CCR5 | 2.5 | 8.6 | PRO 140 | 0.97 ± 0.07 | 0.13 | 0.96 ± 0.14 | 0.37 |
| UK-427,857 | CCR5 | 5.3 | 27 | PRO 140 | 0.61 ± 0.5 | <0.0001 | 0.40 ± 0.06 | <0.0001 |
| SCH-D | CCR5 | 3.2 | 16 | PRO 140 | 0.51 ± 0.05 | <0.0001 | 0.36 ± 0.06 | <0.0001 |
| TAK-779 | CCR5 | 11 | >200 | PRO 140 | 0.38 ± 0.08 | <0.0001 | N/A | N/A |
| RANTES | CCR5 | 2.4 | 38 | PRO 140 | 0.59 ± 0.08 | 0.0022 | 0.43 ± 0.05 | 0.0002 |
| RANTES | CCR5 | 2.4 | 38 | UK-427,857 | 0.48 ± 0.03 | 0.0017 | 0.18 ± 0.01 | <0.0001 |
| SCH-D | CCR5 | 3.2 | 16 | UK-427,857 | 0.86 ± 0.03 | 0.016 | 0.75 ± 0.02 | 0.0033 |
| SCH-D | CCR5 | 3.2 | 16 | TAK-779 | 1.3 ± 0.18 | 0.12 | N/A | N/A |
| 2D7 | CCR5 | 3.7 | 58 | PRO 140 | 1.0 ± 0.14 | 0.61 | 1.9 ± 0.61 | 0.024 |
| enfuvirtide | gp41 | 8.6 | 66 | PRO 140 | 0.84 ± 0.16 | 0.040 | 0.89 ± 0.20 | 0.19 |
| PRO 542 | gp120 | 8.9 | 91 | PRO 140 | 0.96 ± 0.17 | 0.56 | 0.94 ± 0.19 | 0.45 |
| BMS-378806 | gp120 | 5.2 | 20 | PRO 140 | 1.3 ± 0.19 | 0.0015 | 1.1 ± 0.22 | 0.19 |

[a] Statistically significant results (P < 0.0023 after application of the Bonferroni correction for multiple comparisons) are indicated in italicized bold text IC50 and IC90 denote values for the 1.sup.st inhibitor. N/A = not applicable; TAK-779 did not consistently achieve 90% inhibition in the assay. CI values represent the means and standard deviations of 4-12 independent assay

Potent synergy was observed for PRO 140 in combination with each of three small-molecule CCR5 antagonists (UK-427,857, SCH-D and TAK-779), and the findings were statistically significant even when the data were corrected for multiple comparisons via the Bonferroni method (Table 13). CI values ranged from 0.36 to 0.61, and these synergies translated into dose reductions ranging from 3- to 8-fold across the different conditions. Synergies were greater at 90% inhibition than at 50% inhibition. Synergy between PRO 140 and small-molecule CCR5 antagonists was robust in that it was observed at both the 50% and 90% inhibition levels in every instance. The exception was TAK-779, which did not mediate 90% inhibition when used individually, and therefore a CI90 was not determined. Similarly potent synergy was observed when RANTES was used in combination with either PRO 140 or UK-427,857 (Table 12). Additional tests examined combinations of two small-molecule CCR5 antagonists (SCH-D/UK-427,857 and SCH-D/TAK-779) or two CCR5 mAbs (PRO 140/2D7). No significant synergy was observed for these combinations, although the SCH-D/UK-427,857 CI90 values trended towards significance. The findings are consistent with prior observations of overlapping binding sites for PRO 140 and 2D7 (Olson et al. 1999) and for SCH-D and TAK-779 (Seibert et al. 2006). PRO 140 was also tested in combination with the gp41 fusion inhibitor enfuvirtide and with the gp120 attachment inhibitors PRO 542 and BMS-378806 (Table 13). CI values ranged from 0.84 to 128, and none of these combinations demonstrated synergy

that met the criteria for statistical significance. For the PRO 140/BMS-378806 combination, modest antagonism was observed at 50% but not 90% inhibition. The biological significance of this result is unclear.

**Inhibition of HIV-1 Pseudoviruses**

Single-cycle HIV-1 reporter viruses were used to examine whether the synergistic effects were limited to cell-cell fusion or whether they extended to other modes of HIV-1 entry. Signals in this assay require both viral entry and reverse transcription, so that both NRTI and NNRTI may be included in the analyses. Each combination was tested against reporter viruses pseudotyped with envelopes from HIV-1.sub.JR-FL and HIV-1.sub.SF162 in at least 4 independent assays per virus. A PRO 140/PRO 140 mock combination was again included as an assay control, and demonstrated additive effects against both HIV-1.sub.JR-FL and HIV-1.sub.SF162 pseudoviruses, as expected (Table 13).

PRO 140 potently synergized with both UK-427,857 and SCH-D in blocking virus-cell fusion, and the results met the criteria for statistical significance. Comparable levels of synergy were observed against both HIV-1.sub.JR-FL and HIV-1.sub.SF162 pseudoviruscs at 50% and 90% inhibition (Table 14), with CI values ranging from 0.18 to 0.64. These synergies translated into dose reductions ranging to 14-fold These results are in good agreement with those obtained in the cell-cell fusion assay (Table 13). Neither TAK-779 nor RANTES mediated consistent, high-level inhibition of HIV-1 pseudovirus entry, and therefore these compounds were not included in this analysis (data not shown).

Table 13. CI values for inhibition of HIV-1 reporter viruses pseudotyped with envelopes from HIV $-1_{JR-FL}$ andHIV-$1_{SF162}$.[a]

| 1st Inhibitor | Target | HIV-1 Envelope | IC50, nM | IC90, nM | 2nd Inhibitor | CI50 | P value | CI190 | P value |
|---|---|---|---|---|---|---|---|---|---|
| PRO 140 | CCR5 | JRFL | 22 | 28 | PRO 140 | 12 ± 0.32 | 0.16 | 0.90 ± 0.15 | 0.047 |
| | | SF162 | 1.3 | 20 | PRO 140 | 1.0 ± 027 | 1.0 | 0.86 ± 033 | 021 |
| SCH-D | CCR5 | JRFL | 2.4 | 44 | PRO 140 | 0.47 ± 0.15 | <0.001 | 0.18 ± 0.04 | <0.001 |
| | | SF162 | 0.34 | 14 | PRO 140 | 0.60 ± 0.17 | <0.001 | 0.28 ± 0.11 | <0.001 |
| UK- | CCR5 | JRFL | 7.4 | 46 | PRO 140 | 0.44 ± 0.06 | <0.001 | 0.24 ± 0.11 | <0.001 |
| 427,857 | | SF162 | 0.87 | 13 | PRO 140 | 0.64 ± 007 | <0.001 | 0.31 ± 0.11 | <0.001 |
| UK- | CCR5 | JRFL | 7.4 | 46 | PRO 140 | 0.71 ± 0.11 | 0.16 | 12 ± 0.15 | 032 |
| 427,857 | | SF162 | 0.87 | 13 | PRO 140 | 0.87 ± 0.06 | 0.19 | 0.86 ± 028 | 0.61 |
| 2D7 | CCR5 | JRFL | 8.8 | >200 | PRO 140 | 15 ± 025 | 0.024 | N/A | N/A |
| | | SF162 | 22 | 74 | PRO 140 | 1.1 ± 0.47 | 0.61 | 1.0 ± 0.16 | 0.65 |
| PRO 542 | gp120 | JRFL | 0.19 | 2.9 | PRO 140 | 12 ± 0.32 | 022 | 1.0 ± 0.18 | 0.92 |
| | | SF162 | 0.36 | 7.1 | PRO 140 | 0.98 ± 028 | 0.84 | 0.64 ± 026 | 0.010 |
| BMS- | gp120 | JRFL | 12 | 11 | PRO 140 | 12 ± 0.38 | 0.43 | 0.74 ± 023 | 0.059 |
| 378806 | | SF162 | 0.03 | 0.42 | PRO 140 | 1.1 ± 028 | 036 | 0.82 ± 021 | 0.068 |
| nevirapine | RT | JRFL | 30 | 310 | PRO 140 | 12 ± 0.38 | 036 | 0.73 ± 028 | 0.068 |
| | | SF162 | 42 | 280 | PRO 140 | 12 ± 0.34 | 030 | 0.63 ± 0.19 | 0.033 |

(continued)

| 1st Inhibitor | Target | HIV-1 Envelope | IC50, nM | IC90, nM | 2nd Inhibitor | CI50 | P value | CI190 | P value |
|---|---|---|---|---|---|---|---|---|---|
| zidovudine | RT | JRFL | 140 | 1900 | PRO 140 | 1.1 ± 0.38 | 037 | 0.85± 026 | 021 |
| | | SF162 | 86 | 2100 | PRO 140 | 0.99 ± 027 | 0.91 | 1.0 ± 0.38 | 1.0 |

[a] Statistically significant results (P < 0.0016 after application of the Bonferroni correction for multiple comparisons) are indicated in italicized bold text IC50 and IC90 refer to values for the 1.sup.st inhibitor. N/A = not applicable; 2D7 did not consistently achieve 90% inhibition in the assay. CI values represent the means and standard deviations of 4 or more independent assays

Additive effects were observed for both the UK-427,857/SCH-D and PRO 140/2D7 combinations (Table 13). Similarly, additivity was observed for PRO 140 used in combination with the gp120 inhibitors PRO 542 and BMS-378806. No antagonism was observed for the PRO 140/BMS-378806 combination against either virus. Overall, these findings are consistent with those seen for cell-cell fusion Lastly, additive effects were observed for PRO 140 in combination with either zidovudine (NRTI) or nevirapine (NNRTI).

Competition Binding Studies

As described above, additive antiviral effects were observed for inhibitors known (PRO 140 and 2D7) or inferred (UK-427,857 and SCH-D) to compete for CCR5 binding; however, little is known regarding the competitive binding of synergistic compounds (e.g., PRO 14Q/UK-427,857 and PRO 140/SCH-D). Since non-competitive binding provides a possible mechanism for synergy between CCR5 inhibitors, this issue was explored using labeled forms of UK-427,857 and PRO 140.

Flow cytometry was used to examine inhibition of PRO 140-PE binding to CEM.NRK.CCR5 cells by unlabeled PRO 140, UK-427,857 and SCH-D. PRO 140-PE binding was efficiently inhibited by unlabeled PRO 140, as expected. Complete inhibition was observed in terms of both MFI values (FIG. 16A) and the percent of cells gated for positive binding (FIG. 16B). The EC50 based on MR data was 2.5 nM (FIG. 16A), and this value compares favorably with the antiviral IC50 of PRO 140 (Tables 13 and 14). Since percent cells gated is a readout for essentially complete inhibition of binding, an EC90 value was calculated as 17 nM, and this value is similar to the antiviral IC90 values observed for PRO 140 (Tables 13 and 14). 2D7 also completely inhibited binding of PRO 140-PE to CEM.NKR-CCR5. The CCR5 specificity of PRO 140-PE was also demonstrated by its inability to bind parental CEM.NKR cells.

In sharp contrast, modest levels of inhibition were observed for UK-427,857 and SCH-D (FIG. 16). Micromolar concentrations of UK-427,857 and SCH-D reduced PRO 140-PE MFI values by 50% or less (FIG. 16A). More dramatically, UK-427,857 and SCH-D had little impact on the percent of cells gated for positive binding of PRO 140-PE (FIG. 16B). The findings suggest that UK-427,857 and SCH-D partially reduce the number of PRO 140-PE molecules bound per cell; however, these compounds do not reduce the number of cells that bind measurable amounts of PRO 140-PE. Therefore, UK-427,857 and SCH-D represent partial antagonists of PRO 140 binding, and this finding provides a mechanism for the antiviral synergy observed between PRO 140 and these small-molecule CCR5 antagonists.

Inhibition of .sup.3H-UK-427,857 binding by unlabeled UK-427,857, SCH-D and PRO 140 was next examined. Binding of.sup.3H-UK-427,857 to CEM.NKR-CCR5 cells was efficiently inhibited by unlabeled UK-427,857 (FIG. 17A). The EC50 for binding was 4.3 nM and is similar to the antiviral IC50 values observed for UK-427,857 (Tables 12 and 13).

SCH-D also blocked .sup.3H-UK-427,857 binding to background levels (FIG. 17A). However, there was no correlation between the compounds' antiviral potency and their potency in blocking .sup.3H-UK-427,857 binding. For example, whereas SCH-D demonstrated equal or slightly greater antiviral potency than UK-427,857 (Tables 12 and 13), SCH-D was less potent in blocking .sup.3H-UK-427,857 binding (EC50=17 nM, FIG. 17A). This result is consistent with minor differences in the CCR5 binding sites of these compounds.

Surprisingly, PRO 140 also blocked .sup.3H-UK-427,857 binding to background levels (FIG. 17A), and this result contrasts with the modest inhibition of PRO 140-PE binding by UK-427,857 (FIG. 16). PRO 140 inhibited .sup.3H-UK-427,857 binding with an EC50 of 14 nM, which is 5-10 fold higher than the antiviral IC50 of PRO 140 (Tables 13 and 14).

A final experiment examined the stability of UK-427,857 binding to CEM.NKR-CCR5 cells under the conditions of the competition assay. For this, cells were pre-incubated with .sup.3H-UK-427,857 and then the dissociation was examined in the presence of unlabeled UK-427,857, SCH-D and PRO 140. As indicated in FIG. 17B, there was minimal dissociation of .sup.3H-UK-427,857 over 30 min at ambient temperature, and UK-427,857 wasn't displaced by either PRO 140 or SCH-D. Therefore, the inability of UK-427,857 to efficiently compete PRO 140 binding to CCR5 (FIG. 16)

is not due to rapid dissociation of UK-427,857 from CCR5 during the course of the assay. Collectively, the data indicate that PRO 140 can bind CCR5 in the presence of pre-bound UK-427,857.

Discussion

This study explores interactions between mAb and small-molecule CCR5 inhibitors and examines combinations of CCR5 drugs that currently are in development for HIV-1 therapy. Surprisingly, potent antiviral synergy between the CCR5 mAb PRO 140 and each of three structurally distinct small-molecule CCR5 antagonists was observed. Consistent, high-level synergy was observed across varying assay systems, viral isolates, target cells and inhibition levels. PRO 140 and small-molecule CCR5 antagonists were more potently synergistic when used together rather than in combination with inhibitors that block other stages of HIV-1 entry. In contrast, additive effects were observed for combinations of two small-molecule CCR5 antagonists. Competition binding studies revealed complex and non-reciprocal patterns of CCR5 binding by mAb and small-molecule CCR5 inhibitors, and suggest that the synergistic interactions occur at the level of receptor binding.

Robust synergy between mAb and small-molecule CCR5 inhibitors was observed in this study. Potent synergy was observed for both cell-cell and virus-cell fusion, and there was a good concordance of findings in these two well-established assay systems. Comparable levels of synergy were observed for PRO 140 in combination with each of 3 small-molecule CCR5 antagonists from unrelated chemical series. In addition, consistent synergy was observed for each of two well-characterized HIV-1 envelopes and two CCR5 target cells. Synergy increased with increasing levels of viral inhibition and translated into *in vitro* dose reductions of up to 14-fold. Viewed alternatively, this degree of synergy provides a corresponding increase in antiviral pressure at a given concentration of drugs, thereby improving viral suppression and potentially delaying the emergence of drug-resistant virus. This is supported by preliminary studies indicating the mAb and small-molecule CCR5 inhibitors possess complementary patterns of viral resistance (Kuhmann et al. 2004 and Marozsan et al. 2005). The present findings provide a rationale for clinical exploration of regimens that combine mAb and small-molecule CCR5 inhibitors.

Potent synergy was also observed for RANTES used in combination with either UK-427,857 or PRO 140. Endogenous levels of RANTES may afford some protection against HIV-1 disease progression during natural infection (Garzino-Demo et al. 1999; Lui et al. 1999), and therefore this finding of synergy has important and positive implications for CCR5-targetedtherapies of HIV-1. Antiviral synergy between RANTES and PRO 140 is not surprising based on a prior observation that RANTES signaling is not blocked by antiviral concentrations of murine PRO 140 (PA14) (Olson et al. 1999). Synergy between RANTES and UK-427,857 is less easily explained given that UK-427,857 is a potent CCR5 antagonist. However, these findings are consistent with prior observations of synergy between the small-molecule CCR5 antagonist SCH-C and aminooxypentane-RANTES (AOP-RANTES) (Tremblay et al. 2002), a RANTES derivative that has been evaluated as a potential topical microbicide (Kawamura et al. 2000).

In contrast to the robust synergy observed between mAb and small-molecule CCR5 antagonists, additive effects were observed for combinations of small-molecule CCR5 antagonists. Lack of cooperativity is consistent with the view that these molecules compete for binding to a common pocket on CCR5 (Dragic et al. 2000; Nishikawa et al. 2005; Tsamis et al. 2003; Watson et al. 2005). The *in vitro* studies do not provide a basis for combining small-molecule CCR5 antagonists in the clinic based solely on inhibition of wild-type virus.

Similarly, potent synergy was not observed between PRO 140 and inhibitors of HIV-1 attachment (PRO 542 and BMS-378806), fusion (enfuvirtide), or reverse transcriptase (zidovudine and nevirapine), and these findings underscore the significance of the synergy observed for PRO 140 and small-molecule CCR5 antagonists. A number of prior studies have examined interactions between various small-molecule CCR5 antagonists (UK-427,857, SCH-C, TAK-220, TAK-652 and E913) and drugs from each of the existing HIV-1 treatment classes. Most (Tremblay et al. 2005 Antivir. Ther.; Tremblay 35 et al. 2005 Antimicrob. Agents Chemother; Tremblay et al. 2002) but not all (Dorr et al. 2005; Maeda et al. 2001) studies have reported broad synergy between CCR5 inhibitors and the other HIV-1 treatment classes, and the divergent results may reflect differences in the compounds and methods used for antiviral testing as well as differences in the methods used for data analysis. When UK-427,857 was tested against 20 licensed antiretroviral agents, additive effects were observed in all but three cases, where modest synergy was reported (Dorr et al. 2005). This result is consistent with the present findings for combinations of PRO 140 and HIV-1 inhibitors that do not target CCR5.

Without intending to be bound by theory, synergy between anti-HIV-1 drugs may stem from a variety of mechanisms. In mixed virus cultures, one compound may inhibit virus resistant to a second compound (Johnson et al. 1991), andNRTUNNRTI combinations may overcome specific RT-mediated resistance mechanisms (Basavapathrani et aL 2004; Borkow et aL 1999). Metabolic interactions between inhibitors may increase their effective intracellular drug concentrations (Molla et aL 2002), and synergistic entry inhibitors may disrupt interdependent steps in the entry cascade (Nagashima et al. 2001; Tremblay et al. 2000). The present study examined clonal viral envelopes rather than mixed populations, and the extracellular nature of the target argues against metabolic interactions. Multiple domains of gp120 contribute to CCR5 binding (Cormier et al. 2002), but it is unclear at present whether these interactions represent separate

or discrete events during infection.

The present findings indicate that antiviral synergy between mAb and small-molecule CCR5 inhibitors may occur at the level of the receptor. As discussed above, mAbs and small molecules bind distinct loci on CCR5 (Dragic et al. 2000; Nishikawa et al. 2005; Tsamis et al. 2003; Olson et al. 1999; Watson et al. 2005). When pre-incubated with CCR5 cells in the present study, PRO 140 completely blocked subsequent binding of UK-427,857 to the receptor; although the PRO 140 concentrations were higher than those needed to block HIV-1 entry into the same cells. In contrast, pre-incubation of CCR5 cells with super-saturating concentrations of UK-427,857 or SCH-D reduced PRO 140 binding by 50% or less. As one possible explanation, PRO 140 could recognize CCR5 conformers that are not bound by UK-427,857 or SCH-D. Although cell-surface CCR5 exists in multiple conformations (Lee et al. 1999), it seems unlikely that the small-molecule antagonists could demonstrate potent antiviral activity while failing to bind a significant fraction of cell-surface CCR5. In this regard, it is important to note that a common cellular background (CEM.NKR-CCR5 cells) was used for competition binding and antiviral studies, and therefore the findings are not related to cell-specific differences in CCR5 expression.

Without intending to be bound by theory, another plausible explanation for the present findings is that PRO 140 is capable of forming a ternary complex with UK-427,857-bound CCR5, and this ternary complex provides an increased barrier to HIV-1 entry. Within the context of this model, PRO 140 may bind UK-427,857-bound CCR5 somewhat less efficiently than free CCR5, as evidenced by the modest reduction in PRO 140 binding in the presence of UK-427,857.

The combination index method is widely used to assess drug-drug interactions. In this method, cooperativity often is defined on the basis of empirical CI values (e.g., <0.9 for synergy and >1.1 for antagonism) irrespective of inter-assay variability. Statistical analyses are performed infrequently, and even more rarely are adjustments made for multiple comparisons. In the absence of such analyses, there is increased potential to overestimate the number of synergistic combinations.

A rigorous and conservative approach to identifying synergistic effects was employed CI values were tested for statistical significance against the null hypothesis of additivity (CI=1). In addition, these studies determined 20-30 different CI values per experiment (Tables 13 and 14), as is common in synergy studies. In order to reduce the potential for spurious positive results, the significance level was reduced using the Bonferroni correction. A mock combination was also evaluated as a test of these methods for antiviral testing and data analysis. It was therefore concluded that numerous apparent synergies (CI<0.9) could not be distinguished from inter-assay variation based on the available data. However, despite the rigorous nature of these methods, PRO 140 and small-molecule inhibitors demonstrated significant synergy under every test condition, lending credence to this finding. Combinations with CI values that trended towards significance in the present survey could be explored in future studies. For example, data for the PRO 140/enfuvirtide combination suggested modest synergy that trended towards significance; thus this combination may also be useful for treating HIV-1 infection.

A growing body of data indicates that mAb and small-molecule CCR5 antagonists represent distinct subclasses of CCR5 inhibitors, and a number of important parallels can be drawn between NRTI and NNRTI on the one hand and between mAb and small-molecule CCR5 antagonists on the other. In each instance, there are distinct binding loci for the inhibitors on the target protein (reverse transcriptase or CCR5). One set of inhibitors (NNRTI or small-molecule CCR5 antagonists) acts via allosteric mechanisms, while the other set (NRTI or CCR5 mAbs) acts as a competitive inhibitor. Like NRTI and NNRTI, mAb and small-molecule CCR5 inhibitors are synergistic and possess complementary patterns of viral resistance *in vitro* in preliminary testing (Kuhmann et al. 2004; Marozsan et al. 2005). NRTI and NNRTI represent important and distinct treatment classes even though they target the same protein, and mAb and small-molecule CCR5 inhibitors similarly may offer distinct HIV-1 treatment modalities.

**Part IV**

Materials And Methods

PRO 140 and small-molecule CCR5 antagonists were prepared and/or obtained as described herein above. The primary R5 HIV-1 isolates JR-FL and Case C 1/85 (CC1/85) were passaged weekly *in vitro* on peripheral blood mononuclear cells (PBMCC) in the presence or absence of progressively increasing concentrations of PRO 140 or SCH-D, and viral cultures were examined for susceptibility to these and other CCR5 inhibitors. For susceptibility testing, viruses were cultured *in vitro* on stimulated PBMC. In the presence and absence of serially diluted drug, and the extent of viral replication was determined by p24 EUSA.

Results

For both JR-FL and CC1/85, drug-resistant variants were generated in the presence of PRO 140 and SCH-D. At passage 12, the escape mutants were approximately 10-to 100-fold less susceptible to the drug used for selection.

In each case, the escape mutants continued to require CCR5 for replication on PBMC. Complementary patterns of resistance were observed: SCH-D escape mutants were efficiently inhibited by PRO 140 and PRO 140 escape mutants were efficiently inhibited by SCH-D.

Discussion

PRO 140 escape mutants continue to require CCR5 for entry and remain susceptible to small-molecule CCR5 antagonists. In addition, PRO 140 is active against viruses resistant to small-molecule CCR5 antagonists. These findings indicate that PRO 140 and small-molecule CCR5 antagonists may represent distinct subclasses of CCR5 inhibitors.

REFERENCES

U.S. Pat. No. 4,816,567, issued Mar. 28,1989 to Cabilly et al.
U.S. Pat No. 5,225,539, issued JuL 6,1993 to Gregory Winter.
U.S. Pat. No. 5,229,275, issued Jul. 20, 1993 to Goroff.
U.S. Pat. No. 5,545,806, issued Aug. 13, 1996 to Lonberg et aL
U.S. Pat. No. 5,545,807, issued Aug. 13, 1996 to Surani et al.
U.S. Pat. No. 5,565,332, issued Oct. 15,1996 to Hoogenboom et al.
U.S. Pat No. 5,567,610, issued Oct. 22,1996 to Borrebaeck et al.
U.S. Pat No. 5,585,089, issued Dec. 17, 1996 to Queen et al.
U.S. Pat No. 5,591,669, issued Jan. 7, 1997 to Krimpenfort et al.
U.S. Pat No. 5,693,761, issued Dec. 2,1997 to Queen et al.
U.S. Pat No. 6,150,584, issued Nov. 21, 2000 to Kucherlapati et al.
U.S. Pat No. 6,476,034 B2, issued Nov. 5, 2002 to Wang et al.
U.S. Pat. No. 6,759,519 B2, issued JuL 6, 2004 to Li et al.
PCT International Publication No. WO 90/07861, published JuL 26,1990.
PCT International Publication No. WO 00/35409, published Jun. 22, 2000.
PCT International Publication No. WO 01/55439, published Aug. 2, 2001.
PCT International Publication No. WO 01/90106 A2, published Nov. 29,2001.
PCT International Publication No. WO 02/22077, published Mar. 21, 2002.
PCT International Publication No. WO 01/62255 A1, published Aug. 30, 2001.
PCT International Publication No. WO 03/082289 A1,published Oct. 9, 2003.
Alkhatib, G., et al. (1996) Science 272: 1955.
Alonzo III, F., et aL (2013) Nature 493(7430): 51-55.
Allaway, G. P., et al. (1993) AIDS Res. Hum. Retrovir. 9: 581-587.
Allaway, G. P., et aL (1995) AIDS Research and Human Retroviruses. 11: 533-539.
Asano, S., et aL (2014) ACS Med. Chem. Lett. 5: 133-137.
Baba, M., et al. (2005) 12th Conference on Retroviruses and Opportunistic Infections. Boston, Mass., Feb. 22-25, 2005, Abstract 541.
Baba, M, et al. (1999) Proc. Nab. Acad. Sci. USA 96: 5698-5703.
Balotta, C, P. et aL (1997) AIDS 11: F67-F71.
Basavapathruni, A., et al. (2004) J Biol Chem. 279:6221-6224
Berger, E. A. (1997) AIDS 11 (Suppl A): S3-S16.
Bieniasz, P. D. and B. R. Cullen (1998) Frontiers in Bioscience 3: d44-58.
Biti, R., R. et al. (1997) Nature Med. 3: 252-253.
Borkow, G., et al, (1999) Antimicrob. Agents Chemother. 43:259-263
Buddy, L., et aL (1992) J. Immunol. 149: 1779-1787.
Buddy, L., et al. (1995) J. ViroL 69: 4267-4273.
Choe, H., et al. (1996) Cell 85: 1135-1148.
Chou, T. C. and D. C. Rideout (1991) Synergism and antagonism in chemotherapy. Academic Press, New York.
Chou, T. C. and P. Talalay (1984) Adv. Enzyme Regulation 22: 27-55.
Cocchi, F., et al. (1995) Science 270: 1811-1815.
Combadiere, C, et al. (1996) J. Leukocyte BioL 60: 147-152.
Connor, R. I., et al. (1997) J. Exp. Med. 185: 621-628.
Cormier, E. G. and T. Dragic (2002) J ViroL 76:8953-8957.
Cudeck, R. and L. L. ODell (1994) Psychol. Bull. 115:475-487.

Dalgleish, A. G., et al. (1984) Nature 312: 763-766.
Demarest, J., et aL (2004) 11th Conference on Retroviruses and Opportunistic Infections, Abstract 139. San Francisco, Calif., Feb. 8-11, 2004.
Deng, H., et al. (1996) Nature 381: 661-666.
Dorr, P., et al. (2003) 10th Conference on Retroviruses and Opportunistic Infections, Boston, Mass., Feb. 10-14,2003, Paper #12.
Dorr, T, P., et al. (2005) Antimicrobial Agents and Chemotherapy 49:4721-4732.
Dragic, T., et aL (1997) Advances in Research and Therapy 7: 2-13.
Dragic, T., et al. (1992) J. ViroL 66: 4794-4802.
Dragic, T., et al. (1996) Nature 381: 667-673.
Dragic, T., et al. (2000) Proc Nab Acad Sci USA 97:5639-44.
Este J A. (2002) Cum Opin. Investig. Drugs. 3: 379-383.
Fatkenheuer, G., et al. (2005) Nat Med 11:1170-1172.
Feng, Y., et al. (1996) Science 272: 872-877.
Finke, P. E. et al. (2001) Bioorg. Med. Chem Lett. 11: 2475-2479.
Garzino-Demo, A., et al. (1999) Proc Natl Acad Sci USA. 96:11986-11991.
Haim-Boukobza, S., et al. (2013) Journal of Hepatology 59: 613-615.
Hale, J. J. et al. (2001) Bioorg. Med. Chem. Lett 11: 2741-2745.
Hale, J. J. et aL (2002) Bioorg. Med. Chem. Lett. 12: 2997-3000.
Hegde, V. R. et al. (2004) Bioorg. Med. Chem. Lett. 12: 5339-5342.
HGS Press Release (2004) Human Genome Sciences characterizes panel of novel human monoclonal antibodies that specifically antagonize the CCR5 receptor and block HIV-1 entry. Nov. 2, 2004. HGS Press Release (2005) Human Genome Sciences begins dosing of patients in a phase 1 clinical trial of CCR5 mAb in patients infected with HIV-1. Mar. 30, 2005.
Huang, Y., et al. (1996) Nature Med. 2: 1240-1243.
Huffnagle, G. B., et al. (1999) Immunol. 163: 4642-4646.
Y. Iizawa, et al. (2003) 10th Conference on Retroviruses and Opportunistic Infections. Boston, Mass., Feb. 10-14, 2003
Imamura, S. et al. (2004a) Bioorg. Med. Chem 12: 2295-2306.
Imamura, S. et al. (2004b) Chem. Pharm. Bull. (Tokyo) 52: 63-73.
Imamura, S. et al. (2005) Bioorg. Med. Chem 13: 397-416.
Jayasuriya, H. et al. (2004) J. Nat. Prod. 67: 1036-1038.
Johnson, V. A., et al. (1991) Journal of Infectious Diseases 164:646-655.
Kawamura, T., et aL (2000) J Exp Med. 192:1491-1500.
Kuhmann, S. E., et al. (2004) J Virol 78:2790-2807.
Ketas, T. J., et al. (2003) J. Virol. 77: 2762-2767.
Kim D. et aL (2001a) Bioorg. Med. Chem Lett. 11: 3099-3102.
Kim D. et al. (2001b) Bioorg. Med. Chem. Lett. 11: 3103-3106.
Kim D. et al. (2005) Bioorg. Med. Chem Lett. 15: 2129-2134.
Klatzmann, D., et aL (1984) Nature 312: 382-385.
Koyanagi, Y., et al. (1987) Science 236: 819-822.
Kuhmann, S. E. et al. (2004) J. ViroL 78: 2790-2807.
Kumar, Set al (2003) J. Pharmacol Exp. Ther. 304: 1161-1171.
Laal, S., et al. (1994) J. Virol. 68: 4001-4008.
Lalezari, J. P., et al. (2003) New Engl. J. of Med. 348: 2175-2185.
Lalezari, J., et al. (2004) 44th Annual Interscience Conference on Antimicrobial Agents and Chemotherapy, Abstract 2871, Washington, D.C., Oct 30-Nov. 2, 2004.
Lalezari, J., et aL (2005) AIDS 19:1443-1448.
Lapidot, T. (2001) Ann. N.Y. Acad. Sci. 938: 83-95.
Lazzarin, A., et al. (2003) New Engl. J. Med. 348: 2186.
Lee, B., et al. (1999) Journal of Biological Chemistry 274:9617-9626.
Li, A., et al. (1997) AIDS Res. Hum. Retrovir. 13: 647-656.
Li, A., et al. (1998) J. ViroL 72: 3235-3240.
Lin, P. F., et al. (2003) Proc. Natl. Acad. Sci. USA 100: 11013-11018.
Lin, P. F., et al. (2002) 9th Conference on Retroviruses and Opportunistic Infections. Seattle, Wash., Feb. 24-28, 2002
Littman, D. R. (1998) Cell 93: 677-680.
Litwin, V., et al. (1996) J. Virol. 70: 6437-6441.
Liu, R., et al. (1996) Cell 86: 367-377.

Liu, H., at aL (1999) Proceedings of the National Academy of Sciences of the United States of America 96:4581-4585
Lynch, C. L. et al. (2003a) Bioorg. Med. Chem Lett 12: 3001-3004.
Lynch, C. L. at al. (2003b) Bioorg. Med. Chem. Lett. 13: 119-123.
Lynch, C. L. et al. (2002) Bioorg. Med. Chem. Lett. 12: 677-679.
Lynch, C. L. et al. (2003c Org. Lett. 5: 2473-2475.
Maddon, P. J., et al. (1986) Cell 47: 333-348.
Maeda, K. et al. (2004) J. Virol. 78: 8654-8662.
Maeda, K. et al. (2001) J. BioL Chem. 276: 35194-35200.
Marozsan, A. J. et al. (2005) Virology 338: 182-199.
McCombie, S. W. et al. (2001) Bioorg. Med. Chem. Lett. 13: 567-571.
McDougal, J. S., et al. (1986) Science 231: 382-385.
Merluzzi, V. J., et al. (1990) Science 250: 1411-1413.
Michael, N. L., et al. (1997) Nature Med. 3: 338-340.
Molla, A., et al. (2002) Antimicrob. Agents Chemother. 46:2249-2253.
Moore, J. P., Q. J. Sattentau, P. J. Klasse and L. C. Burkly (1992) J. Virol. 66: 4784-4793.
Murga, J., et al. (2006) Antimicrobial Agents and Chemotherapy: 3289-3296.
Nagashima, K. A., at al. (2001) J. Infect Dis. 183: 1121-1125.
Nakata, H. et al. (2005) J. ViroL 79: 2087-2096.
Nishikawa, M., et al. (2005) Antimicrob. Agents Chemother. 49: 4708-4715.
O'Brien, T. R., et al. (1997) Lancet 349:1219.
Olson, W. C. et al. (2009) Curr Opin HIV AIDS 4(2): 104-111.
Olson, W. C, et al. (1999) J. ViroL 73: 4145-4155.
Olson, W. C. and P. J. Maddon (2003) Current Drug Targets-Infectious Disorders 3:283-294.
Palani, A, et al. (2002) J. Med. Chem. 45: 3143-3160.
Palani, A, et al. (2001) J. Med. Chem 44: 3339-3342.
Palani, A, et al. (2003a) Bioorg. Med. Chem Lett. 13: 705-708.
Palani, A, et al. (2003b) Bioorg. Med. Chem. Lett 13: 709-712.
Palella, F. J., et al. (1998) The New England Journal of Medicine 338:853.
Palmer, L. A., et al. (2010) Biol Blood Marrow Transplant 16(3): 311-319.
Raport, C. J., et al. (1996) J. Leukocyte Biol. 59: 18-23.
Ray, N. and R. W. Doms (2006) Curr. Top. Microbiol Immunol. 303:97-120.
Reshef, R., et al. (2012) N Engl J Med. 367(2): 135-145.
Reshef, R., et al. (2011) Biology of Blood and Marrow Transplant 17: S331.
Reyes, G. (2001) Development of CCR5 antagonists as a new class of anti-HTV therapeutic. 8th Conference on Retroviruses and Opportunistic Infections. Chicago, Ill., Feb. 5, 2001.
Reynes, J., et al. (2002) SCH C: Safety and antiviral effects of a CCR5 receptor antagonist in HIV-1 infected subjects. 9th Conference on Retroviruses and Opportunistic Infections. Seattle, Wash., Feb. 25, 2002
Robinson, B. S., et al. (2000) Antimicrob. Agents Chemother. 44: 2093-2099.
Roschke, V., et al. (2004) 44th Annual Interscience Conference on Antimicrobial Agents and Chemotherapy, Abstract 2871, Washington, D.C., Oct. 30-Nov. 2, 2004, Abstract #2871.
Samson, M., at al. (1997) J. BioL Chem 272: 24934-24941.
Schecter, A. D., et al. (2000) J. BioL Chem. 275: 5466-5471.
Schols, D., et al. (1997) J. Ex. Med. 186: 1383-1388.
Schuh, J. M., et al. (2002) FASEB J, 16: 228-230.
Schurmann, D., et al. (2004) Abstract 140LB, San Francisco, Calif., Feb. 8-11, 2004.
Seibert, C., et aL (2006) Virology 349(1):41-54.
Seto, M. et aL (2005) Bioorg. Med. Chem. Left. 13: 363-386.
Seto, M. et aL (2004a) Chem Pharm. Bull. (Tokyo). 52: 818-829.
Seto, M. et aL (2004b) Chem. Pharm. Bull. (Tokyo). 52: 577-590.
Shah, S. K. et al. (2005) Bioorg. Med. Chem. Lett. 15: 977-982.
Shankaran, K. et al. (2004a) Bioorg. Med. Chem. Lett. 14: 3589-3593.
Shankaran, K. et al. (2004b) Bioorg. Med. Chem Lett. 14: 3419-3424.
Shen, D. M. et aL (2004a) Bioorg. Med. Chem. Lett. 14: 935-939.
Shen, D. M. et al. (2004b) Bioorg. Med. Chem. Lett. 14: 941-945.
Shiraishi, M., et al. (2000) J. Med. Chem. 43: 2049-2063.
Shu, M. et aL (2004) Bioorg. Med. Chem Lett. 14: 947-52.
Si, Z., et al. (2004) Proc. Natl. Acad. Sci. USA 101: 5036-5041.
Simmons, G., et al. (1996) J. ViroL 70: 8355-8360.

Spenlehauer, C., et al. (2001) Virology 280:292-300.
Strizki, J. M. et al. (2001) Proc. Natl. Acad. Sci. USA. 98: 12718-12723.
Sung, A. D., et al. (2013) Best Pract Res Clin Haematol: 26(3).
Sung, A. D., et al. (2013) Stem Cells Translational Medicine 2: 25-32.
Tagat, J. R. et al. (2001a) J. Med. Chem. 44: 3343-3346.
Tagat, J. R. et al. (2001b)Bioorg. Med. Chem. Lett. 11: 2143-2146.
Tagat, J. R., et al. (2004) J. Med. Chem. 47: 2405-2408.
Takashima, K., et al. (2005) Antimicrob. Agents Chemother. 49:374-3482.
Thali, M., et al. (1992) J. Acquir. Immune Defic. Syndr. 5: 591-599.
Thoma, G. et al. (2004) J. Med. Chem. 47: 1939-1955.
Tilley, S. A., et al. (1992) AIDS Res. Hum. Retrovir. 8: 461-467.
Tran, E. H., et al. (2000) Eur. J. ImmunoL 30: 1410-1415.
Tremblay, C., et al. (2000) Journal of Acquired Immune Deficiency Syndromes and Human Retrovirology 25:99-102
Tremblay, C. L., et al. (2002) Antimicrobal Agents and Chemotherapy 46:1336-1339.
Tremblay, C. L., et al. (2005) 12th Conference on Retroviruses and Opportunistic Infections. Boston, Mass., Feb. 22-25, 2005, Abstract 542.
Tremblay, C. L., et aL (2005) Antivir. Ther. 10:967-968.
Tremblay, C. L., et aL (2005) Antimicrob. Agents Chemother. 49:3483-3485.
Trkola, A., et al. (2001) J. Virol. 75: 579-588.
Trkola, A., et aL (1999) Journal of Virology 73:8966-8974.
Trkola, A., et aL (1998) J. ViroL 72: 1876-1885.
Tsamis, F., et aL (2003) Journal of Virology 77:5201-5208.
Vijh-Warrier, S., et aL (1996) J. Virol. 70: 4466-4473.
Watson, C., et aL (2005) Mol Pharmacol. 67:1268-1282.
Westby, M, et aL (2010) Antiviral Chemistry & Chemotherapy 20: 179-192.
Wild, C., et aL (1992) PNAS 89:10537-10541.
Willoughby, C. A. et al. (2001) Bioorg, Med. Chem. Lett. 11: 3137-41.
Willoughby, C. A. et al. (2003) Bioorg. Med. Chem. Lett. 13: 427-431.
Wu, L., et al, (1997) J. Exp. Med. 186: 1373-1381.
Zhou, Y., et aL (1998) J. ImmunoL 160: 4018-4025.
Zhu, P., et aL (2001) J. ViroL 75: 6682-6686.

The present application also provides aspects according to the following numbered paragraphs:

1. A combined dosage regime comprising an anti-CCR5 receptor monoclonal antibody comprising (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:HuPRO140 HG2-VH (ATCC Deposit Designation PTA-4098) or the plasmid designated pVg4:HuPRO140 (mut B+D+I)-VH (ATCC Deposit Designation PTA-4099) and a small molecule CCR5 receptor antagonist, wherein the small molecule CCR5 receptor antagonist binds a hydrophobic pocket within the transmembrane domains of a CCR5 receptor, and wherein the combined dosage regime permits one of a dose reduction of the anti-CCR5 receptor monoclonal antibody and a dose reduction of the non-antibody CCR5 receptor antagonist.
2. The combined dosage regime of paragraph 1, wherein the anb-CCR5 receptor monoclonal antibody is a humanized, human, or chimeric antibody.
3. The combined dosage regime of paragraph 1, wherein the anb-CCR5 receptor monoclonal antibody is PRO 140.
4. The combined dosage regime of paragraph 3, wherein the PRO 140 comprises one of an intravenous infusion dosage form and a subcutaneous injection dosage form.
5. The combined dosage regime of paragraph 1, wherein the small molecule CCR5 receptor antagonist comprises an oral dosage form.
6. A method of potentiating the GVHD inhibitory activity of a small molecule CCR5 receptor antagonist in a subject who has been administered, or will be administered, the small molecule CCR5 receptor antagonist in the treatment of the subject's GVHD, comprising administering to the subject an inhibitory amount of an anti-CCR5 receptor monoclonal antibody, wherein the amounts of the anti-CCR5 receptor monoclonal antibody and the small molecule CCR5 receptor antagonist combined, produces a synergistic therapeutic effect on treating GVHD, thereby potentiating the GVHD inhibitory activity of the small molecule CCR5 receptor antagonist, wherein the anti-CCR5 receptor monoclonal antibody is PRO 140, said PRO 140 comprising (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated

pVg4:HuPRO140 HG2-VH (ATCC Deposit Designation PTA-4098) or the plasmid designated pVg4:HuPRO140 (mut B+D+I)-VH (ATCC Deposit Designation PTA-4099) and wherein the small molecule CCR5 receptor antagonist binds a hydrophobic pocket within the transmembrane domains of the CCR5 receptor.

7. The method of paragraph 6, wherein the anti-CCR5 receptor monoclonal antibody is a humanized, human, or chimeric antibody.

8. The method of paragraph 6, wherein the PRO 140 is administered via one or intravenous infusion and subcutaneous injection.

9. The method of paragraph 6, wherein the small molecule CCR5 receptor antagonist is orally administered.

10. The method of paragraph 6, wherein the anb-CCR5 receptor monoclonal antibody is administered concurrently with administration of the small molecule CCR5 receptor antagonist.

11. The method of paragraph 6, wherein the anti-CCR5 receptor monoclonal antibody is administered prior to administration of the small molecule CCR5 receptor antagonist.

12. The method of paragraph 6, wherein the anti-CCR5 receptor monoclonal antibody is administered subsequent to administration of the small molecule CCR5 receptor antagonist.

13. The method of paragraph 6, wherein the anti-CCR5 receptor monoclonal antibody administered dose delivers between 75 mg and 600 mg of the anti-CCR5 receptor monoclonal antibody.

14. The method of paragraph 6, wherein the anti-CCR5 receptor monoclonal antibody administered dose delivers one of 75 mg, 150 mg, 300 mg, and 600 mg of the anti-CCR5 receptor monoclonal antibody.

15. The method of paragraph 6, wherein the non-antibody CCR5 receptor antagonist provides for an approximately four-fold to ten-fold dose reduction of the anti-CCR5 receptor monoclonal antibody.

16. The method of paragraph 6, wherein the non-antibody CCR5 receptor antagonist provides for an approximately three-fold to sixteen-fold dose reduction of the non-antibody CCR5 receptor antagonist.

17. The method of paragraph 6, wherein the synergistic effect comprises a reduction in GVHD progression in the subject that is maintained for at least one week.

18. A method of preventing, treating, or mitigating GVHD in a subject, comprising administering to the subject an inhibitory amount of an anti-CCR5 receptor monoclonal antibody, wherein the amounts of the anti-CCR5 receptor monoclonal antibody and wherein the anti-CCR5 receptor monoclonal antibody is PRO 140, said PRO 140 comprising (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:HuPRO140 HG2-VH (ATCC Deposit Designation PTA-4098) or the plasmid designated pVg4:HuPRO140 (mut B+D+I)-VH (ATCC Deposit Designation PTA-4099).

19. The method of paragraph 18, wherein the PRO 140 is administered via one or intravenous infusion and subcutaneous injection..

20. The method of paragraph 18, wherein the anti-CCR5 receptor monoclonal antibody is administered concurrently with administration of a small molecule CCR5 receptor antagonist.

## Claims

1. An anti-CCR5 receptor monoclonal antibody, wherein the anti-CCR5 receptor monoclonal antibody is PRO 140, said PRO 140 comprising (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:HuPRO140 HG2-VH (ATCC Deposit Designation PTA-4098) or the plasmid designated pVg4:HuPRO140 (mut B+D+I)-VH (ATCC Deposit Designation PTA-4099) for use in preventing, treating, or mitigating graft-versus-host disease (GVHD) in a subject.

2. The anti-CCR5 receptor monoclonal antibody for use of claim 1, wherein the anti-CCR5 receptor monoclonal antibody is administered concurrently with administration of a small molecule CCR5 receptor antagonist selected from the group consisting of (1-[(4,6-dimethyl-5-pyrimidinyl) carbonyl]-4-[4-[2-methoxy-1(R)-4-(trifluoromethyl)phenyl]ethyl-3(S)-methyl-1-piperazinyli-4-methylpiperidine)) (UK-427,857) having the structure:

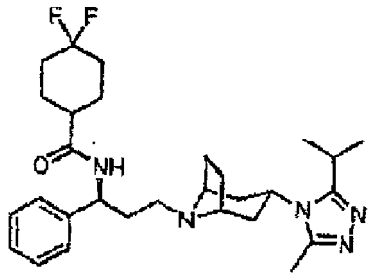

TAK-779 having the structure:

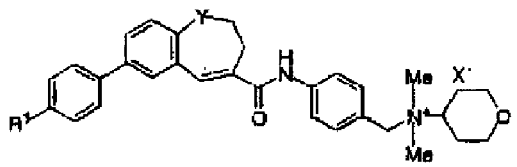

where Y = $-CH_2$, X = -C1, R1 = $-CH_3$; TAK-652 having the structure:

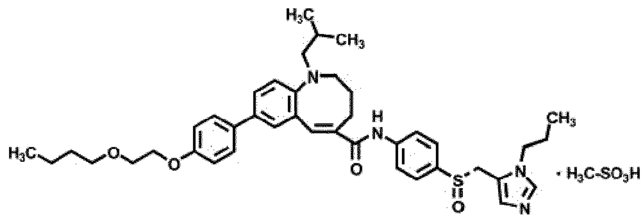

SCH-D having the structure:

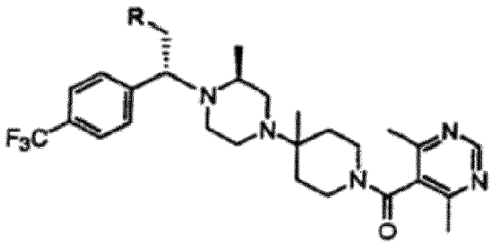

where R = $CH_3$ (S, S) or R = $OCH_3$ (R, S); or GW873140.

**3.** The anti-CCR5 receptor monoclonal antibody for use of claim 2, wherein the use comprises potentiating the GVHD inhibitory activity of the small molecule CCR5 receptor antagonist, wherein the amounts of the anti-CCR5 receptor monoclonal antibody and the small molecule CCR5 receptor antagonist combined, produces a synergistic therapeutic effect on treating GVHD, thereby potentiating the GVHD inhibitory activity of the small molecule CCR5 receptor antagonist, and wherein the small molecule CCR5 receptor antagonist binds a hydrophobic pocket within the transmembrane domains of the CCR5 receptor.

**4.** An anti-CCR5 receptor monoclonal antibody for use in preventing, treating, or mitigating GVHD in a subject, wherein the anti-CCR5 receptor monoclonal antibody is PRO 140, said PRO 140 comprising (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:HuPRO140 HG2-VH (ATCC Deposit Designation PTA-4098) or the plasmid designated pVg4:HuPRO140 (mut B+D+I)-VH (ATCC Deposit Designation PTA-4099),
wherein the use comprises potentiating the GVHD inhibitory activity of a small molecule CCR5 receptor antagonist in a subject who has been administered, or will be administered, the small molecule CCR5 receptor antagonist in the treatment of the subject's GVHD,

wherein the subject is administered an inhibitory amount of the anti-CCR5 receptor monoclonal antibody, wherein the amounts of the anti-CCR5 receptor monoclonal antibody and the small molecule CCR5 receptor antagonist combined, produces a synergistic therapeutic effect on treating GVHD, thereby potentiating the GVHD inhibitory activity of the small molecule CCR5 receptor antagonist, and wherein the small molecule CCR5 receptor antagonist binds a hydrophobic pocket within the transmembrane domains of the CCR5 receptor; and wherein the small molecule CCR5 receptor antagonist is selected from the group consisting of (1-[(4,6-dimethyl-5-pyrimidinyl)carbonyl]-4-[4-[2-methoxy-1(R)-4-(trifluoromethyl)phenyl]ethyl-3(S) methyl-1-piperazinyli-4-methylpiperidine)) (UK-427,857) having the structure:

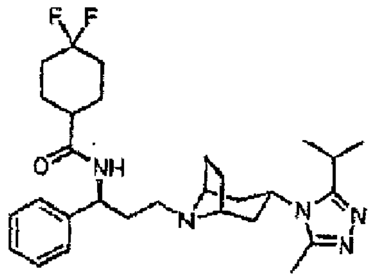

TAK-779 having the structure:

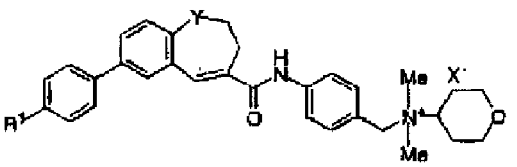

where Y = $-CH_2$, X = -C1, R1 = $-CH_3$;
TAK-652 having the structure:

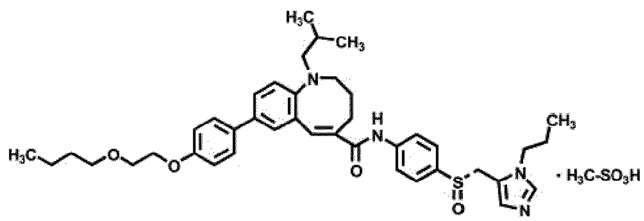

SCH-D having the structure:

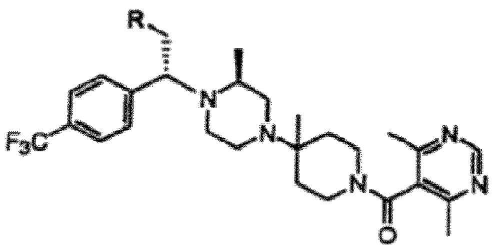

where R = $CH_3$ (S, S) or R = $OCH_3$ (R, S); or GW873140.

**5.** The anti-CCR5 receptor monoclonal antibody for use of claim 4, wherein the small molecule CCR5 receptor antagonist is orally administered.

**6.** The anti-CCR5 receptor monoclonal antibody for use of claim 4, wherein the anti-CCR5 receptor monoclonal antibody is administered concurrently with administration of the small molecule CCR5 receptor antagonist, or wherein the anti-CCR5 receptor monoclonal antibody is administered prior to administration of the small molecule CCR5 receptor

antagonist, or wherein the anti-CCR5 receptor monoclonal antibody is administered subsequent to administration of the small molecule CCR5 receptor antagonist.

**7.** The anti-CCR5 receptor monoclonal antibody for use of claim 4, wherein the anti-CCR5 receptor monoclonal antibody administered dose delivers between 75 mg and 600 mg of the anti-CCR5 receptor monoclonal antibody, or wherein the anti-CCR5 receptor monoclonal antibody administered dose delivers one of 75 mg, 150 mg, 300 mg, and 600 mg of the anti-CCR5 receptor monoclonal antibody.

**8.** The anti-CCR5 receptor monoclonal antibody for use of claim 4, wherein the small molecule CCR5 receptor antagonist provides for a four-fold to ten-fold dose reduction of the anti-CCR5 receptor monoclonal antibody, or wherein the anti-CCR5 receptor monoclonal antibody provides for a three-fold to sixteen-fold dose reduction of the small molecule CCR5 receptor antagonist.

**9.** The anti-CCR5 receptor monoclonal antibody for use of claim 4, wherein the synergistic effect comprises a reduction in GVHD progression in the subject that is maintained for at least one week.

**10.** A combined dosage regime for use in preventing, treating or mitigating GVHD in a subject comprising:

an anti-CCR5 receptor monoclonal antibody wherein the anti-CCR5 receptor monoclonal antibody is PRO 140, said PRO140 comprising (i) two light chains, each light chain comprising the expression product of the plasmid designated pVK:HuPRO140-VK (ATCC Deposit Designation PTA-4097), and (ii) two heavy chains, each heavy chain comprising the expression product of either the plasmid designated pVg4:HuPRO140 HG2-VH (ATCC Deposit Designation PTA-4098) or the plasmid designated pVg4:HuPRO140 (mut B+D+I)-VH (ATCC Deposit Designation PTA-4099);and,
a small molecule CCR5 receptor antagonist,

wherein the small molecule CCR5 receptor antagonist binds a hydrophobic pocket within the transmembrane domains of a CCR5 receptor, and wherein the combined dosage regime permits one of a dose reduction of the anti-CCR5 receptor monoclonal antibody and a dose reduction of the small molecule CCR5 receptor antagonist, and
wherein the small molecule CCR5 receptor antagonist is selected from the group consisting of (1-[(4,6-dimethyl-5-pyrimidinyl)carbonyl]-4-[4-[2-methoxy-1(R)-4-trifluoromethyl) phenyl]ethyl-3(S)-methyl-1-piperazinyli-4-methylpiperidine)) (UK-427,857) having the structure:

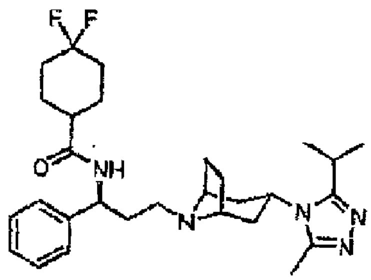

TAK-779 having the structure:

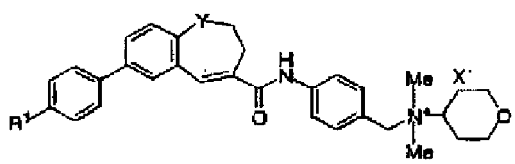

where Y = $-CH_2$, X = -C1, R1 = $-CH_3$;
TAK-652 having the structure:

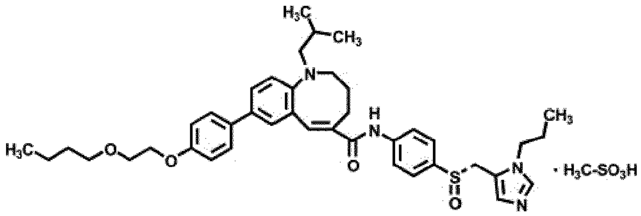

SCH-D having the structure:

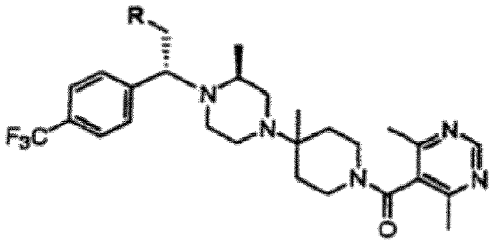

where R = $CH_3$ (S, S) or R = $OCH_3$ (R, S); or GW873140.

**11.** The combined dosage regime for use according to claim 10, wherein the small molecule CCR5 receptor antagonist comprises an oral dosage form.

**12.** The anti-CCR5 receptor monoclonal antibody for use of any of claims 1 or 4, or the combined dosage regime for use according to claim 10, wherein the PRO 140 comprises one of an intravenous infusion dosage form and a subcutaneous injection dosage form.

**13.** The combined dosage regime for use according to claim 10 wherein the anti CCR5 receptor monoclonal antibody and small molecule CCR5 receptor antagonist are administered in the same composition

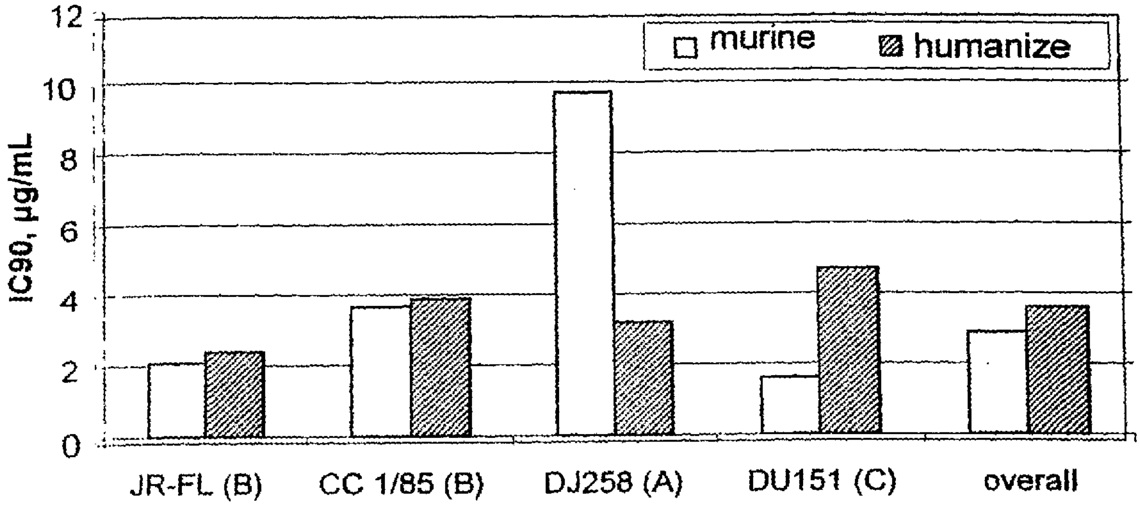
12
10
8
6
4
2
0
IC90, µg/mL
murine
humanize
JR-FL (B)
CC 1/85 (B)
DJ258 (A)
DU151 (C)
overall

Figure 1

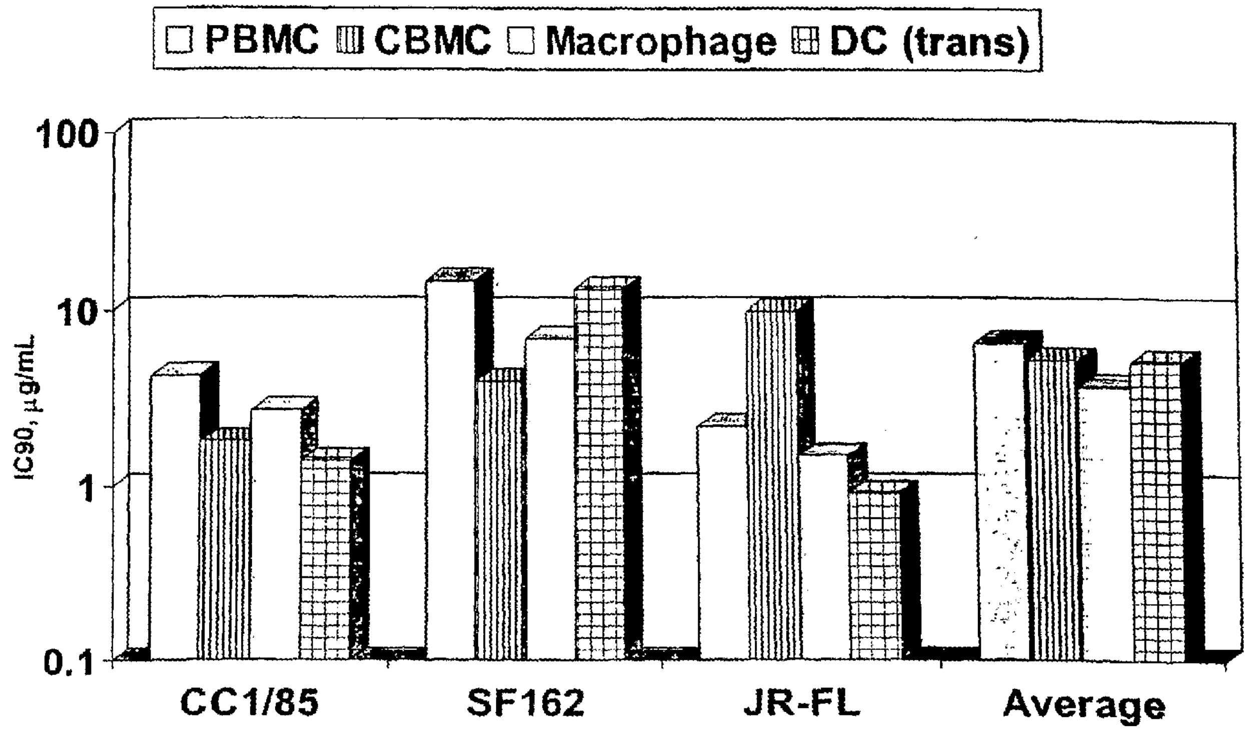

PBMC
CBMC
Macrophage
DC (trans)
100
10
1
0.1
IC90, μg/mL
CC1/85
SF162
JR-FL
Average


Figure 2

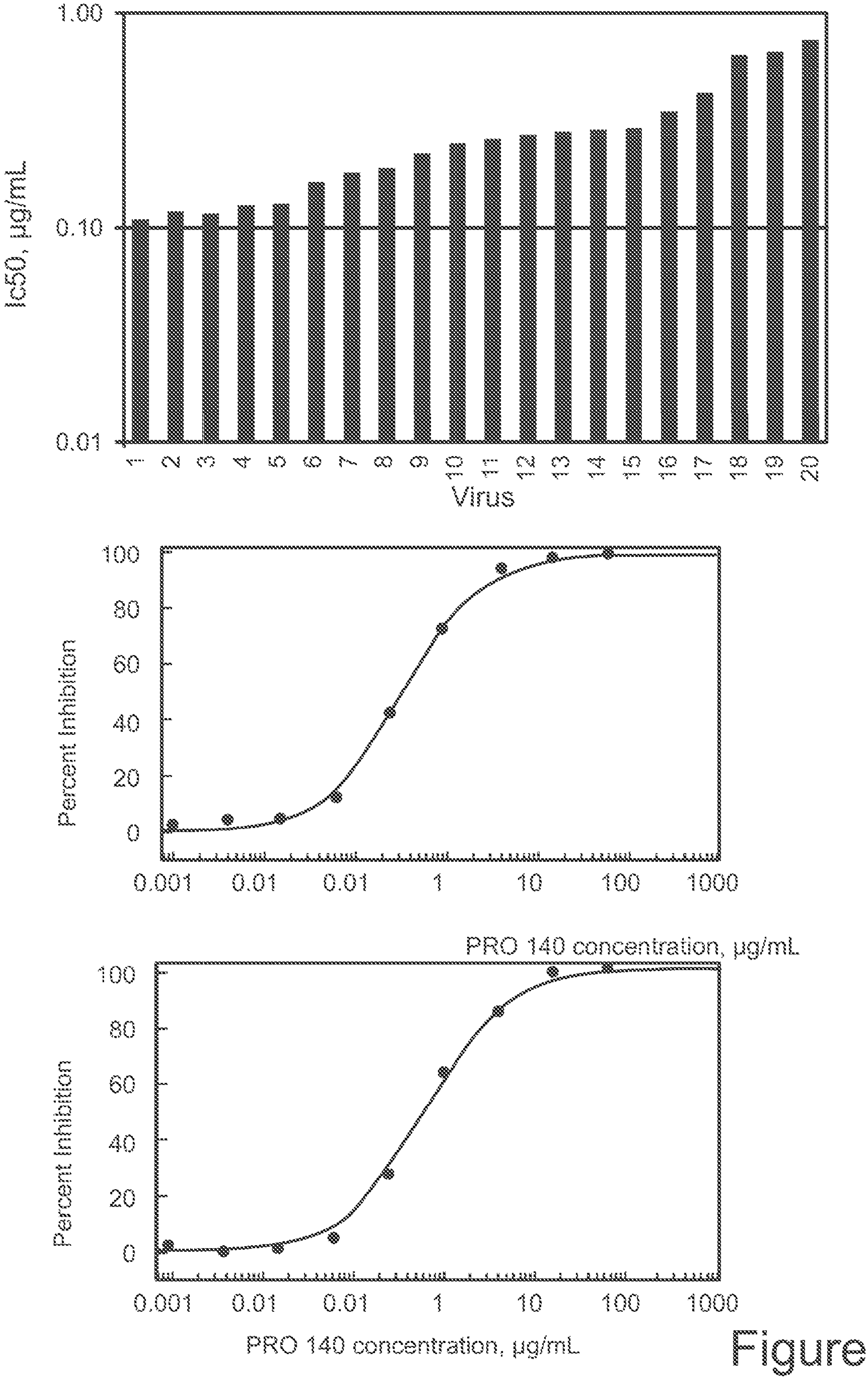
1.00
0.10
0.01
Ic50, µg/mL
1
2
3
4
5
6
7
8
9
10
11
12
13
14
15
16
17
18
19
20
Virus
100
80
60
40
20
0
Percent Inhibition
0.001
0.01
0.01
1
10
100
1000
PRO 140 concentration, µg/mL
100
80
60
40
20
0
Percent Inhibition
0.001
0.01
0.01
1
10
100
1000
PRO 140 concentration, µg/mL

Figure 3

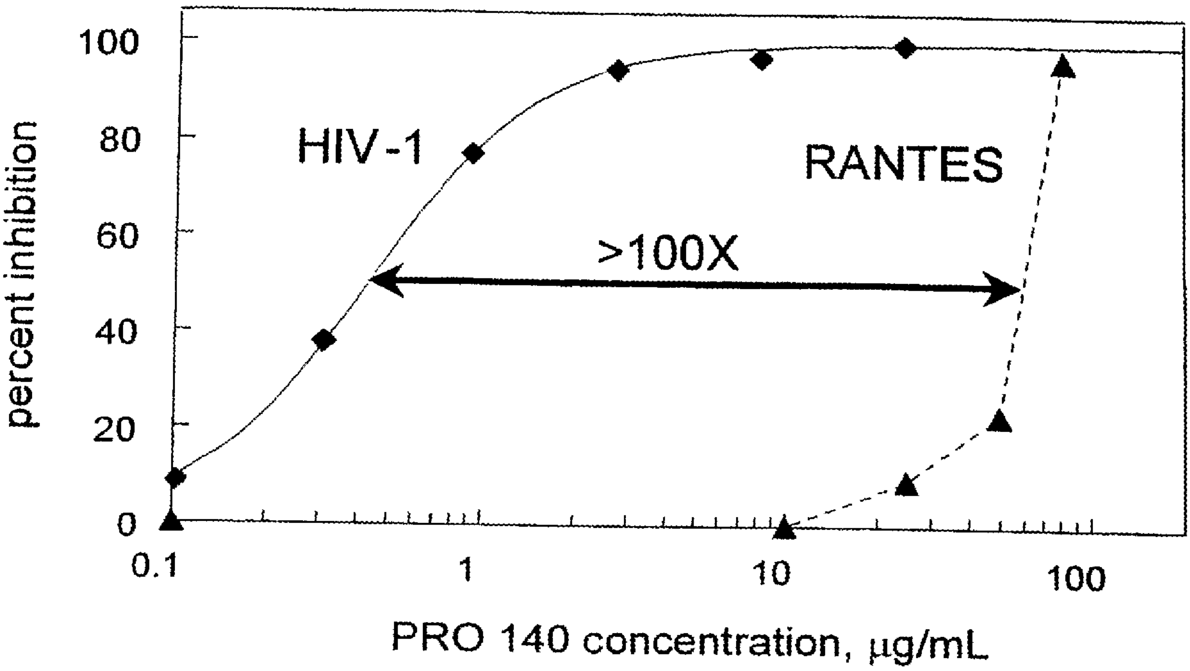
100
80
60
40
20
0
percent inhibition
HIV-1
RANTES
>100X
0.1
1
10
100
PRO 140 concentration, µg/mL

Figure 4

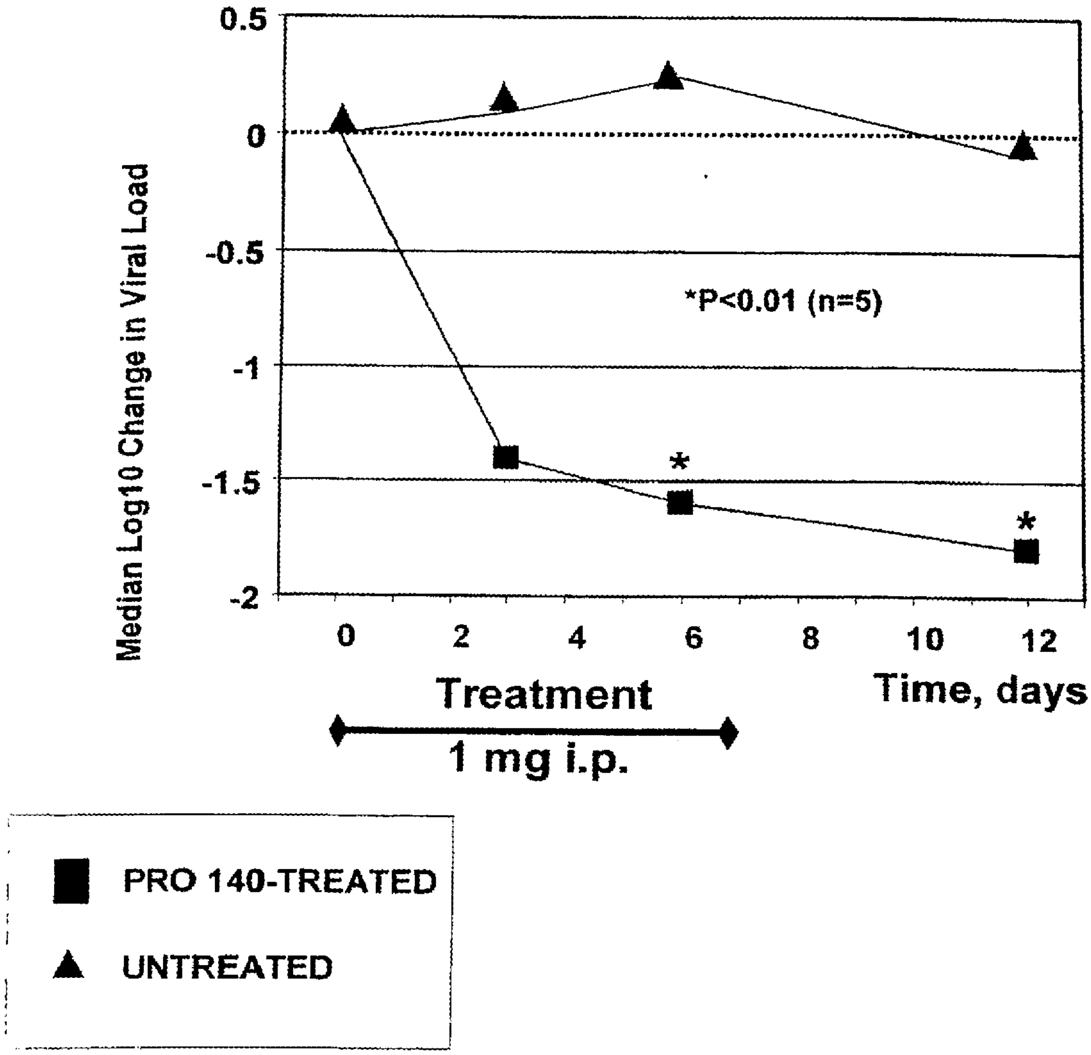
0.5
0
-0.5
-1
-1.5
-2
Median Log10 Change in Viral Load
*P<0.01 (n=5)
*
*
0
2
4
6
8
10
12
Treatment
Time, days
1 mg i.p.
PRO 140-TREATED
UNTREATED

Figure 5

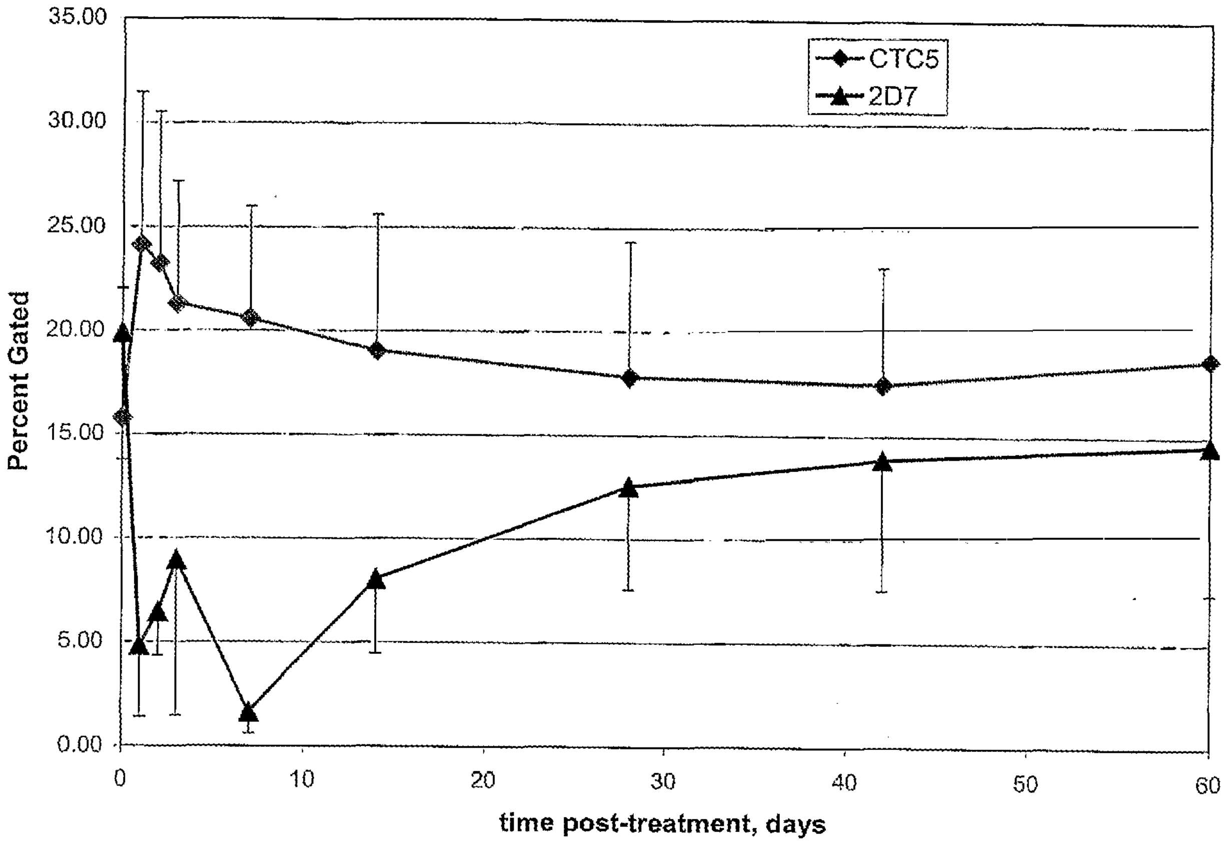
CTC5
2D7
Percent Gated
35.00
30.00
25.00
20.00
15.00
10.00
5.00
0.00
0
10
20
30
40
50
60
time post-treatment, days

Figure 6

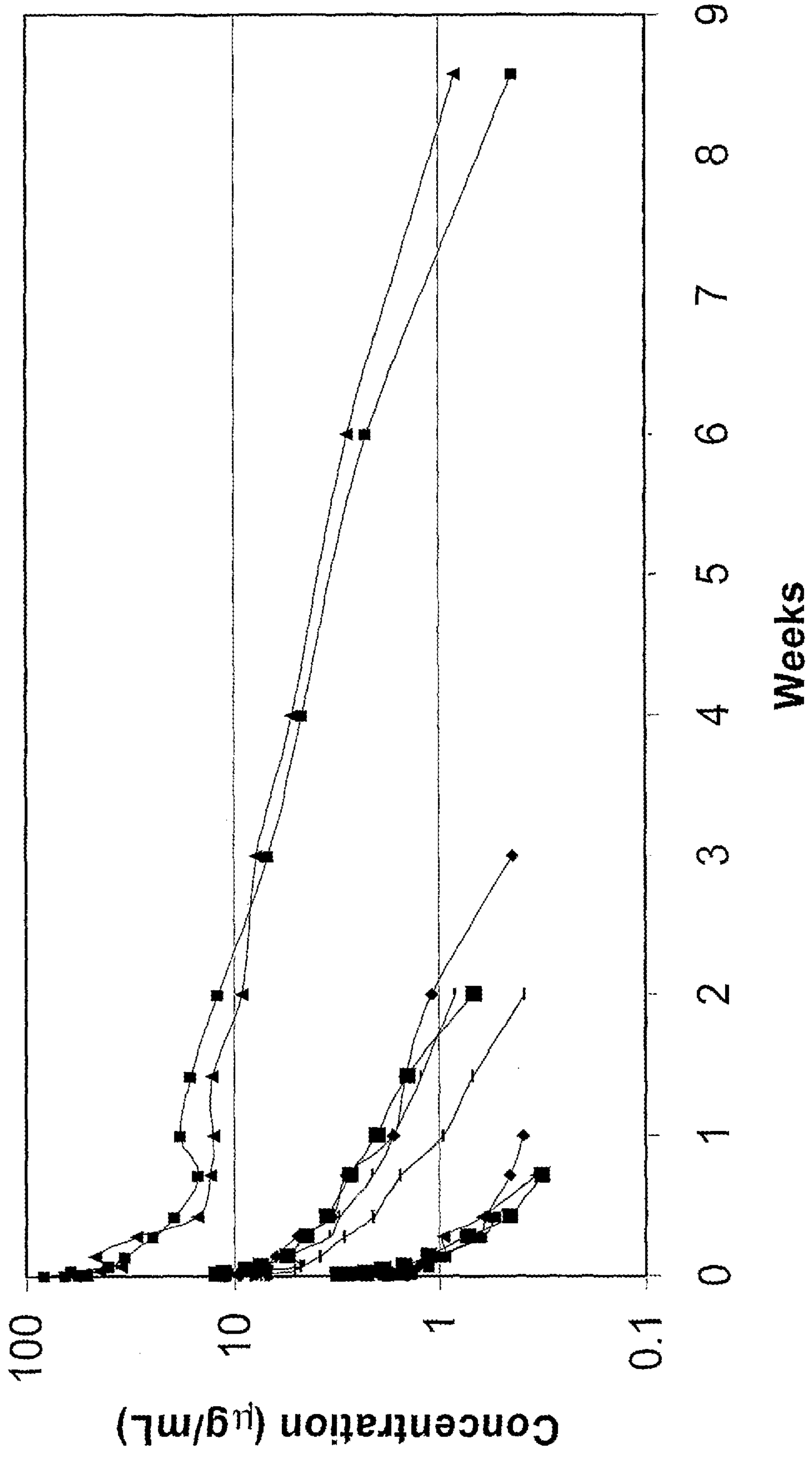


Figure 7

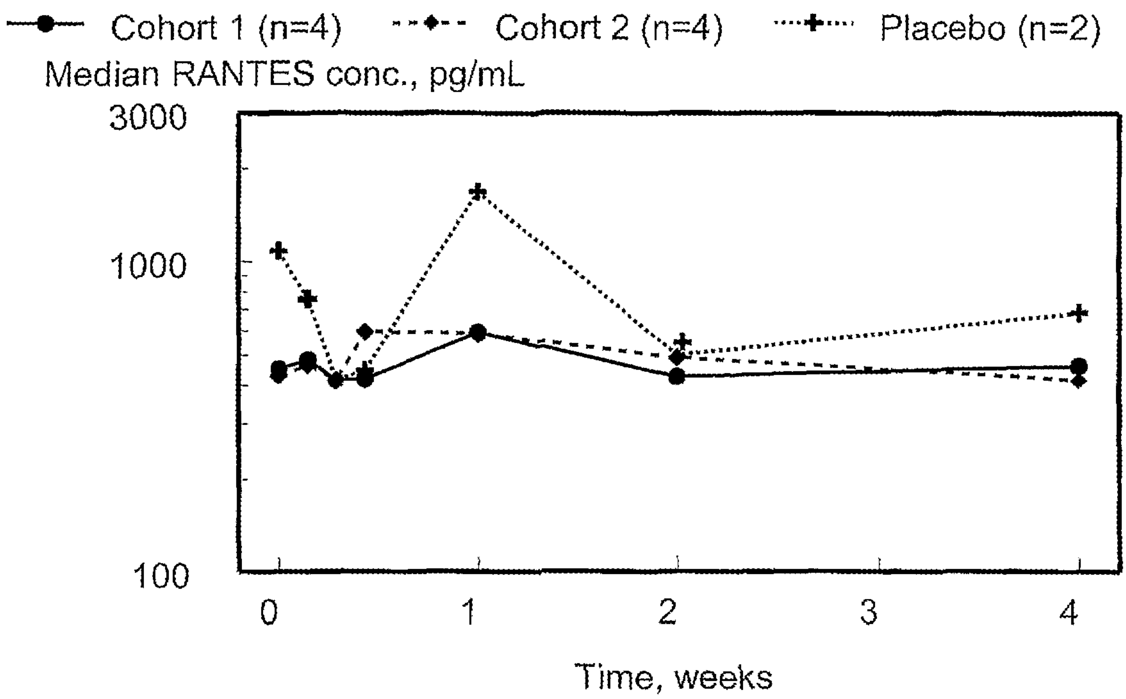
Cohort 1 (n=4)
Cohort 2 (n=4)
Placebo (n=2)
Median RANTES conc., pg/mL
3000
1000
100
0
1
2
3
4
Time, weeks

Figure 8

## Scheme 1. The $S_N2$ Displacement Route

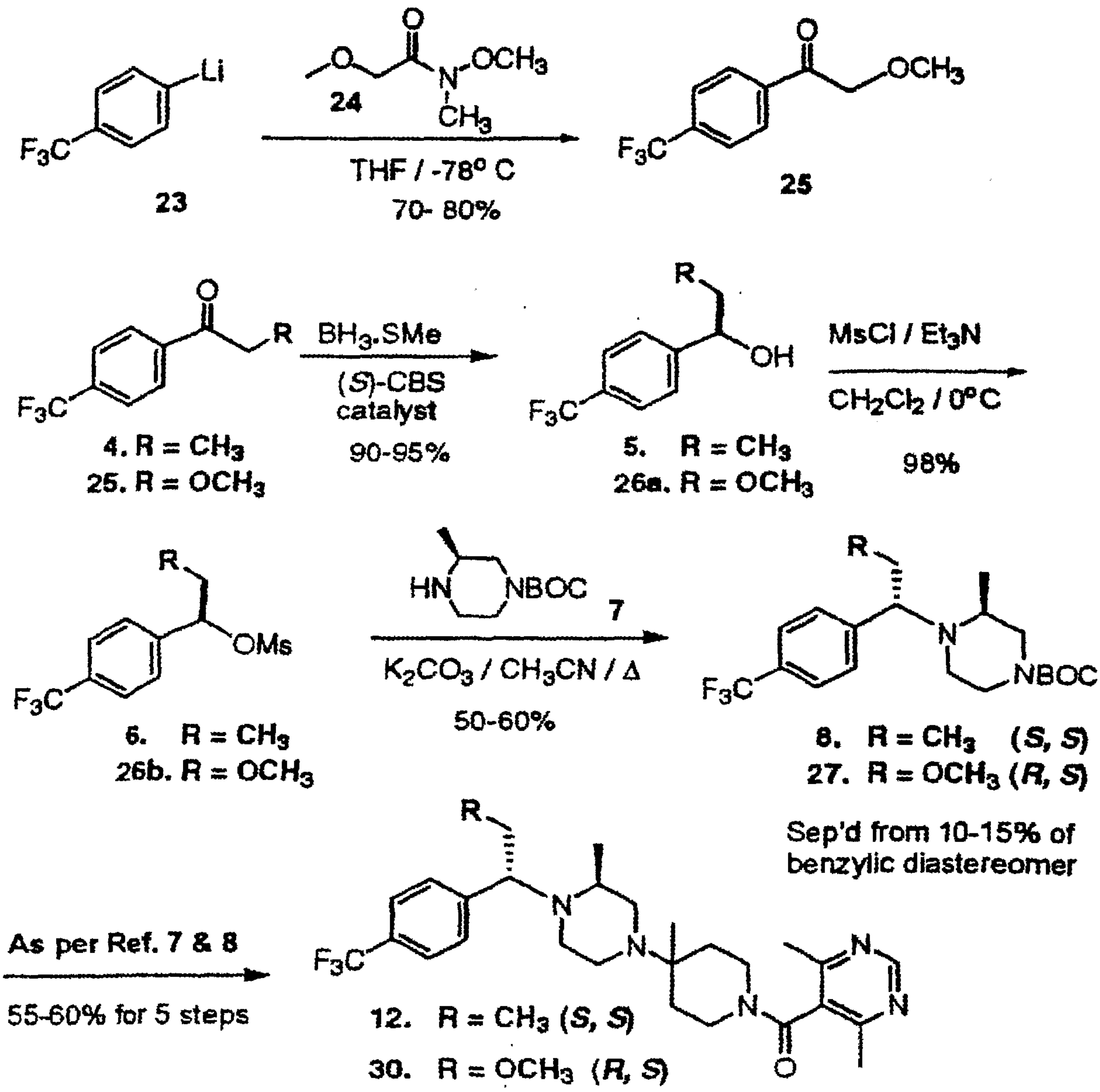


Figure 9

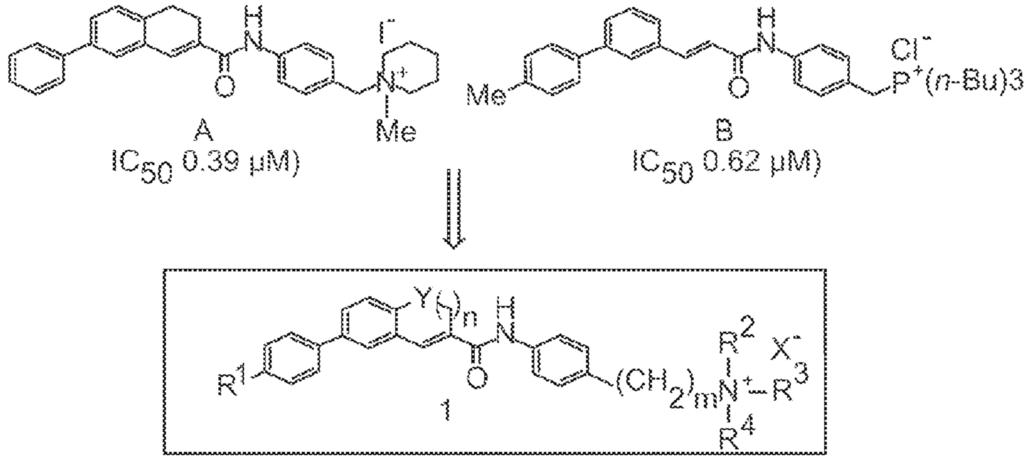

A
$IC_{50}$ 0.39 µM)

B
$IC_{50}$ 0.62 µM)

1

Figure 1. Structures of lead compounds (A,B) and design of anilide derivatives 1 with quarternary ammonium moiety.

Scheme 1[a]

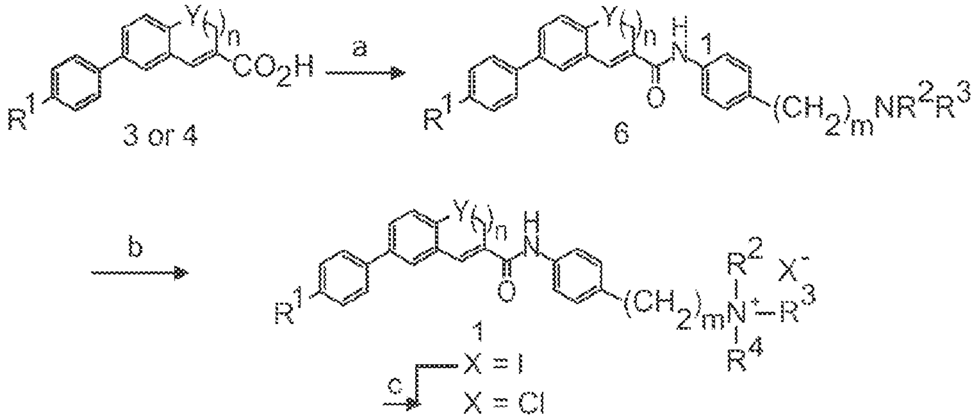


3 or 4

6

1

[a](a) (1) $(COCl)_2$,cat. DMF/$CH_2Cl_2$, (2) 5, $NEt_3$/THF or 5, HOBt, WSC, $NEt_3$/DMF; (b) MeI/DMF; (c) opm-exchange resin ($Cl^-$)/aq MeOH.

Scheme 2[a]

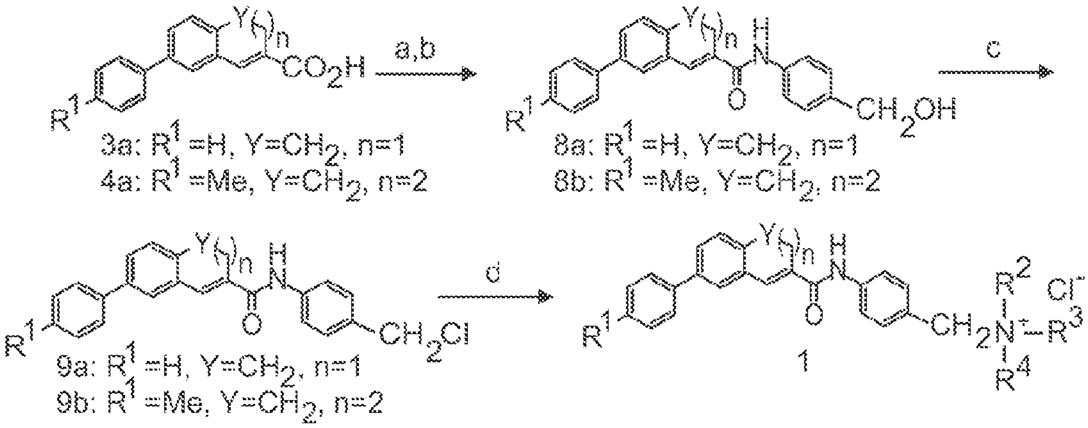


3a: $R^1$ =H, Y=$CH_2$, n=1
4a: $R^1$ =Me, Y=$CH_2$, n=2

8a: $R^1$ =H, Y=$CH_2$, n=1
8b: $R^1$ =Me, Y=$CH_2$, n=2

9a: $R^1$ =H, Y=$CH_2$, n=1
9b: $R^1$ =Me, Y=$CH_2$, n=2

1

[a](a) (1) $(COCl)_2$,cat. DMF/$CH_2Cl_2$, (2) 7, $NEt_3$/THF ; (b) HCl/acetone; (c) $SOCl_2$, pyridine/$CHCl_3$; (d) $NR^2R^3R^4$/DMF.

# Figure 10

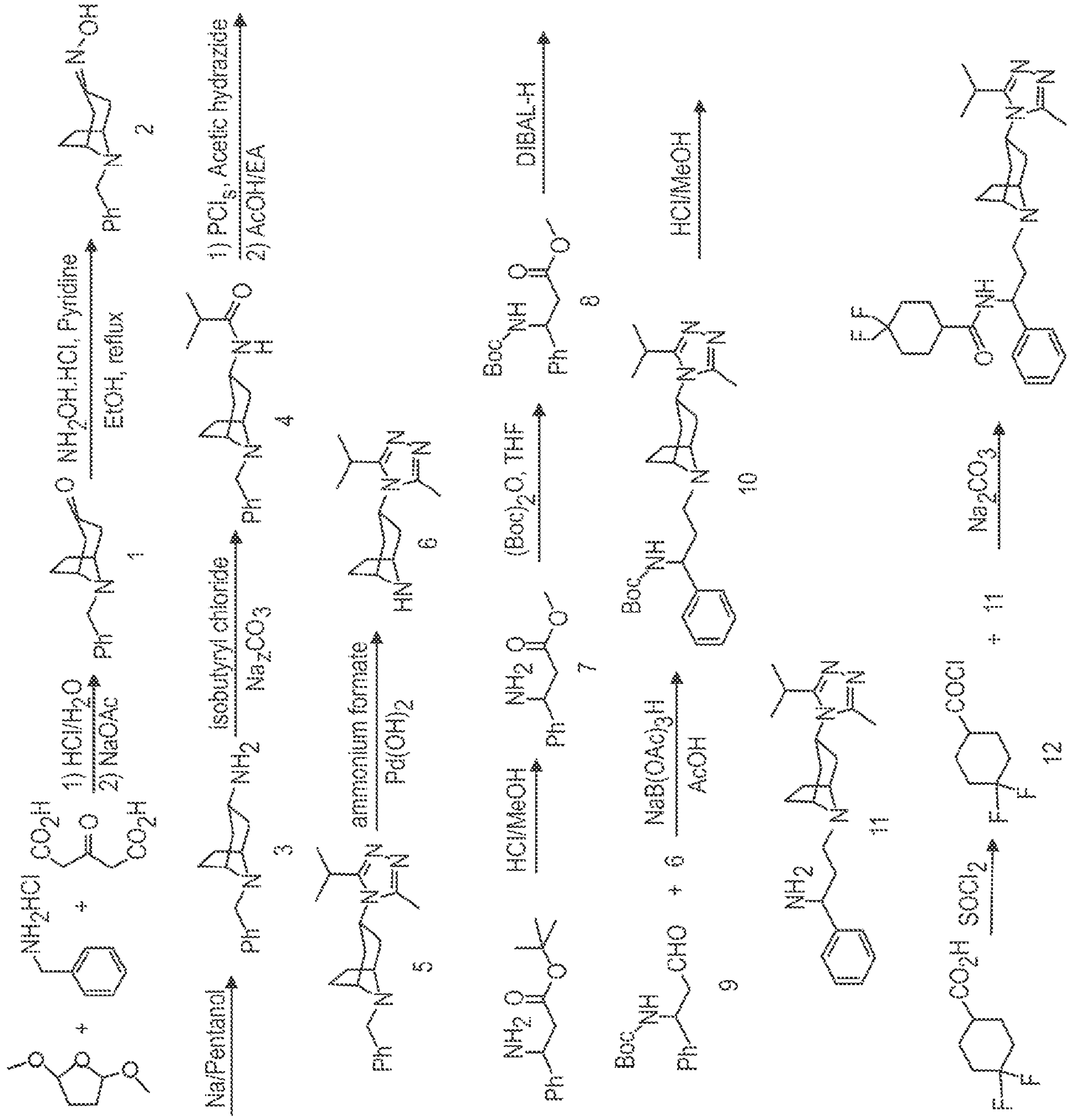


1, 2, 3, 4, 5, 6, 7, 8, 9, + 6, 10, 11, 12, + 11

Figure 11

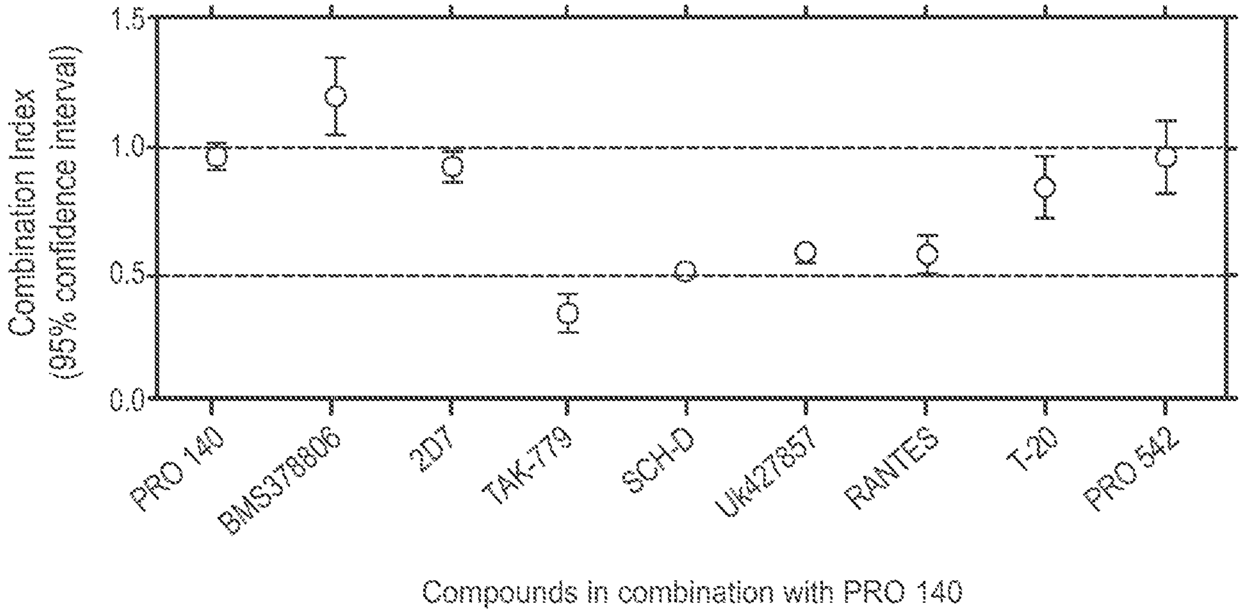

1.5
1.0
0.5
0.0
Combination Index
(95% confidence interval)
PRO 140
BMS378806
2D7
TAK-779
SCH-D
Uk427857
RANTES
T-20
PRO 542
Compounds in combination with PRO 140


Figure 12

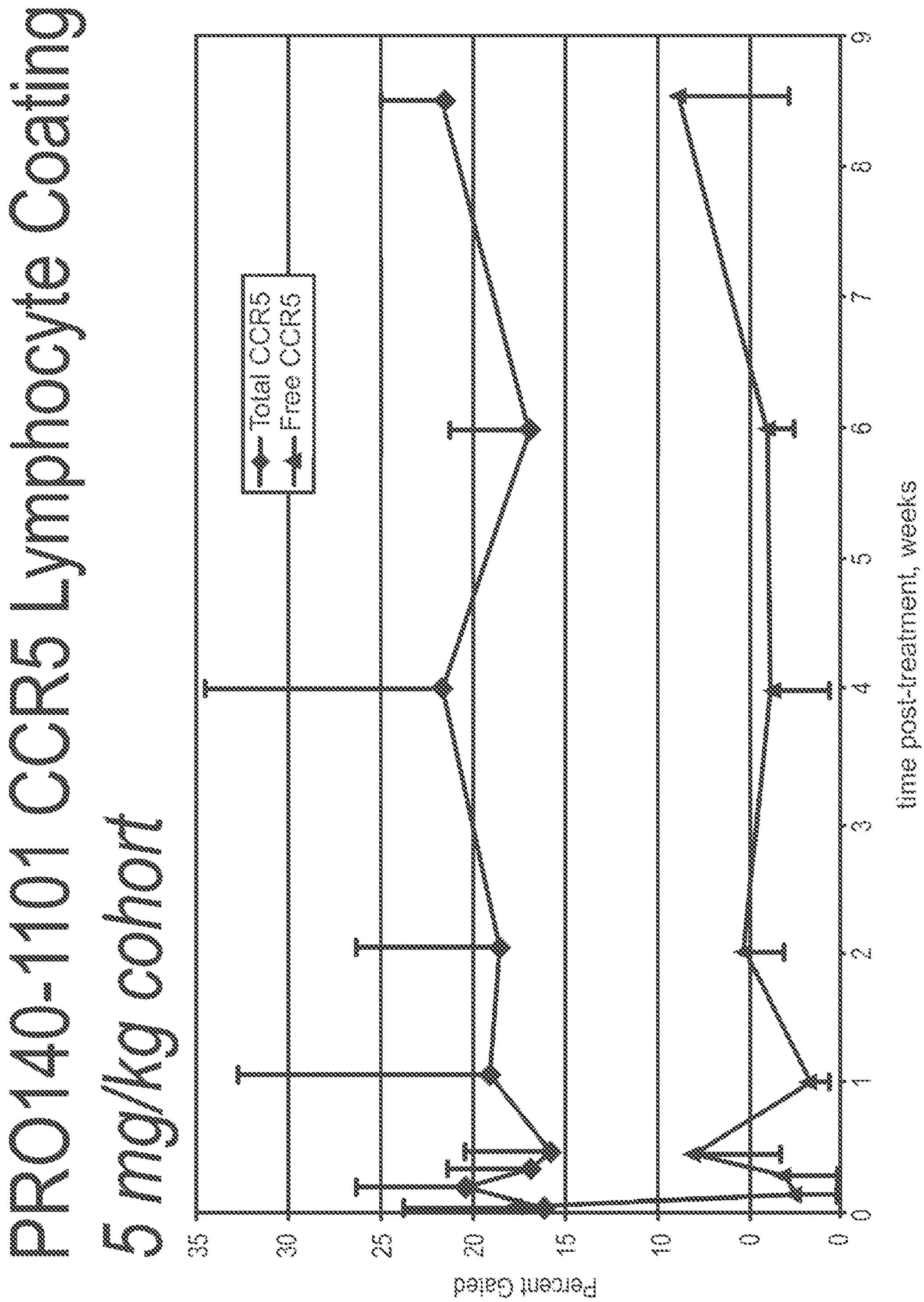
PRO140-1101 CCR5 Lymphocyte Coating
5 mg/kg cohort
Total CCR5
Free CCR5
Percent Gated
time post-treatment, weeks
0 1 2 3 4 5 6 7 8 9

Figure 13

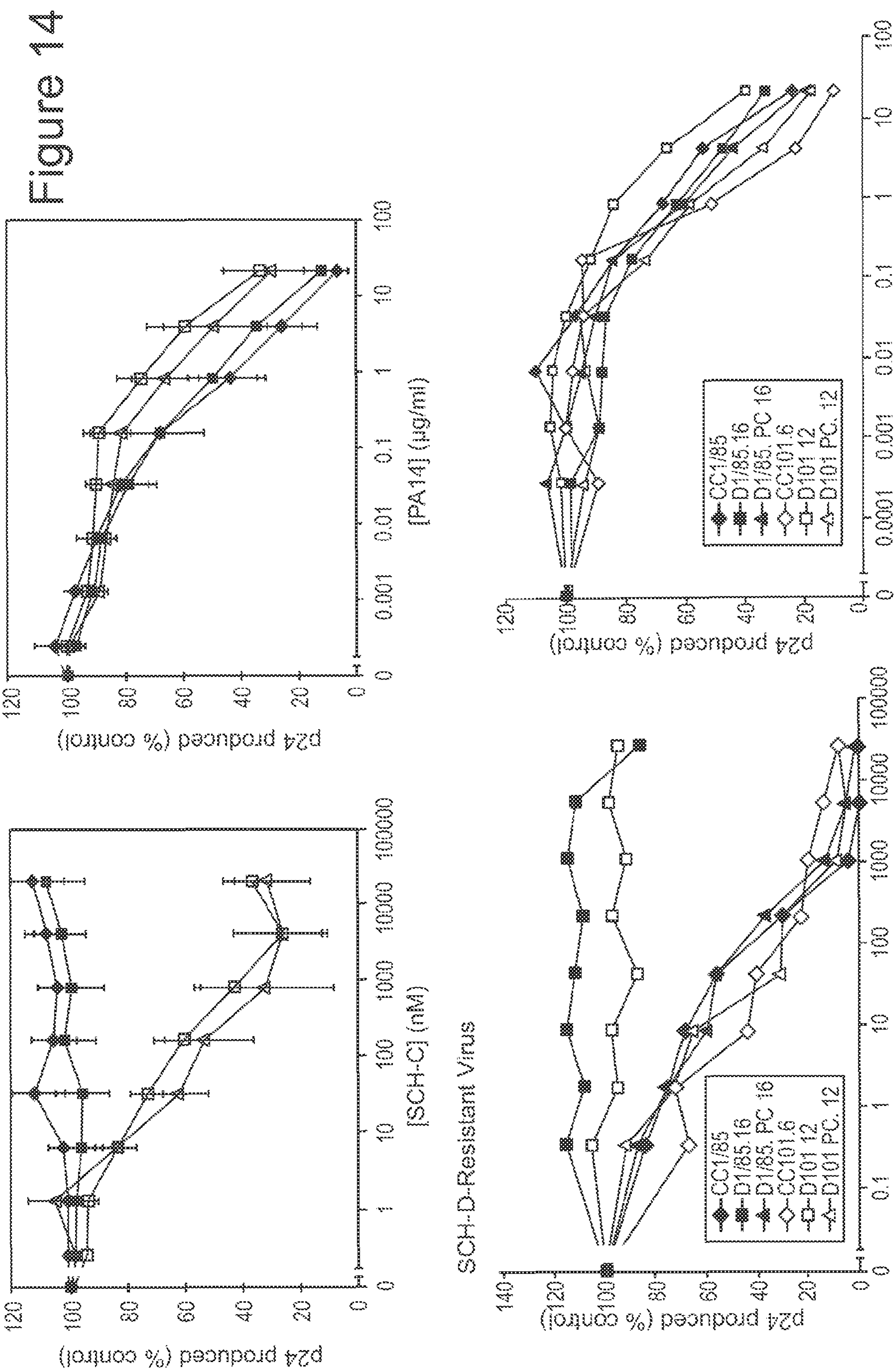

p24 produced (% control)
[SCH-C] (nM)
[PA14] (µg/ml)
SCH-D-Resistant Virus
[SCH-C] nM
[PA14] µg/ml
CC1/85
D1/85.16
D1/85. PC 16
CC101.6
D101 12
D101 PC. 12


Figure 14

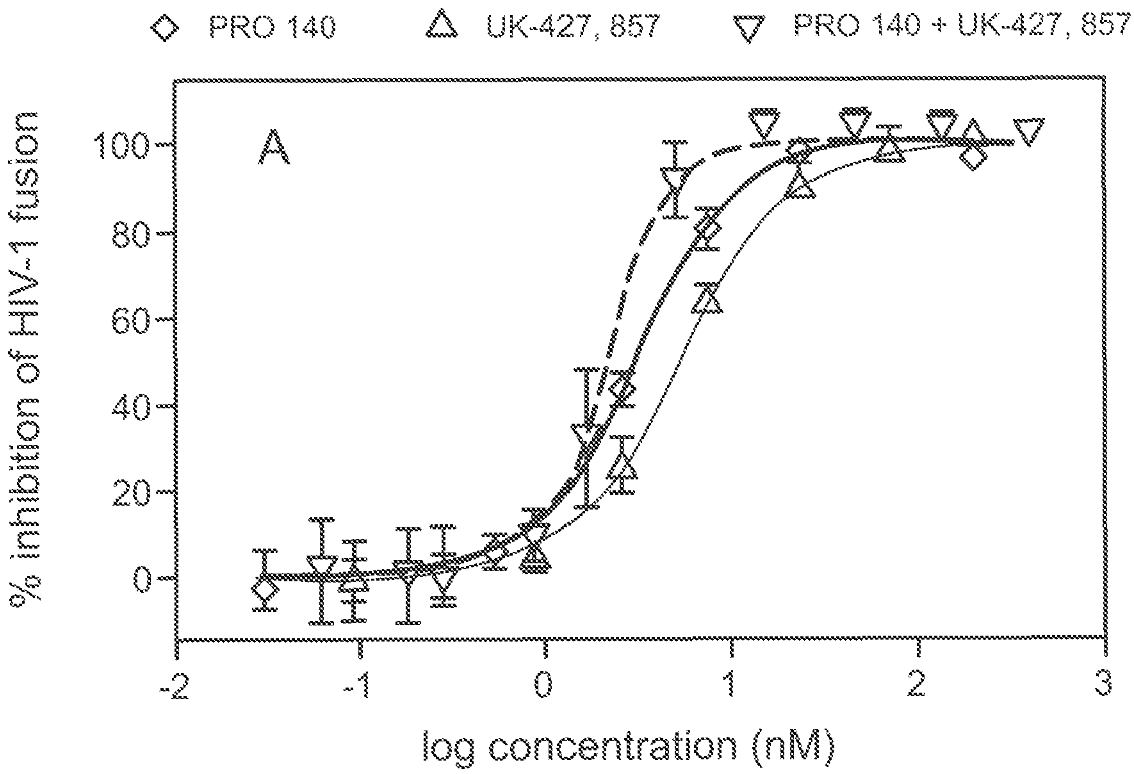
◇ PRO 140
△ UK-427, 857
▽ PRO 140 + UK-427, 857
A
% inhibition of HIV-1 fusion
100
80
60
40
20
0
-2
-1
0
1
2
3
log concentration (nM)

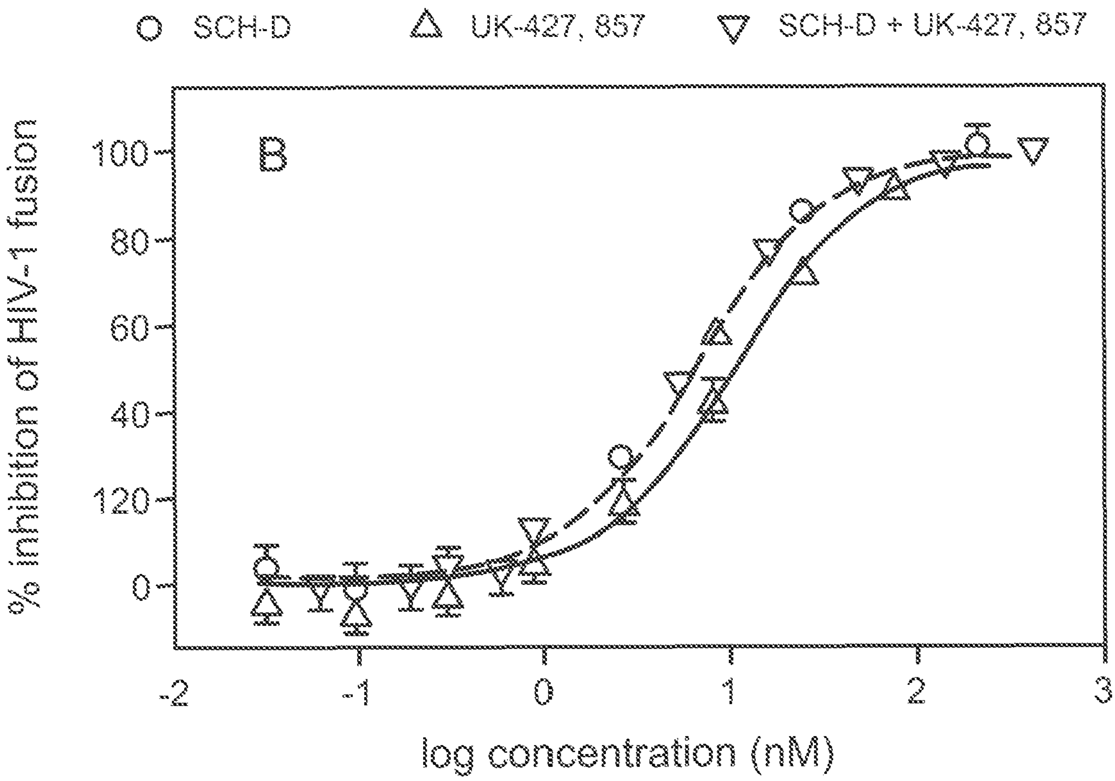
○ SCH-D
△ UK-427, 857
▽ SCH-D + UK-427, 857
B
% inhibition of HIV-1 fusion
100
80
60
40
120
0
-2
-1
0
1
2
3
log concentration (nM)

Figure 15

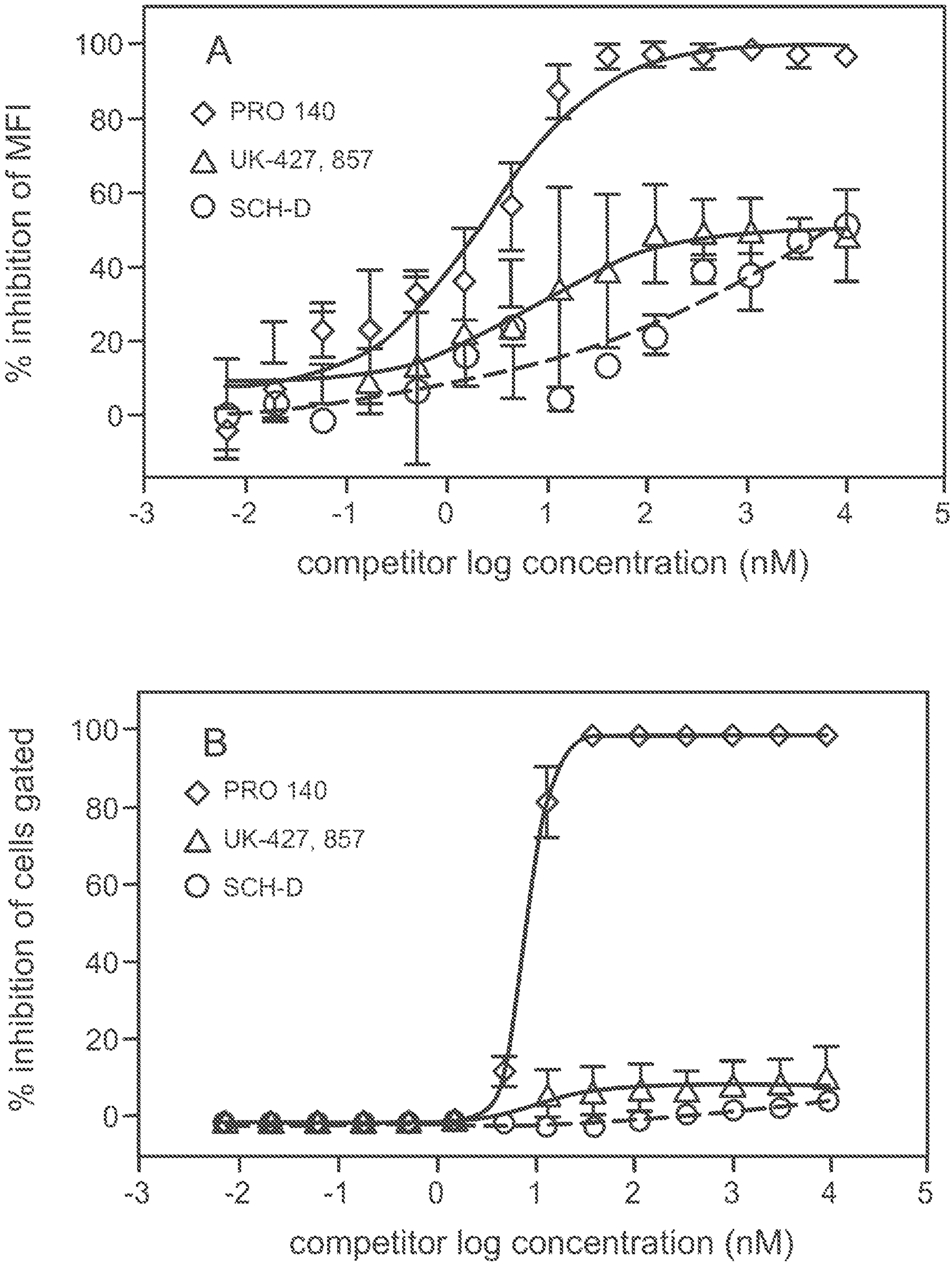
A
PRO 140
UK-427, 857
SCH-D
% inhibition of MFI
competitor log concentration (nM)
B
PRO 140
UK-427, 857
SCH-D
% inhibition of cells gated
competitor log concentration (nM)

Figure 16

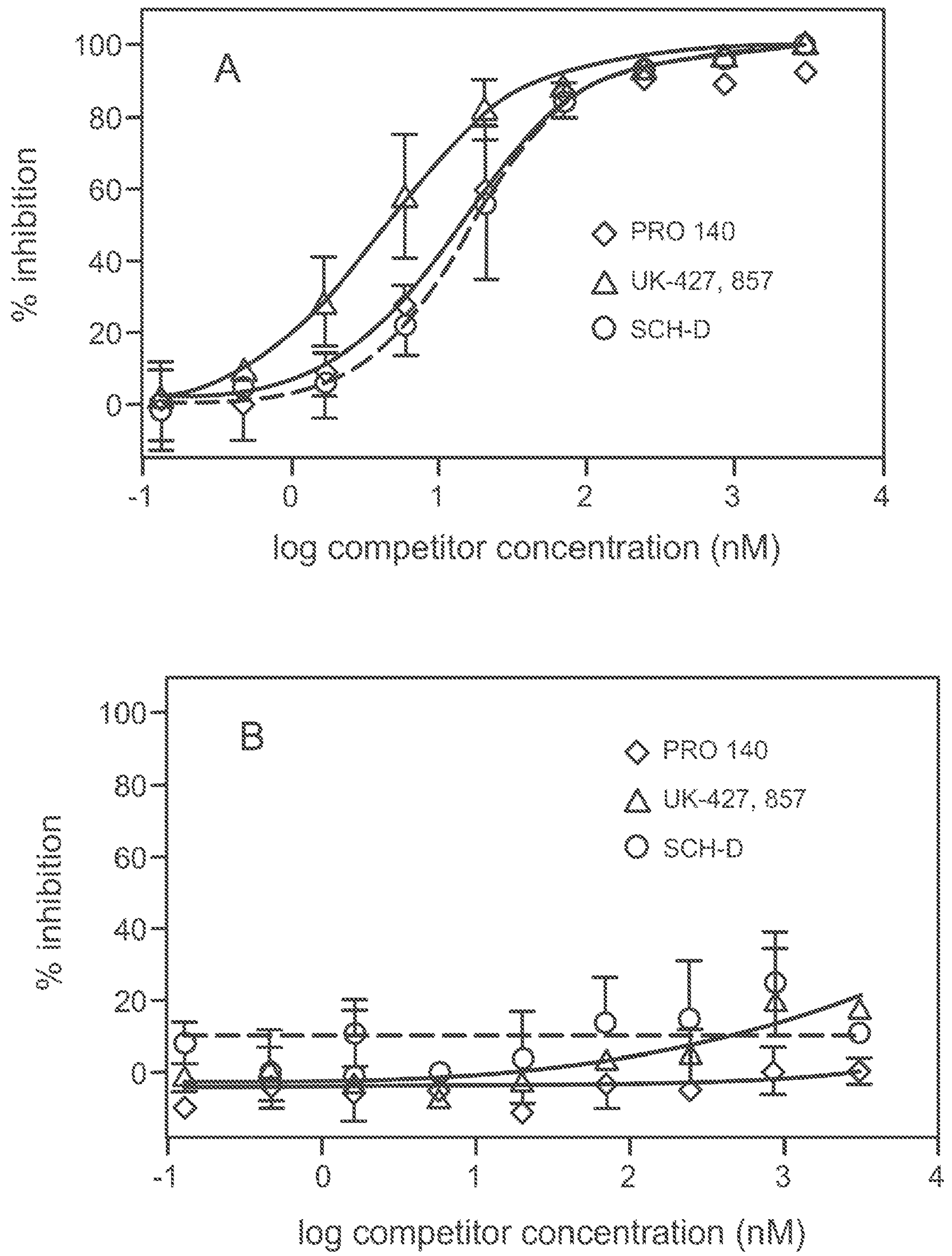
A
% inhibition
100
80
60
40
20
0
-1
0
1
2
3
4
log competitor concentration (nM)
PRO 140
UK-427, 857
SCH-D
B
% inhibition
100
80
60
40
20
0
-1
0
1
2
3
4
log competitor concentration (nM)
PRO 140
UK-427, 857
SCH-D

Figure 17

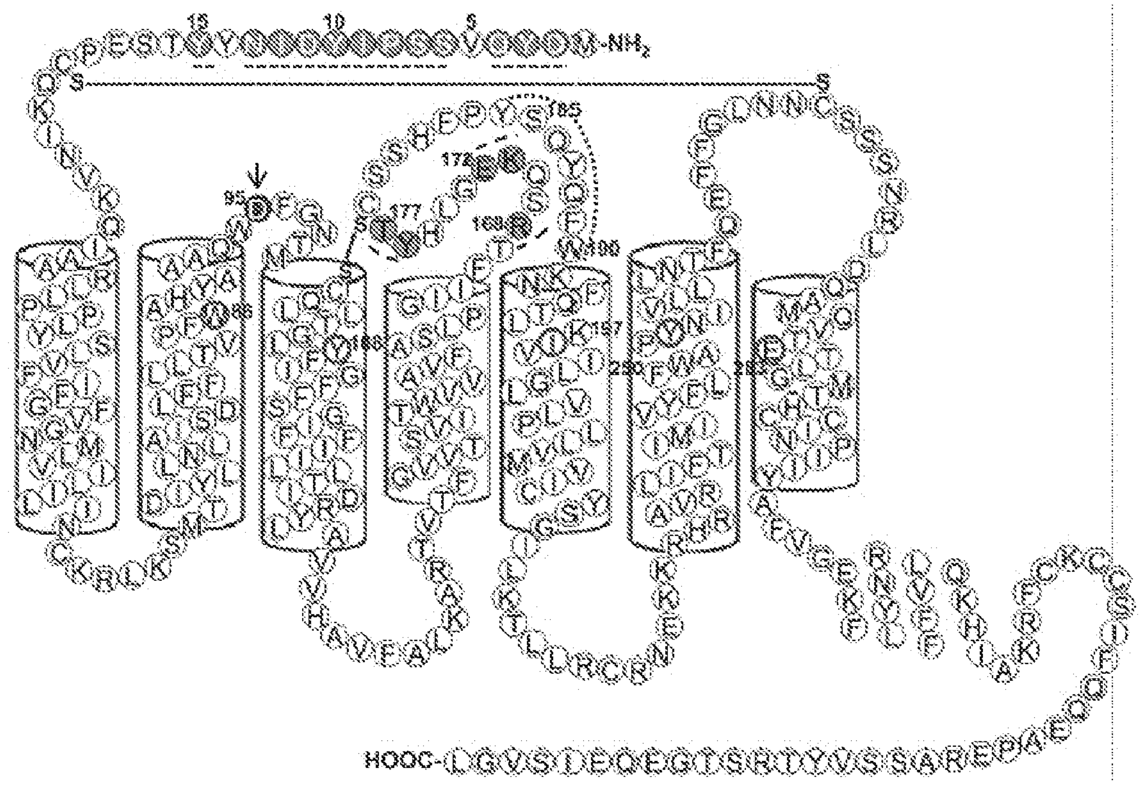


**PRIOR ART**

Figure 18

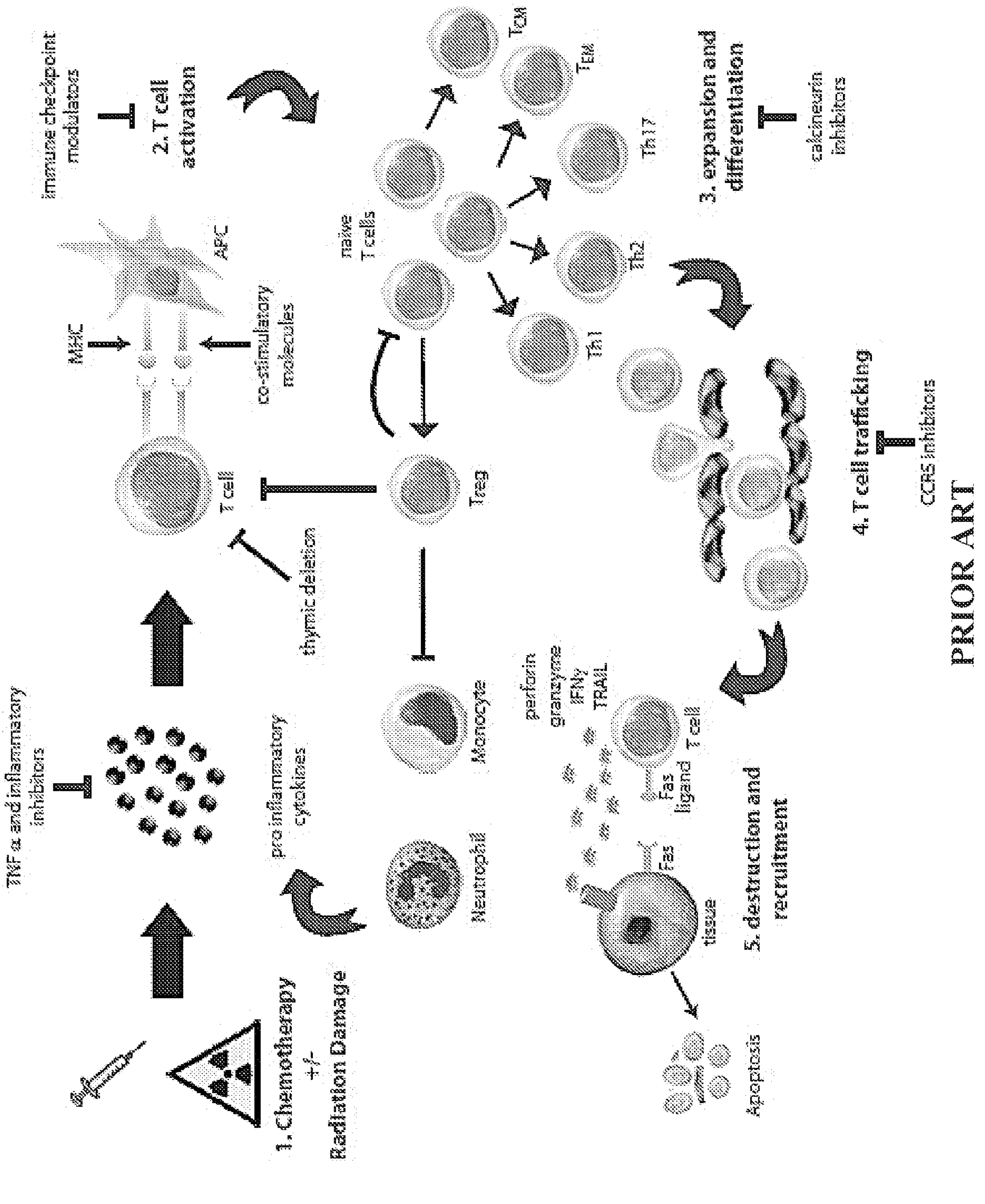

1. Chemotherapy
+/-
Radiation Damage
TNF α and inflammatory inhibitors
pro inflammatory cytokines
Neutrophil
Monocyte
thymic deletion
T cell
MHC
co-stimulatory molecules
APC
immune checkpoint modulators
2. T cell activation
Treg
naive T cells
Th1
Th2
Th17
TEM
TCM
3. expansion and differentiation
calcineurin inhibitors
4. T cell trafficking
CCR5 inhibitors
perforin
granzyme
IFNγ
TRAIL
Fas ligand
T cell
Fas
tissue
Apoptosis
5. destruction and recruitment
PRIOR ART


Figure 19

Europäisches Patentamt
European Patent Office
Office européen des brevets

# EUROPEAN SEARCH REPORT

Application Number
EP 20 19 9697

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GILLIAM BRUCE L ET AL: "Clinical use of CCR5 inhibitors in HIV and beyond", JOURNAL OF TRANSLATIONAL MEDICINE, vol. 9, no. Suppl 1, S9, 27 January 2011 (2011-01-27), pages 1-14, XP021088891, BIOMED CENTRAL, LONDON, GB ISSN: 1479-5876, DOI: 10.1186/1479-5876-9-S1-S9 * abstract * ----- | 1-13 | INV. A61K39/00 A61K39/395 A61P29/00 |
| X | RESHEF RAN ET AL: "Blockade of Lymphocyte Chemotaxis in Visceral Graft-versus-Host Disease", NEW ENGLAND JOURNAL OF MEDICINE, vol. 367, no. 2, July 2012 (2012-07), pages 135-145, XP002771948, ISSN: 0028-4793, DOI: 10.1056/NEJMoa1201248 * abstract * ----- | 1-13 | |
| X | CASTOR MARINA G M ET AL: "The role of chemokines in mediating graft versus host disease: opportunities for novel therapeutics.", FRONTIERS IN PHARMACOLOGY, vol. 3, 23, 2012, pages 1-13, XP002771949, ISSN: 1663-9812, DOI: 10.3389/fphar.2012.00023 * page 9, left-hand column, paragraph 3 - right-hand column, paragraph 2 * ----- | 1-13 | TECHNICAL FIELDS SEARCHED (IPC) A61K |
| A | US 2003/228306 A1 (OLSON WILLIAM C [US] ET AL) 11 December 2003 (2003-12-11) * figures 8-15 * ----- -/-- | 1-13 | |

1 The present search report has been drawn up for all claims

| Place of search | Date of completion of the search | Examiner |
|---|---|---|
| Munich | 11 May 2021 | Fleitmann, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 19 9697

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | YUAN JING ET AL: "Prophylaxis of acute graft-versus-host disease by CCR5 blockade combined with cyclosporine A in a murine model", INFLAMMATION RESEARCH, vol. 64, no. 2, 4 January 2015 (2015-01-04), pages 137-144, XP035426366, BIRKHAEUSER VERLAG, BASEL, CH ISSN: 1023-3830, DOI: 10.1007/S00011-014-0793-6 [retrieved on 2015-01-04] * abstract * ----- | 1-13 | |
| | | | TECHNICAL FIELDS SEARCHED (IPC) |

The present search report has been drawn up for all claims

1

| Place of search | Date of completion of the search | Examiner |
|---|---|---|
| Munich | 11 May 2021 | Fleitmann, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 19 9697

This annex lists the patent family members relating to the patent documents cited in the above-mentioned European search report. The members are as contained in the European Patent Office EDP file on
The European Patent Office is in no way liable for these particulars which are merely given for the purpose of information.

11-05-2021

| Patent document cited in search report | Publication date | Patent family member(s) | Publication date |
|---|---|---|---|
| US 2003228306 A1 | 11-12-2003 | US 2003228306 A1<br>US 2007031408 A1<br>US 2008107595 A1 | 11-12-2003<br>08-02-2007<br>08-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61941352
- WO 0162255A1 A1
- WO 03082289 A1
- WO 0035409 A
- WO 0155439 A
- WO 0222077 A
- WO 0190106A2 A
- WO 0190106 A
- US 6476034 B
- WO 03072766 A
- US 4816567 A, Cabilly
- US 5225539 A, Gregory Winter
- US 5229275 A, Goroff
- US 5545806 A, Lonberg
- US 5545807 A, Surani
- US 5565332 A, Hoogenboom
- US 5567610 A, Borrebaeck
- US 5585089 A, Queen
- US 5591669 A, Krimpenfort
- US 5693761 A, Queen
- US 6150584 A, Kucherlapati
- US 6476034 B2, Wang
- US 6759519 B2, Li
- WO 9007861 A
- WO 0190106 A2
- WO 0162255 A1


### Non-patent literature cited in the description


- **LISA PALMER ; GEORGE SALE ; JOHN BALOGUN ; DAN LI ; DAN JONES ; JEFFREY MOLLDREM ; RAINER STORB ; QING MA.** Chemokine Receptor CCR5 Mediates Allo-Immune Responses in Graft-vs-Host Disease. *Biol Blood Marrow Transplant,* March 2010, vol. 16 (3), 311-319
- *Transplants Data and Outcomes,* 31 October 2013, http://bloodcell.transplant.hrsa.gov/research/transplant_data/index.html
- Annual Report: Stem Cell Donor Registries. World Marrow Donor Association and European Blood and Marrow Transplantation Group, 2010
- Annual Report: Unrelated Cord Blood Banks/Registries. World Marrow Donor Association, European Blood and Marrow Transplantation Group, and NetCord Foundation, 2010
- **ANTHONY D. SUNG ; NELSON J. CHAO.** Concise Review: Acute Graft-Versus-Host Disease: Immunobiology, Prevention, and Treatment. *Stem Cells Tranlations Medicine,* 2013, vol. 2, 25-32
- **M. MURAI ; H. YONEYAMA ; A. HARADA ; Z. YI ; C. VESTERGAARD ; B. GUO ; K. SUZUKI ; H. ASAKURA ; K. MATSUSHIMA.** Active participation of CCR5(+)CD8(+) T lymphocytes in the pathogenesis of liver injury in graft-versus-host disease. *J. CLIN. INVEST.,* July 1999, vol. 104, 49-57
- **LISBETH A. WELNIAK ; ZHAO WANG ; KAI SUN ; WILLIAM KUZIEL ; MIRIAM R. ANVER ; BRUCE R. BLAZAR ; WILLIAM J. MURPHY.** An absence of CCR5 on donor cells results in acceleration of acute graft-vs-host disease. *EXPERIMENTAL HEMATOLOGY,* March 2004, vol. 32 (3), 318-324
- **GERO HÜTTER, M.D. ; DANIEL NOWAK, M.D. ; MAXIMILIAN MOSSNER, B.S. ; SUSANNE GANEPOLA, M.D. ; ARNE MÜßIG, M.D. ; KRISTINA ALLERS, PH.D. ; THOMAS SCHNEIDER, M.D., PH.D. ; JÖRG HOFMANN, PH.D. ; CLAUDIA KÜCHERER, M.D. ; OLGA BLAU, M.D.** Long-Term Control of HIV by CCR5 Delta32/Delta32 Stem-Cell Transplantation. *N. ENG. J. MED.,* 2009, vol. 360, 692-698
- **R. RESHEF ; S.M. LUGER ; A.W. LOREN ; N.V. FREY ; S.C. GOLDSTEIN ; E.O. HEXNER ; E.A STADTMAUER ; J. SMITH ; R. MICK ; D.F. HEITJAN.** Feasibility, Safety and Efficacy of Maraviroc, a CCR5 Antagonist, in Graft-Versus-Host Disease Prevention After Reduced intensity Conditioned (RIC) Allogeneic Stem Cell Transplant (SCT) a Phase I/II Study. *BIOLOGY OF BLOOD AND MARROW TRANSPLANTATION,* February 2011, vol. 17 (2), S331
- **WILLIAM C. OLSON ; JEFFREY M. JACOBSON.** CCR5 Monoclonal Antibodies for HIV-1 Therapy. *Curr Opin HIV AIDS,* March 2009, vol. 4 (2), 104-111
- **ALKHATIB, G. et al.** *Science,* 1996, vol. 272, 1955
- **ALONZO III, F. et al.** *Nature,* 2013, vol. 493 (7430), 51-55
- **ALLAWAY, G. P. et al.** *AIDS Res. Hum. Retrovir.,* 1993, vol. 9, 581-587
- **ALLAWAY, G. P. et al.** *AIDS Research and Human Retroviruses,* 1995, vol. 11, 533-539

- **ASANO, S. et al.** *ACS Med. Chem. Lett.,* 2014, vol. 5, 133-137
- **BABA, M. et al.** *12th Conference on Retroviruses and Opportunistic Infections,* 2005
- **BABA, M et al.** *Proc. Nab. Acad. Sci. USA,* 1999, vol. 96, 5698-5703
- **BALOTTA, C, P. et al.** *AIDS,* 1997, vol. 11, F67-F71
- **BASAVAPATHRUNI, A. et al.** *J Biol Chem.,* 2004, vol. 279, 6221-6224
- **BERGER, E. A.** *AIDS,* 1997, vol. 11, S3-S16
- **BIENIASZ, P. D. ; B. R. CULLEN.** *Frontiers in Bioscience,* 1998, vol. 3, d44-58
- **BITI, R., R. et al.** *Nature Med.,* 1997, vol. 3, 252-253
- **BORKOW, G. et al.** *Antimicrob. Agents Chemother.,* 1999, vol. 43, 259-263
- **BUDDY, L. et al.** *J. Immunol.,* 1992, vol. 149, 1779-1787
- **BUDDY, L. et al.** *J. ViroL,* 1995, vol. 69, 4267-4273
- **CHOE, H et al.** *Cell,* 1996, vol. 85, 1135-1148
- **CHOU, T. C. ; D. C. RIDEOUT.** Synergism and antagonism in chemotherapy. Academic Press, 1991
- **CHOU, T. C. ; P. TALALAY.** *Adv. Enzyme Regulation,* 1984, vol. 22, 27-55
- **COCCHI, F. et al.** *Science,* 1995, vol. 270, 1811-1815
- **COMBADIERE, C et al.** *J. Leukocyte BioL,* 1996, vol. 60, 147-152
- **CONNOR, R. I. et al.** *J. Exp. Med.,* 1997, vol. 185, 621-628
- **CORMIER, E. G. ; T. DRAGIC.** *J ViroL,* 2002, vol. 76, 8953-8957
- **CUDECK, R. ; L. L. ODELL.** *Psychol. Bull.,* 1994, vol. 115, 475-487
- **DALGLEISH, A. G. et al.** *Nature,* 1984, vol. 312, 763-766
- **DEMAREST, J. et al.** *11th Conference on Retroviruses and Opportunistic Infections,* 08 February 2004
- **DENG, H. et al.** *Nature,* 1996, vol. 381, 661-666
- **DORR, P. et al.** *10th Conference on Retroviruses and Opportunistic Infections,* 2003
- **DORR, T, P. et al.** *Antimicrobial Agents and Chemotherapy,* 2005, vol. 49, 4721-4732
- **DRAGIC, T. et al.** *Advances in Research and Therapy,* 1997, vol. 7, 2-13
- **DRAGIC, T. et al.** *J. ViroL,* 1992, vol. 66, 4794-4802
- **DRAGIC, T. et al.** *Nature,* 1996, vol. 381, 667-673
- **DRAGIC, T. et al.** *Proc Nab Acad Sci USA,* 2000, vol. 97, 5639-44
- **ESTE J A.** *Cum Opin. Investig. Drugs.,* 2002, vol. 3, 379-383
- **FATKENHEUER, G. et al.** *Nat Med,* 2005, vol. 11, 1170-1172
- **FENG, Y. et al.** *Science,* 1996, vol. 272, 872-877
- **FINKE, P. E. et al.** *Bioorg. Med. Chem Lett.,* 2001, vol. 11, 2475-2479
- **GARZINO-DEMO, A. et al.** *Proc Natl Acad Sci USA.,* 1999, vol. 96, 11986-11991
- **HAIM-BOUKOBZA, S. et al.** *Journal of Hepatology,* 2013, vol. 59, 613-615
- **HALE, J. J. et al.** *Bioorg. Med. Chem. Lett,* 2001, vol. 11, 2741-2745
- **HALE, J. J. et al.** *Bioorg. Med. Chem. Lett.,* 2002, vol. 12, 2997-3000
- **HEGDE, V. R. et al.** *Bioorg. Med. Chem. Lett.,* 2004, vol. 12, 5339-5342
- **HUANG, Y. et al.** *Nature Med.,* 1996, vol. 2, 1240-1243
- **HUFFNAGLE, G. B. et al.** *Immunol.,* 1999, vol. 163, 4642-4646
- **Y. IIZAWA et al.** *10th Conference on Retroviruses and Opportunistic Infections,* 10 February 2003
- **IMAMURA, S. et al.** *Bioorg. Med. Chem,* 2004, vol. 12, 2295-2306
- **IMAMURA, S. et al.** *Chem. Pharm. Bull.,* 2004, vol. 52, 63-73
- **IMAMURA, S. et al.** *Bioorg. Med. Chem,* 2005, vol. 13, 397-416
- **JAYASURIYA, H. et al.** *J. Nat. Prod.,* 2004, vol. 67, 1036-1038
- **JOHNSON, V. A. et al.** *Journal of Infectious Diseases,* 1991, vol. 164, 646-655
- **KAWAMURA, T. et al.** *J Exp Med.,* 2000, vol. 192, 1491-1500
- **KUHMANN, S. E. et al.** *J Virol,* 2004, vol. 78, 2790-2807
- **KETAS, T. J. et al.** *J. Virol.,* 2003, vol. 77, 2762-2767
- **KIM D. et al.** *Bioorg. Med. Chem Lett.,* 2001, vol. 11, 3099-3102
- **KIM D. et al.** *Bioorg. Med. Chem. Lett.,* 2001, vol. 11, 3103-3106
- **KIM D. et al.** *Bioorg. Med. Chem Lett.,* 2005, vol. 15, 2129-2134
- **KLATZMANN, D. et al.** *Nature,* 1984, vol. 312, 382-385
- **KOYANAGI, Y. et al.** *Science,* 1987, vol. 236, 819-822
- **KUHMANN, S. E. et al.** *J. ViroL,* 2004, vol. 78, 2790-2807
- **KUMAR, S et al.** *J. Pharmacol Exp. Ther.,* 2003, vol. 304, 1161-1171
- **LAAL, S. et al.** *J. Virol.,* 1994, vol. 68, 4001-4008
- **LALEZARI, J. P. et al.** *New Engl. J. of Med.,* 2003, vol. 348, 2175-2185

- **LALEZARI, J. et al.** *44th Annual Interscience Conference on Antimicrobial Agents and Chemotherapy,* 2004
- **LALEZARI, J. et al.** *AIDS,* 2005, vol. 19, 1443-1448
- **LAPIDOT, T.** *Ann. N.Y. Acad. Sci.,* 2001, vol. 938, 83-95
- **LAZZARIN, A. et al.** *New Engl. J. Med.,* 2003, vol. 348, 2186
- **LEE, B. et al.** *Journal of Biological Chemistry,* 1999, vol. 274, 9617-9626
- **LI, A. et al.** *AIDS Res. Hum. Retrovir.,* 1997, vol. 13, 647-656
- **LI, A. et al.** *J. ViroL,* 1998, vol. 72, 3235-3240
- **LIN, P. F. et al.** *Proc. Natl. Acad. Sci. USA,* 2003, vol. 100, 11013-11018
- **LIN, P. F. et al.** *9th Conference on Retroviruses and Opportunistic Infections,* 2002
- **LITTMAN, D. R.** *Cell,* 1998, vol. 93, 677-680
- **LITWIN, V. et al.** *J. Virol.,* 1996, vol. 70, 6437-6441
- **LIU, R. et al.** *Cell,* 1996, vol. 86, 367-377
- **LIU, H.** *Proceedings of the National Academy of Sciences of the United States of America,* 1999, vol. 96, 4581-4585
- **LYNCH, C. L. et al.** *Bioorg. Med. Chem Lett,* 2003, vol. 12, 3001-3004
- **LYNCH, C. L.** *Bioorg. Med. Chem. Lett.,* 2003, vol. 13, 119-123
- **LYNCH, C. L. et al.** *Bioorg. Med. Chem. Lett.,* 2002, vol. 12, 677-679
- **LYNCH, C. L. et al.** *Org. Lett.,* 2003, vol. 5, 2473-2475
- **MADDON, P. J. et al.** *Cell,* 1986, vol. 47, 333-348
- **MAEDA, K. et al.** *J. Virol.,* 2004, vol. 78, 8654-8662
- **MAEDA, K. et al.** *J. BioL Chem.,* 2001, vol. 276, 35194-35200
- **MAROZSAN, A. J. et al.** *Virology,* 2005, vol. 338, 182-199
- **MCCOMBIE, S. W. et al.** *Bioorg. Med. Chem. Lett.,* 2001, vol. 13, 567-571
- **MCDOUGAL, J. S. et al.** *Science,* 1986, vol. 231, 382-385
- **MERLUZZI, V. J. et al.** *Science,* 1990, vol. 250, 1411-1413
- **MICHAEL, N. L. et al.** *Nature Med.,* 1997, vol. 3, 338-340
- **MOLLA, A. et al.** *Antimicrob. Agents Chemother.,* 2002, vol. 46, 2249-2253
- **MOORE, J. P ; Q. J. SATTENTAU ; P. J. KLASSE ; L. C. BURKLY.** *J. Virol.,* 1992, vol. 66, 4784-4793
- **MURGA, J. et al.** *Antimicrobial Agents and Chemotherapy,* 2006, 3289-3296
- **NAGASHIMA, K. A.** *J. Infect Dis.,* 2001, vol. 183, 1121-1125
- **NAKATA, H. et al.** *J. ViroL,* 2005, vol. 79, 2087-2096
- **NISHIKAWA, M. et al.** *Antimicrob. Agents Chemother,* 2005, vol. 49, 4708-4715
- **O'BRIEN, T. R. et al.** *Lancet,* 1997, vol. 349, 1219
- **OLSON, W. C. et al.** *Curr Opin HIV AIDS,* 2009, vol. 4 (2), 104-111
- **OLSON, W. C et al.** *J. ViroL,* 1999, vol. 73, 4145-4155
- **OLSON, W. C. ; P. J. MADDON.** *Current Drug Targets-Infectious Disorders,* 2003, vol. 3, 283-294
- **PALANI, A et al.** *J. Med. Chem.,* 2002, vol. 45, 3143-3160
- **PALANI, A et al.** *J. Med. Chem,* 2001, vol. 44, 3339-3342
- **PALANI, A et al.** *Bioorg. Med. Chem Lett.,* 2003, vol. 13, 705-708
- **PALANI, A et al.** *Bioorg. Med. Chem. Lett,* 2003, vol. 13, 709-712
- **PALELLA, F. J. et al.** *The New England Journal of Medicine,* 1998, vol. 338, 853
- **PALMER, L. A. et al.** *Biol Blood Marrow Transplant,* 2010, vol. 16 (3), 311-319
- **RAPORT, C. J. et al.** *J. Leukocyte Biol.,* 1996, vol. 59, 18-23
- **RAY, N. ; R. W. DOMS.** *Curr. Top. Microbiol Immunol.,* 2006, vol. 303, 97-120
- **RESHEF, R. et al.** *N Engl J Med.,* 2012, vol. 367 (2), 135-145
- **RESHEF, R. et al.** *Biology of Blood and Marrow Transplant,* 2011, vol. 17, S331
- **REYES, G.** Development of CCR5 antagonists as a new class of anti-HTV therapeutic. *8th Conference on Retroviruses and Opportunistic Infections,* 2001
- **REYNES, J. et al.** SCH C: Safety and antiviral effects of a CCR5 receptor antagonist in HIV-1 infected subjects. *9th Conference on Retroviruses and Opportunistic Infections,* 2002
- **ROBINSON, B. S. et al.** *Antimicrob. Agents Chemother,* 2000, vol. 44, 2093-2099
- **ROSCHKE, V. et al.** *44th Annual Interscience Conference on Antimicrobial Agents and Chemotherapy,* 2004
- **SAMSON, M.** *J. BioL Chem,* 1997, vol. 272, 24934-24941
- **SCHECTER, A. D. et al.** *J. BioL Chem.,* 2000, vol. 275, 5466-5471
- **SCHOLS, D. et al.** *J. Ex. Med.,* 1997, vol. 186, 1383-1388
- **SCHUH, J. M. et al.** *FASEB J,* 2002, vol. 16, 228-230
- **SCHURMANN, D. et al.** *Abstract 140LB, San Francisco,* 2004
- **SEIBERT, C. et al.** *Virology,* 2006, vol. 349 (1), 41-54

- **SETO, M. et al.** *Bioorg. Med. Chem. Left.,* 2005, vol. 13, 363-386
- **SETO, M. et al.** *Chem Pharm. Bull.,* 2004, vol. 52, 818-829
- **SETO, M. et al.** *Chem. Pharm. Bull.,* 2004, vol. 52, 577-590
- **SHAH, S. K. et al.** *Bioorg. Med. Chem. Lett.,* 2005, vol. 15, 977-982
- **SHANKARAN, K. et al.** *Bioorg. Med. Chem. Lett.,* 2004, vol. 14, 3589-3593
- **SHANKARAN, K. et al.** *Bioorg. Med. Chem Lett.,* 2004, vol. 14, 3419-3424
- **SHEN, D. M. et al.** *Bioorg. Med. Chem. Lett.,* 2004, vol. 14, 935-939
- **SHEN, D. M. et al.** *Bioorg. Med. Chem. Lett.,* 2004, vol. 14, 941-945
- **SHIRAISHI, M. et al.** *J. Med. Chem.,* 2000, vol. 43, 2049-2063
- **SHU, M. et al.** *Bioorg. Med. Chem Lett.,* 2004, vol. 14, 947-52
- **SI, Z. et al.** *Proc. Natl. Acad. Sci. USA,* 2004, vol. 101, 5036-5041
- **SIMMONS, G. et al.** *J. ViroL,* 1996, vol. 70, 8355-8360
- **SPENLEHAUER, C. et al.** *Virology,* 2001, vol. 280, 292-300
- **STRIZKI, J. M. et al.** *Proc. Natl. Acad. Sci. USA.,* 2001, vol. 98, 12718-12723
- **SUNG, A. D. et al.** *Best Pract Res Clin Haematol,* 2013, vol. 26 (3
- **SUNG, A. D. et al.** *Stem Cells Translational Medicine,* 2013, vol. 2, 25-32
- **TAGAT, J. R. et al.** *J. Med. Chem.,* 2001, vol. 44, 3343-3346
- **TAGAT, J. R. et al.** *Bioorg. Med. Chem. Lett.,* 2001, vol. 11, 2143-2146
- **TAGAT, J. R. et al.** *J. Med. Chem.,* 2004, vol. 47, 2405-2408
- **TAKASHIMA, K. et al.** *Antimicrob. Agents Chemother.,* 2005, vol. 49, 374-3482
- **THALI, M. et al.** *J. Acquir. Immune Defic. Syndr.,* 1992, vol. 5, 591-599
- **THOMA, G. et al.** *J. Med. Chem.,* 2004, vol. 47, 1939-1955
- **TILLEY, S. A. et al.** *AIDS Res. Hum. Retrovir.,* 1992, vol. 8, 461-467
- **TRAN, E. H. et al.** *Eur. J. ImmunoL,* 2000, vol. 30, 1410-1415
- **TREMBLAY, C. et al.** *Journal of Acquired Immune Deficiency Syndromes and Human Retrovirology,* 2000, vol. 25, 99-102
- **TREMBLAY, C. L. et al.** *Antimicrobal Agents and Chemotherapy,* 2002, vol. 46, 1336-1339
- **TREMBLAY, C. L. et al.** *12th Conference on Retroviruses and Opportunistic Infections,* 22 February 2005
- **TREMBLAY, C. L. et al.** *Antivir. Ther.,* 2005, vol. 10, 967-968
- **TREMBLAY, C. L. et al.** *Antimicrob. Agents Chemother.,* 2005, vol. 49, 3483-3485
- **TRKOLA, A. et al.** *J. Virol.,* 2001, vol. 75, 579-588
- **TRKOLA, A. et al.** *Journal of Virology,* 1999, vol. 73, 8966-8974
- **TRKOLA, A. et al.** *J. ViroL,* 1998, vol. 72, 1876-1885
- **TSAMIS, F. et al.** *Journal of Virology,* 2003, vol. 77, 5201-5208
- **VIJH-WARRIER, S. et al.** *J. Virol.,* 1996, vol. 70, 4466-4473
- **WATSON, C. et al.** *Mol Pharmacol.,* 2005, vol. 67, 1268-1282
- **WESTBY, M et al.** *Antiviral Chemistry & Chemotherapy,* 2010, vol. 20, 179-192
- **WILD, C. et al.** *PNAS,* 1992, vol. 89, 10537-10541
- **WILLOUGHBY, C. A. et al.** *Bioorg, Med. Chem. Lett.,* 2001, vol. 11, 3137-41
- **WILLOUGHBY, C. A. et al.** *Bioorg. Med. Chem. Lett.,* 2003, vol. 13, 427-431
- **WU, L. et al.** *J. Exp. Med.,* 1997, vol. 186, 1373-1381
- **ZHOU, Y. et al.** *J. ImmunoL,* 1998, vol. 160, 4018-4025
- **ZHU, P. et al.** *J. ViroL,* 2001, vol. 75, 6682-6686